# EUROPEAN PATENT SPECIFICATION

(11) **EP 2 748 332 B1**
(45) Date of publication and mention of the grant of the patent: **13.06.2018**
(21) Application number: 12761842.9
(22) Date of filing: 24.08.2012
(51) Int. Cl.: C12Q 1/68

(54) **COMPOSITIONS AND METHODS FOR THE DETECTION OF MULTIPLE MICROORGANISMS**
ZUSAMMENSETZUNGEN UND VERFAHREN ZUM NACHWEIS MEHRERER MIKROORGANISMEN
COMPOSITIONS ET PROCÉDÉS PERMETTANT LA DÉTECTION DE MULTIPLES MICROORGANISMES

(30) Priority: 24.08.2011 US 201161527107 P; 29.06.2012 US 201261666325 P
(43) Date of publication of application: 02.07.2014
(73) Proprietor: OXOID LIMITED, Basingstoke Hampshire RG24 8PW (GB)
(72) Inventor: TEBBS, Robert, Carlsbad, California 92008 (US); CUMMINGS, Craig, Pacifica California 94044 (US); BRZOSKA, Pius, Carlsbad California 92008 (US); MATHENY, Sharon, Carlsbad California 92008 (US); O'CONNELL, Catherine, Carlsbad California 92008 (US); FURTADO, Manohar, Carlsbad California 92008 (US); FANG, Rixun, Carlsbad California 92008 (US); PETRAUSKENE, Olga, San Carlos California 94070 (US); LIEW, Sueh-Ning, Union City California 94587 (US); SCHUMAKER, Michael, Berkeley California 94703 (US)
(74) Representative: CSY St Albans
(86) International application number: PCT/US2012/052383
(87) International publication number: WO 2013/029021

(56) References cited:
- CN-A- 101 113 475
- US-A1- 2010 267 012
- SHARMA V K ET AL: "Semi-automated fluorogenic PCR assays (TaqMan) forrapid detection of Escherichia coli O157:H7 and other Shiga toxigenic E. coli", MOLECULAR AND CELLULAR PROBES, ACADEMIC PRESS, LONDON, GB, vol. 13, no. 4, 1 August 1999 (1999-08-01) , pages 291-302, XP004441558, ISSN: 0890-8508, DOI: 10.1006/MCPR.1999.0251
- IBEKWE A M ET AL: "Multiplex fluorogenic real-time PCR for detection and quantification of Escherichia coli O157:H7 in dairy wastewater wetlands", APPLIED AND ENVIRONMENTAL MICROBIOLOGY, AMERICAN SOCIETY FOR MICROBIOLOGY, US, vol. 68, no. 10, 1 October 2002 (2002-10-01), pages 4853-4862, XP008099936, ISSN: 0099-2240, DOI: 10.1128/AEM.68.10.4853-4862.2002

### SEQUENCE LISTING

<110> LIFE TECHNOLOGIES CORPORATION
<120> COMPOSITIONS AND METHODS FOR DETECTION OF MULTIPLE MICROORGANISMS
<130> LT00569 PCT
<140>
   <141>
<150> 61/666,325
   <151> 2012-06-29
<150> 61/527,107
   <151> 2011-08-24
<160> 38
<170> PatentIn version 3.5
<210> 1
   <211> 25
   <212> DNA
   <213> Artificial Sequence
<220>
   <221> source
   <223> /note="Description of Artificial Sequence: Synthetic primer"
<400> 1
   gtgacagtag ctataccacg ttaca 25
<210> 2
   <211> 22
   <212> DNA
   <213> Artificial Sequence
<220>
   <221> source
   <223> /note="Description of Artificial Sequence: Synthetic primer"
<400> 2
   agtgttgtac gaaatcccct ct 22
<210> 3
   <211> 21
   <212> DNA
   <213> Artificial Sequence
<220>
   <221> source
   <223> /note="Description of Artificial Sequence: Synthetic probe"
<400> 3
   atggcgattt atctgcatcc c 21
<210> 4
   <211> 24
   <212> DNA
   <213> Artificial Sequence
<220>
   <221> source
   <223> /note="Description of Artificial Sequence: Synthetic primer"
<400> 4
   cgttttgacc atcttcgtct gatt 24
<210> 5
   <211> 18
   <212> DNA
   <213> Artificial Sequence
<220>
   <221> source
   <223> /note="Description of Artificial Sequence: Synthetic primer"
<400> 5
   tgcgacacgt tgcagagt 18
<210> 6
   <211> 24
   <212> DNA
   <213> Artificial Sequence
<220>
   <221> source
   <223> /note="Description of Artificial Sequence: Synthetic primer"
<400> 6
   cgttttgacc atcttcgtct gatt 24
<210> 7
   <211> 18
   <212> DNA
   <213> Artificial Sequence
<220>
   <221> source
   <223> /note="Description of Artificial Sequence: Synthetic primer"
<400> 7
   tgcgacacgt tgcagcgt 18
<210> 8
   <211> 28
   <212> DNA
   <213> Artificial Sequence
<220>
   <221> source
   <223> /note="Description of Artificial Sequence: Synthetic primer"
<400> 8
   gatttctcac atatttcagt gcctgatg 28
<210> 9
   <211> 23
   <212> DNA
   <213> Artificial Sequence
<220>
   <221> source
   <223> /note="Description of Artificial Sequence: Synthetic primer"
<400> 9
   ccagatctgc gattcgctgt aat 23
<210> 10
   <211> 17
   <212> DNA
   <213> Artificial Sequence
<220>
   <221> source
   <223> /note="Description of Artificial Sequence: Synthetic probe"
<400> 10
   acggacagca gttatac 17
<210> 11
   <211> 25
   <212> DNA
   <213> Artificial Sequence
<220>
   <221> source
   <223> /note="Description of Artificial Sequence: Synthetic primer"
<400> 11
   gcgaatactg gcgagactat ttcaa 25
<210> 12
   <211> 32
   <212> DNA
   <213> Artificial Sequence
<220>
   <221> source
   <223> /note="Description of Artificial Sequence: Synthetic primer"
<400> 12
   gctcatcata gtctttctta ttgtatgact ca 32
<210> 13
   <211> 32
   <212> DNA
   <213> Artificial Sequence
<220>
   <221> source
   <223> /note="Description of Artificial Sequence: Synthetic primer"
<400> 13
   gctcgtcata gtctttcttg ttgtatgact ca 32
<210> 14
   <211> 23
   <212> DNA
   <213> Artificial Sequence
<220>
   <221> source
   <223> /note="Description of Artificial Sequence: Synthetic probe"
<400> 14
   ccgctcatgc ggaaatagcc gtt 23
<210> 15
   <211> 26
   <212> DNA
   <213> Artificial Sequence
<220>
   <221> source
   <223> /note="Description of Artificial Sequence: Synthetic primer"
<400> 15
   tggaaactat cgaacaacat caaggt 26
<210> 16
   <211> 28
   <212> DNA
   <213> Artificial Sequence
<220>
   <221> source
   <223> /note="Description of Artificial Sequence: Synthetic primer"
<400> 16
   tgaatacttc atctttggtt agccatcc 28
<210> 17
   <211> 18
   <212> DNA
   <213> Artificial Sequence
<220>
   <221> source
   <223> /note="Description of Artificial Sequence: Synthetic probe"
<400> 17
   attgtacaag gcgatttc 18
<210> 18
   <211> 27
   <212> DNA
   <213> Artificial Sequence
<220>
   <221> source
   <223> /note="Description of Artificial Sequence: Synthetic primer"
<400> 18
   atgttatttg gatgccgtta tctccat 27
<210> 19
   <211> 27
   <212> DNA
   <213> Artificial Sequence
<220>
   <221> source
   <223> /note="Description of Artificial Sequence: Synthetic primer"
<400> 19
   gcttgtaacg tgaaatgtaa accatca 27
<210> 20
   <211> 19
   <212> DNA
   <213> Artificial Sequence
<220>
   <221> source
   <223> /note="Description of Artificial Sequence: Synthetic probe"
<400> 20
   ccgaactcat cataaaaag 19
<210> 21
   <211> 29
   <212> DNA
   <213> Artificial Sequence
<220>
   <221> source
   <223> /note="Description of Artificial Sequence: Synthetic primer"
<400> 21
   gaaataattc ctttgatcag ggtagaggt 29
<210> 22
   <211> 30
   <212> DNA
   <213> Artificial Sequence
<220>
   <221> source
   <223> /note="Description of Artificial Sequence: Synthetic primer"
<400> 22
   aatatttgaa gaaggaaggc ataaggacat 30
<210> 23
   <211> 21
   <212> DNA
   <213> Artificial Sequence
<220>
   <221> source
   <223> /note="Description of Artificial Sequence: Synthetic probe"
<400> 23
   caggctccaa tacccatatg t 21
<210> 24
   <211> 28
   <212> DNA
   <213> Artificial Sequence
<220>
   <221> source
   <223> /note="Description of Artificial Sequence: Synthetic primer"
<400> 24
   acagagtatt gggagaggtt tatgagaa 28
<210> 25
   <211> 29
   <212> DNA
   <213> Artificial Sequence
<220>
   <221> source
   <223> /note="Description of Artificial Sequence: Synthetic primer"
<400> 25
   gaagaaaacc taccaagaca acaattgaa 29
<210> 26
   <211> 22
   <212> DNA
   <213> Artificial Sequence
<220>
   <221> source
   <223> /note="Description of Artificial Sequence: Synthetic probe"
<400> 26
   ttgaggtttg ttctttattt cc 22
<210> 27
   <211> 30
   <212> DNA
   <213> Artificial Sequence
<220>
   <221> source
   <223> /note="Description of Artificial Sequence: Synthetic primer"
<400> 27
   catgcgttat agaaatgcaa ttgatagaca 30
<210> 28
   <211> 27
   <212> DNA
   <213> Artificial Sequence
<220>
   <221> source
   <223> /note="Description of Artificial Sequence: Synthetic primer"
<400> 28
   ccactgaaca gatgaataac agaacca 27
<210> 29
   <211> 17
   <212> DNA
   <213> Artificial Sequence
<220>
   <221> source
   <223> /note="Description of Artificial Sequence: Synthetic probe"
<400> 29
   aacgcgaatt attttcg 17
<210> 30
   <211> 24
   <212> DNA
   <213> Artificial Sequence
<220>
   <221> source
   <223> /note="Description of Artificial Sequence: Synthetic primer"
<400> 30
   ggcactgatg ctaaagctgt aaac 24
<210> 31
   <211> 25
   <212> DNA
   <213> Artificial Sequence
<220>
   <221> source
   <223> /note="Description of Artificial Sequence: Synthetic primer"
<400> 31
   gcaggcctaa aataccttgg atcta 25
<210> 32
   <211> 16
   <212> DNA
   <213> Artificial Sequence
<220>
   <221> source
   <223> /note="Description of Artificial Sequence: Synthetic probe"
<400> 32
   ccgggcgatg taatta 16
<210> 33
   <211> 23
   <212> DNA
   <213> Artificial Sequence
<220>
   <221> source
   <223> /note="Description of Artificial Sequence: Synthetic primer"
<400> 33
   cttcctgcaa cttgcaactt gaa 23
<210> 34
   <211> 23
   <212> DNA
   <213> Artificial Sequence
<220>
   <221> source
   <223> /note="Description of Artificial Sequence: Synthetic primer"
<400> 34
   ctcgcctgat cgacaacaaa atg 23
<210> 35
   <211> 24
   <212> DNA
   <213> Artificial Sequence
<220>
   <221> source
   <223> /note="Description of Artificial Sequence: Synthetic probe"
<400> 35
   agctggcgta atacttatac tcta 24
<210> 36
   <211> 29
   <212> DNA
   <213> Artificial Sequence
<220>
   <221> source
   <223> /note="Description of Artificial Sequence: Synthetic primer"
<400> 36
   tgacagtatc attacaaagc caaatcagt 29
<210> 37
   <211> 21
   <212> DNA
   <213> Artificial Sequence
<220>
   <221> source
   <223> /note="Description of Artificial Sequence: Synthetic primer"
<400> 37
   ggccactacg cccttaatct c 21
<210> 38
   <211> 22
   <212> DNA
   <213> Artificial Sequence
<220>
   <221> source
   <223> /note="Description of Artificial Sequence: Synthetic probe"
<400> 38
   actgaataaa gagttaaacg cc 22

## Description

### FIELD

The present teachings, in some embodiments, relate to compositions, methods and kits for detection of one or multiple microorganism contaminants in samples. Some embodiments describe compositions, methods and kits for detecting one or more microorganisms producing virulence factors such as shiga toxin or *eae.* In some embodiments, compositions, methods and kits can further identify and detect individual strains and serotypes of shiga toxin-producing microorganisms. Workflows for multiple microbe detection and identification are also described.

### BACKGROUND

Identification of bacterial contamination in food often occurs subsequent to an outbreak of a foodborne illness. Bacteria such as *Escherichia coli* (*E. coli*) and *Shigella* (*Shigella spp*.) are frequently identified as a food contaminant of many foodborne illnesses. Some *E. coli and Shigella spp.* organisms produce a toxin called shiga toxin.

*Shigella spp.* organisms, if consumed in contaminated food, cause shigellosis which is often characterized by dysentery, nausea, fever, vomiting and stomach cramps.

Shiga toxin-producing *E*. *coli* (STEC), sometimes also referred to as verotoxin-producing *E. coli* (VTEC), are found in undercooked meats, raw milk, and contaminated water. These bacteria can cause illness ranging from mild intestinal diseases to bloody diarrhea to life-threatening kidney failure caused by a condition called Hemolytic Uremic Syndrome (HUS).

An *E. coli* serotype known as *E. coli* 0157:H7 produces shiga toxin and is most often associated with outbreaks of foodborne illness in the United States and elsewhere in the world and causes enterohemorrhagic colitis and possibly kidney failure. Detection of pathogenic *E*. *coli,* particularly serotypes causative of hemorrhagic colitis and HUS has become a public health priority. Regulations by the United States government require meat and other food processors to screen for the presence of strains such as O157:H7 in their finished products and more stringent guidelines are being considered in a number of states for the identification of O157:H7 in other commodities and foodstuffs.

As a result of repeated outbreaks and the life-threatening nature of STEC contamination, government public health agencies are now requiring testing of multiple STEC microbes to ensure food safety. Specifically, the USDA-FSIS has identified six O-serotypes of *E. coli* including O26, O45, O103, O111, O121, O145 for future testing while the European Food Safety Association (EFSA) has required testing of four of these *E. coli* serotypes including O26, O103, O111, O145. However, rapid methods for testing and identifying one or more STEC organisms are still lacking.

US2010/267012, CN101113475, SHARMA V K ET AL, "Semi-automated fluorogenic PCR assays (TaqMan) for rapid detection of Escherichia coli 0157:H7 and other Shiga toxigenic E. coli", MOLECULAR AND CELLULAR PROBES, ACADEMIC PRESS, LONDON, GB, (19990801), vol. 13, no. 4, pages 291 - 302, and IBEKWE A M ET AL, "Multiplex fluorogenic real-time PCR for detection and quantification of Escherichia coli O157:H7 in dairy wastewater wetlands", APPLIED AND ENVIRONMENTAL MICROBIOLOGY, AMERICAN SOCIETY FOR MICROBIOLOGY, US, (20021001), vol. 68, no. 10, pages 4853 -4862 disclose multiplex PCR assays targeting, among others, the stx1 gene. Assays for the rapid, sensitive, and specific detection of infectious pathogens are extremely important from both a public health and economic perspective. There exists a need for novel assays and methods for detecting and differentiating pathogenic organisms from other pathogenic and non-pathogenic organisms to test food and other samples, and identification of pathogens for example to determine a pathogenic contaminant in a sample and/or to make a differential diagnosis of what microbe is causing a particular disease.

### SUMMARY

According to the present invention there is provided methods and kits for detection of one or more microorganisms and/or strains and/or serotypes thereof expressing one or more virulence factors (e.g., a microbe that express virulence factors such as shiga toxin and/or eae) according to the amended claims.

Some embodiments relate to compositions comprising isolated nucleic acid sequences that are operable to hybridize to nucleic acid regions that are uniquely found in a microbe that expresses a virulence factor. Nucleic acid regions corresponding to one or more virulence factors and uniquely found in microorganisms that express the virulence factor are referred to herein as virulence-factor specific target nucleic acids.

Virulence factors, in some embodiments, comprise one or more genes, alleles and variants thereof encoding for a shiga toxin. In some embodiments, compositions comprising primer and/or probe nucleic acid sequences that are specific to hybridize to a *stx* (shiga toxin) gene, allele, fragment and/or complement thereof are described, comprising isolated nucleic acid sequences having the nucleotide sequence of SEQ ID NOS: 1-10, complements thereof and sequences having about 90% identity to the foregoing sequences.

In some embodiments, detection of virulence factors in a microorganism comprises detection of one or more genes, alleles and/or portions and/or fragments and/or complements thereof encoding a shiga toxin. Isolated nucleic acid compositions are described here as primers and probes having SEQ ID NOS: 1-10 that are specific to hybridize to a shiga toxin-specific target nucleic acid sequence, including but not limited to, an *stx* gene, allele, fragment, variant and/or complements thereof as well as an amplified fragment of any of the foregoing. These compositions can be used in detection methods described herein to detect the presence of a shiga toxin encoding/expressing microbe. Microorganisms of different species and strains that encode a shiga toxin virulence factor, including alleles *stxl* and *stx2,* can be detected by compositions and methods described herein. Non-limiting examples of microorganisms detected may include various shiga toxin producing *E. coli spp.* and *Shigella spp.*

In some embodiments, a virulence factor that can be detected by the present compositions and methods comprises one or more genes, alleles and variants thereof encoding for an *eae* gene. In some embodiments, detection of virulence factors may comprise detection of one or more *eae* encoding nucleic, an allele of an *eae* gene, or a fragments thereof. Isolated nucleic acid compositions are described here as primers and probes having SEQ ID NOS: 11-14 that are specific to hybridize to an eae-specific target nucleic acid sequence, including but not limited to, an *eae* gene, allele, fragment, variant and/or complements thereof as well as an amplified fragment of any of the foregoing. These compositions can be used in detection methods described herein to detect the presence of an *eae* encoding/expressing microbe. Microorganisms of different species and strains that encode/express an *eae* virulence factor, including alleles and variants of *eae,* can be detected by compositions and methods described herein. Non-limiting examples of microorganisms detected may include various *eae* expressing *E. coli spp.* and *Shigella spp.*

Some embodiments describe compositions comprising primer sequences and/or probe sequences specific to hybridize to an *eae* gene, allele, and/or fragment thereof comprising isolated nucleic acid sequences having the nucleotide sequence of SEQ ID NOS: 11-14, complements thereof and sequences having about 90% identity to the foregoing sequences. SEQ ID NOS: 11-14 can be used in detection methods described herein to detect the presence of an *eae* encoding microbe.

Some embodiments describe oligonucleotide primers for use in detection of virulence factors, the set of oligonucleotide primers comprising: at least one primer set, each set having at least a forward primer and at least a reverse primer, that are operable to hybridize to virulence factors such as one or more variants of shiga toxin encoding nucleic acids, alleles, complements and fragments thereof and/or eae encoding nucleic acids, alleles, complements and fragments thereof.

Some embodiments describe a set of oligonucleotide primers for simultaneous use in a multiplex PCR process for the detection of virulence factors, the set of oligonucleotide primers comprising: at least two primer sets, each set having at least a forward primer and at least a reverse primer, that are operable to hybridize to virulence factors comprising all variants of shiga toxin encoding nucleic acids, alleles and fragments thereof. Primer sets may be directed towards a *stx* gene. An *stx* gene may be a *stx1* gene, a *stx2* gene, an allele of an *stx1* gene an allele of an *stx2* gene, or a fragment thereof. Primer sets in some embodiments may be degenerate primers.

Some embodiments of the present specification relate to compositions comprising isolated nucleic acid sequences that are operable to hybridize to nucleic acid regions that are uniquely found in a STEC microorganism. Nucleic acid regions uniquely found in a STEC microorganism specific are referred to herein as microorganism-specific target nucleic acids or STEC microorganism-specific target nucleic acids.

In some embodiments, the present disclosure describes isolated nucleic acid sequence comprising SEQ ID NO:15-SEQ ID NO:32, fragments thereof, complements thereof, sequences comprising at least 90% nucleic acid sequence identity thereto, labeled derivatives, and chemical and/or biological derivatives thereof. These isolated nucleic acids are primer and/or probe sequences that can hybridize to STEC microorganism specific target nucleic acids and can amplify and/or detect STEC-specific target nucleic acids under appropriate amplification and/or hybridization conditions.

Fragments of nucleic acid sequences described herein include without limitation nucleic acids having at least 10, at least 15, or at least 20 contiguous nucleic acids of a nucleic acid sequence as described herein. Nucleic acid sequences of the disclosure, in some embodiments, are primers and/or probes. In some embodiments, primers of the disclosure may be degenerate primers. Primers and probes may be labeled. In some embodiments, isolated nucleic acid sequence compositions of the disclosure may comprise one or more label, such as, but not limited to, a dye, a radioactive isotope, a chemiluminescent label, a fluorescent moiety, a bioluminescent label, an enzyme, and combinations thereof. In some embodiments primers and probes may be chemical and/or biological derivatives of the sequences described here.

The present application describes compositions and methods for detecting one or more microorganisms in samples. One or more microorganism that may be detected by a method of this disclosure may be different strains of *E. coli* such as an *E. coli* O157:H7, an *E*. *coli* O26, an *E. coli* O45, an *E. coli* O103, an *E. coli* O111, an *E. coli* O121 and an *E. coli* O145 and/or a *Shigella spp.* organism. Some methods disclosed here are singleplex methods and some methods disclosed herein are multiplex methods.

Samples that can be tested by methods of the disclosure to detect a microbial contaminant therein include but are not limited to a food sample (processed food samples and raw food samples), a beverage sample, an agricultural sample, a produce sample, an animal sample, a clinical sample, an environmental sample, a biological sample, a water sample and an air sample.

Some embodiments describe methods for detecting a shiga toxin producing *E*. *Coli* (STEC) microorganism in a sample comprising: hybridizing at least a first pair of nucleic acid amplification primers to a STEC target nucleic acid, a fragment thereof, a complements thereof, an allele thereof, or a variant thereof, to at least a first target polynucleotide sequence present in the sample; amplifying at least the first target polynucleotide sequence or a fragment or a complement thereof to obtain at least one amplified target polynucleotide sequence; and detecting the at least one amplified target polynucleotide sequence; wherein detection of the at least one amplified target polynucleotide sequence is indicative of the presence of an STEC microorganism in the sample. Exemplary STEC organisms detected are *E. coli* 026, *E. coli* 045, *E. coli* 0103, *E. coli* O111, *E. coli* 0121 and/or *E. coli* 0145.

Some embodiments describe methods for detection of an *E. coli* 0121 in a sample comprising: hybridizing at least a first pair of nucleic acid amplification primers comprising nucleic acids of SEQ ID NO: 15 and SEQ ID NO: 16, fragments having at least 10 contiguous nucleotides thereof, complements thereof, and sequences having at least 90% homology thereto, to at least a first target polynucleotide sequence present in the sample; amplifying at least the first target polynucleotide sequence or a fragment or a complement thereof to obtain at least one amplified target polynucleotide sequence; and detecting the at least one amplified target polynucleotide sequence; wherein detection of the at least one amplified target polynucleotide sequence is indicative of the presence of an *E. coli* 0121 in the sample. A method to detect *E. coli* 0121 may further comprise using a probe to detect the at least one amplified target polynucleotide sequence wherein the probe comprises a sequence of SEQ ID NO: 17, fragments having at least 10 contiguous nucleotides thereof, complements thereof, and sequences having at least 90% homology thereto.

Some embodiments describe methods for detection of an *E. coli* 0145 in a sample comprising: hybridizing at least a first pair of nucleic acid amplification primers comprising nucleic acids of SEQ ID NO: 18 and SEQ ID NO: 19, fragments having at least 10 contiguous nucleotides thereof, complements thereof, and sequences having at least 90% homology thereto, to at least a first target polynucleotide sequence present in the sample; amplifying at least the first target polynucleotide sequence or a fragment or a complement thereof to obtain at least one amplified target polynucleotide sequence; and detecting the at least one amplified target polynucleotide sequence; wherein detection of the at least one amplified target polynucleotide sequence is indicative of the presence of an *E. coli* 0145 in the sample. A method to detect an *E. coli* 0145 may further comprise using a probe to detect the at least one amplified target polynucleotide sequence wherein the probe comprises a sequence of SEQ ID NO: 20, fragments having at least 10 contiguous nucleotides thereof, complements thereof, and sequences having at least 90% homology thereto.

Some embodiments describe methods for detection of an *E. coli* 026 in a sample and comprise: hybridizing at least a first pair of nucleic acid amplification primers comprising nucleic acids of SEQ ID NO: 21 and SEQ ID NO: 22, fragments having at least 10 contiguous nucleotides thereof, complements thereof, and sequences having at least 90% homology thereto, to at least a first target polynucleotide sequence present in the sample; amplifying at least the first target polynucleotide sequence or a fragment or a complement thereof to obtain an amplified target polynucleotide sequence; and detecting the at least one amplified target polynucleotide sequence; wherein detection of the at least one amplified target polynucleotide sequence is indicative of the presence of an *E. coli* 026 in the sample. A method to detect an *E. coli* 026 may further comprise using a probe to detect the at least one amplified target polynucleotide sequence wherein the probe comprises a sequence of SEQ ID NO: 23, fragments having at least 10 contiguous nucleotides thereof, complements thereof, and sequences having at least 90% homology thereto.

Some embodiments describe methods for detection of an *E. coli* 045 in a sample comprising: hybridizing at least a first pair of nucleic acid amplification primers comprising nucleic acids of SEQ ID NO: 24 and SEQ ID NO: 25, fragments having at least 10 contiguous nucleotides thereof, complements thereof, and sequences having at least 90% homology thereto, to at least a first target polynucleotide sequence present in the sample; amplifying at least the first target polynucleotide sequence or a fragment or a complement thereof to obtain an amplified target polynucleotide sequence; and detecting the at least one amplified target polynucleotide sequence; wherein detection of the at least one amplified target polynucleotide sequence is indicative of the presence of an *E. coli* 045 in the sample. A method to detect an *E. coli* 045 may further comprise using a probe to detect the at least one amplified target polynucleotide sequence wherein the probe comprises a sequence of SEQ ID NO: 26, fragments having at least 10 contiguous nucleotides thereof, complements thereof, and sequences having at least 90% homology thereto.

Some embodiments describe methods for detection of an *E. coli* 0103 in a sample comprising: hybridizing at least a first pair of nucleic acid amplification primers comprising nucleic acids of SEQ ID NO: 27 and SEQ ID NO: 28, complements thereof, and sequences having at least 90% homology thereto, to at least a first target polynucleotide sequence present in the sample; amplifying at least the first target polynucleotide sequence or a fragment or a complement thereof to obtain an amplified target polynucleotide sequence; and detecting the at least one amplified target polynucleotide sequence; wherein detection of the at least one amplified target polynucleotide sequence is indicative of the presence of an *E. coli* 0103 in the sample. A method to detect an *E. coli* 0103 may further comprise using a probe to detect the at least one amplified target polynucleotide sequence wherein the probe comprises a sequence of SEQ ID NO: 29, fragments having at least 10 contiguous nucleotides thereof, complements thereof, and sequences having at least 90% homology thereto.

Some embodiments describe methods for detection of an *E. coli* O111 in a sample comprising: hybridizing at least a first pair of nucleic acid amplification primers comprising nucleic acids of SEQ ID NO: 30 and SEQ ID NO: 31, fragments having at least 10 contiguous nucleotides thereof, complements thereof, and sequences having at least 90% homology thereto, to at least a first target polynucleotide sequence present in the sample; amplifying at least the first target polynucleotide sequence or a fragment or a complement thereof to obtain an amplified target polynucleotide sequence; and detecting the at least one amplified target polynucleotide sequence; wherein detection of the at least one amplified target polynucleotide sequence is indicative of the presence of an *E. coli* O111 in the sample. A method to detect an *E. coli* O111 may further comprise using a probe to detect the at least one amplified target polynucleotide sequence wherein the probe comprises a sequence of SEQ ID NO: 32, fragments having at least 10 contiguous nucleotides thereof, complements thereof, and sequences having at least 90% homology thereto.

The specification, in some embodiments, describes method for detection of one or more microorganisms, each microorganism expressing one or more virulence factors comprising: 1) detecting the presence of one or more virulence factors comprising: a) contacting a sample suspected of containing one or more microorganisms expressing one or more virulence factors with one or more sets of oligonucleotide primers specific to hybridize to portions of or portions adjoining one or more virulence factors; b) amplifying at least one target nucleic acid sequence encoding at least one of the virulence factors by a multiplex amplification method to obtain one or more amplified virulence factor specific nucleic acids; c) detecting the one or more amplified virulence factor specific nucleic acids or fragments or complements thereof; and d) optionally identifying the amplified virulence factor specific nucleic acids, wherein detecting the one or more amplified virulence factor specific nucleic acids or fragments or complements thereof is indicative of the presence of a microorganism in the sample expressing the one or more virulence factor; and 2) determining the strain of the one or more microorganisms expressing the one or more virulence factors comprising: a) contacting the sample with at least one set of oligonucleotide primers specific to hybridize to a target specific to a strain of the one or more microorganism; b) co-amplifying at least one strain specific target nucleic acid sequence encoding for nucleic acid targets specific to at least one strain of the microorganism by a multiplex amplification method to obtain one or more amplified strain specific nucleic acids; c) detecting the one or more amplified strain-specific nucleic acid or fragments or complements thereof; and d) optionally identifying the amplified strain-specific nucleic acids. In some embodiments of the method each set of oligonucleotide primers specific to the one or more virulence factors is labeled with a different label. In some embodiments of the method, each set of oligonucleotide primers specific to the one or more strain of the microorganism is labeled with a different label and wherein each set of oligonucleotide primers specific to the one or more virulence factors is labeled with a different label.

In some embodiments, steps described in the method above of 1) detecting presence of one or more virulence factors and 2) determining strain of the one or more microorganism expressing the one or more virulence factor can be performed simultaneously, in a sequential manner and/or in any order.

In some embodiments of a method for detection of one or more microorganisms expressing one or more virulence factors, the one or more sets of oligonucleotide primers specific to the one or more virulence factors each comprise: at least one forward primer and at least one reverse primer operable to hybridize to one or more virulence factors. Virulence factors in some embodiments comprise but are not limited to one or more of the following: all variants of shiga toxin (*stx*) encoding nucleic acids, alleles and fragments thereof; and/or all variants of *eae* gene encoding nucleic acids, alleles and fragments thereof. A *stx*-encoding nucleic acid may comprise an *stx1* gene, a *stx2* gene, an allele of an *stx1* gene an allele of an *stx2* gene, or a fragments thereof. For example, see Table 1 which lists several *stx1* and *stx2* subtypes and variants. An *eae* encoding nucleic acids may comprise an *eae* gene, an allele of an *eae* gene, or a fragments thereof. There are over 25 defined allele of the *eae* gene, which encodes the intimin protein. See http:Honlinelibrary.wiley.com/doi/10.1111/j.1574-6968.2006.00328.x/full for more details.

Microorganisms expressing one or more virulence factors include for example Shiga toxin-producing *E. coli* (STEC) and *Shigella spp.*

In some embodiments of a method described above, one or more oligonucleotide primer sets specific for one or more virulence factors comprise and can be selected from: a first primer set having SEQ ID NO: 1 and SEQ ID NO:2; a second primer set having SEQ ID NO: 4 and SEQ ID NO:5; a third primer set having SEQ ID NO: 6 and SEQ ID NO:7; a fourth primer set having SEQ ID NO: 8 and SEQ ID NO: 9; and/or a fifth primer set having SEQ ID NO: 11, SEQ ID NO: 12; and SEQ ID NO: 13. A method may further comprise the steps of detecting the one or more amplified virulence factor specific nucleic acids or fragments or complements thereof comprises hybridizing the one or more amplified virulence factor specific nucleic acids or fragments or complements thereof with a probe selected from SEQ ID NO: 3, SEQ ID NO: 10, and/or SEQ ID NO: 14.

In some embodiments, not detecting any amplified product using methods described above can be used to exclude the presence of a microorganism expressing a virulence factor in a sample.

In some embodiments of a method as described above, the at least one set of oligonucleotide primers specific to the strain of the one or more microorganism comprises and can be selected from: a first primer set having SEQ ID NO: 15 and SEQ ID NO:16; a second primer set having SEQ ID NO: 18 and SEQ ID NO:19; a third primer set having SEQ ID NO: 21 and SEQ ID NO: 22; a fourth primer set having SEQ ID NO: 24 and SEQ ID NO: 25, a fifth primer set having SEQ ID NO: 27, SEQ ID NO: 28; a sixth primer set having SEQ ID NO: 30 and SEQ ID NO: 31; a seventh primer set having SEQ ID NO: 33 and SEQ ID NO:34; and/or an eighth primer set having SEQ ID NO: 36 and SEQ ID NO: 37. In some embodiments, the steps of detecting the one or more amplified strain specific nucleic acids or fragments or complements thereof comprises hybridizing the one or more amplified strain specific nucleic acids or fragments or complements thereof with a probe selected from SEQ ID NO: 17, SEQ ID NO: 20, SEQ ID NO: 23, SEQ ID NO: 26, SEQ ID NO: 29, SEQ ID NO: 32, SEQ ID NO: 35, and SEQ ID NO: 38.

In some embodiments, not detecting any amplified product using methods described above may be used to exclude the presence of a microorganism of a particular strain in a sample.

In some embodiments, detecting an amplified nucleic acid comprises one or more methods such as but not limited to hybridization, mass spectrometry, nanostring, microfluidics, chemiluminescence, enzyme technologies and combinations thereof. Some embodiments may also comprise identifying a microbe and may comprise methods such as DNA sequencing. In some embodiment methods described here, nucleic acids (RNA/DNA) can be extracted from a sample suspected to contain a microorganism to be detected prior to amplification or detection.

In some embodiments, the disclosure describes methods for detecting and identifying one or more Shiga toxin-producing *E. coli* (STEC) microorganisms in a sample comprising the steps of: a) optionally enriching the STEC microorganisms; b) extracting nucleic acids from (STEC microorganisms and others present in) the sample; c) performing a multiplex polymerase chain reaction (PCR) on the extracted sample nucleic acids to amplify and detect the presence of at least one nucleic acids selected from: nucleic acids encoding for a shiga toxin gene, including nucleic acids encoding *stx1* and *stx2;* nucleic acids encoding an *eae* gene; and nucleic acids specific for an *E. coli* strain 0157:H7, wherein the detection of at least one nucleic acid above indicates the presence of an STEC microorganism or the presence of an *E. coli* 0157:H7 microorganism or both; and d) if a nucleic acid is detected in step c) performing a second round of multiplex PCR with primers specific to amplify and detect the presence one or more nucleic acids encoding for target regions associated with one or more STEC microorganisms including an *E. coli* O157:H7, an *E. coli* 026, an *E. coli* 045, an *E. coli* 0103, an *E. coli* O111, an *E. coli* 0121 and an *E. coli* 0145, thereby detecting or identifying the strain of the STEC microorganism.

In other embodiments, not detecting any amplified product using the methods described above may be used to exclude the presence of a STEC microorganism in a sample.

Amplified target nucleic acids of targets including *stx1, stx2, eae, E. coli* O157:H7, an *E. coli* O26, an *E. coli* O45, an *E. coli* O103, an *E. coli* O111, an *E. coli* 0121 and/or an *E. coli* 0145 amplified target nucleic acid can be detected by using a plurality of labels on each different set of primers and/or probes. For example, in step c) above primers and/or probes with a different label for each of *stxl,* sxt2, *eae* and *E. coli* 0157:H7 can be used and in step d) primers and/or a probes with a different label each can be used for the various microorganisms.

In some embodiments, methods of the disclosure can be performed on an automated system. Automation decreases the time as well as efficiency and allows processing multiples samples. Automated systems may comprise magnetic platforms such as but not limited to MagMAX™ Express-96 Magnetic Particle Processor by Life Technologies Corporation and Pathatrix system (http://www.matrixmsci.com/pathatrix.htm).

Detection in the methods above may be performed by a variety of methods, such as but not limited to, by a nucleic acid amplification reaction, the amplification reaction is an end-point determination, the amplification reaction is quantitative, the quantification is a real-time PCR, the real-time PCR is a SYBR® Green Assay or the real-time PCR is a TaqMan® Assay. Detection may in some embodiments be performed by hybridization using probes specific to amplified nucleic acid sequences encoding a target sequence. Combinations of amplification and hybridization may be used for detection according to some embodiments.

In some embodiments, hybridization may comprise at least a first probe and a second probe, the first probe further comprising a first label and said second probe further comprising a second label, wherein both labels are selected from a dye, a radioactive isotope, a chemiluminescent label, and an enzyme, the dye comprises a fluorescein dye, a rhodamine dye, or a cyanine dye, the dye is a fluorescein dye and first probe is labeled with FAM™ dye and said second probe is labeled with VIC® dye.

Some embodiments describe kits suitable for identifying the presence of a shiga toxin producing organism. Such a kit may comprise at least one set of oligonucleotide primers for use in an amplification process (such as PCR and in some embodiments a multiplex PCR) for the detection of *stx* virulence factors, the set of primers comprising primers operable to hybridize to virulence factors comprising all variants, genes, allele and/or fragments and complements of shiga toxin. A kit may further comprise probe sequences to detect amplified *stx* target nucleic acids. In one example embodiment, a kit for detecting microorganisms expressing *stx* comprises primers and probes having SEQ ID NOS: 1-10.

Some embodiments describe kits suitable for identifying the presence of an *eae* encoding organism. Such a kit may comprise at least one set of oligonucleotide primers for use in a PCR process for the detection of an *eae* virulence factor, the set of primers comprising a primers operable to hybridize to *eae* encoding genes, alleles, complementary regions thereof and fragments thereof comprising all variants of *eae.* A kit may further comprise probe sequences to detect amplified *eae* target nucleic acids. In one example embodiment, a kit for detecting microorganisms expressing *eae* comprises primers and probes having SEQ ID NOS: 11-14.

Some kits of the disclosure can detect virulence factors including *stx* and *eae* and comprise primer sets selected from: a first primer set having SEQ ID NO: 1 and SEQ ID NO: 2; a second primer set having SEQ ID NO:4 and SEQ ID NO:5; a third primer set having SEQ ID NO: 6 and SEQ ID NO: 7; an fourth primer set having SEQ ID NO:8 and SEQ ID NO:9; a fifth primer set having as forward primer SEQ ID NO: 11 and as reverse primers SEQ ID NO: 12 or SEQ ID NO: 13; and further optionally additionally at least one more probe selected from probes having SEQ ID NO: 3, SEQ ID NO: 10, and SEQ ID NO: 14, or sequences complementary thereto, or sequences comprising at least 90% nucleic acid sequence identity thereof, or a labeled derivatives thereof.

In some embodiments, kits for detection of STEC organisms including an *E*. *coli* 026, an *E. coli* 045, an *E. coli* 0103, an *E. coli* O111, an *E. coli* 0121 and an *E. coli* 0145 are described. In one example embodiment, kits for detection of a Shiga toxin-producing *E. coli* (STEC) microorganisms comprise: one or more pairs of forward and reverse polymerase chain reaction (PCR) primers selected from a first primer set having SEQ ID NO: 15 and SEQ ID NO:16; a second primer set having SEQ ID NO: 18 and SEQ ID NO:19; a third primer set having SEQ ID NO:21 and SEQ ID NO:22; a fourth primer set having SEQ ID NO: 24 and SEQ ID NO:25, a fifth primer set having SEQ ID NO:27 and SEQ ID NO:28; a sixth primer set having SEQ ID NO: 30 and SEQ ID NO:31, or sequences complementary thereto, or sequences comprising at least 90% nucleic acid sequence identity thereto, fragments having at least 10 contiguous nucleotides thereof, or a labeled derivative thereof; optionally at least one probe selected from SEQ ID NO: 17, SEQ ID NO:20, SEQ ID NO:23, SEQ ID NO:26, SEQ ID NO:29 and SEQ ID NO: 32 or sequences complementary thereto, or sequences comprising at least 90% nucleic acid sequence identity thereof, fragments having at least 10 contiguous nucleotides thereof, or a labeled derivative thereof; and one or more components selected from a group consisting of: at least one enzyme; dNTPs, at least one buffer, at least one salt, at least one control nucleic acid sample and an instruction protocol. The kit described above can be used to detect one or more STEC organisms including an an *E. coli* 026, an *E. coli* 045, an *E. coli* 0103, an *E. coli* O111, an *E. coli* 0121 and an *E. coli* 0145 are described.

In some embodiments, the kit described above can also comprise primers and probes to detect a virulence factor. Accordingly, a kit of the disclosure can also comprise one or more pairs of forward and reverse PCR primers further selected from a seventh primer set having SEQ ID NO: 11 and SEQ ID NO: 12 and SEQ ID NO: 13; an eighth primer set having SEQ ID NO: 1 and SEQ ID NO: 2; a ninth primer set having SEQ ID NO:4 and SEQ ID NO:5; a tenth primer set having SEQ ID NO: 6 and SEQ ID NO: 7; an eleventh primer set having SEQ ID NO:8 and SEQ ID NO:9; and further optionally additionally at least one more probe selected from probes having SEQ ID NO: 14, SEQ ID NO: 3, SEQ ID NO: 10, or sequences complementary thereto, or sequences comprising at least 90% nucleic acid sequence identity thereof, or a labeled derivatives thereof.

In some embodiments, kits of the disclosure may further comprise primers to hybridize to, amplify and thereby detect an *E. coli* O157:H7. Such a kit can additionally comprise one or more pairs of forward and reverse PCR primers further selected from a twelfth primer set having SEQ ID NO: 33 and SEQ ID NO: 34; a thirteenth primer set having SEQ ID NO: 36 and SEQ ID NO: 37; and optionally additionally at least one more probe further selected from probes having SEQ ID NO: 35, SEQ ID NO: 38, or sequences complementary thereto, or sequences comprising at least 90% nucleic acid sequence identity thereof, or labeled derivatives thereof.

Different primers and probes may be labeled with different labels to allow for detection of different amplified products and/or hybridized products.

In the following detailed description, certain aspects and embodiments will become evident. It should be understood that a given embodiment need not have all aspects and features described herein. It should be understood that these aspects and embodiments are merely exemplary and explanatory and are not restrictive of the invention. It is to be understood that both the foregoing general description and the following detailed description are exemplary and explanatory only and are not restrictive of the invention, as claimed.

The accompanying figures, which are incorporated in and constitute a part of this specification, illustrate several exemplary embodiments of the disclosure and together with the description, serve to explain certain teachings. These and other features of the present teachings are set forth herein.

### DESCRIPTION OF THE DRAWINGS

The skilled artisan will understand that the drawings described below are for illustration purposes only. The drawings are not intended to limit the scope of the present teachings in any way.
FIG. 1 illustrates results of a multiplex assay using *E. coli* O157:H7, according to one embodiment of the disclosure;
FIG. 2 illustrates results of a multiplex assay using *E. coli* O104:H4, according to one embodiment of the disclosure;
FIG. 3 illustrates detection of *stx*1 from a panel of 161 bacteria strains (146 *E*. *coli* and 15 non-*E*. *coli* strains), according to one embodiment of the disclosure;
FIG. 4 illustrates detection of *stx2* from a panel of 161 bacteria strains (146 *E*. *coli* and 15 non-*E*. *coli* strains), according to one embodiment of the disclosure;
FIGS 5A and 5B illustrate *eae* inclusion and exclusion testing, according to one embodiment of the disclosure;
FIG. 6A and 6B illustrates results of a multiplex assay versus a single-plex assay, according to one embodiment; and

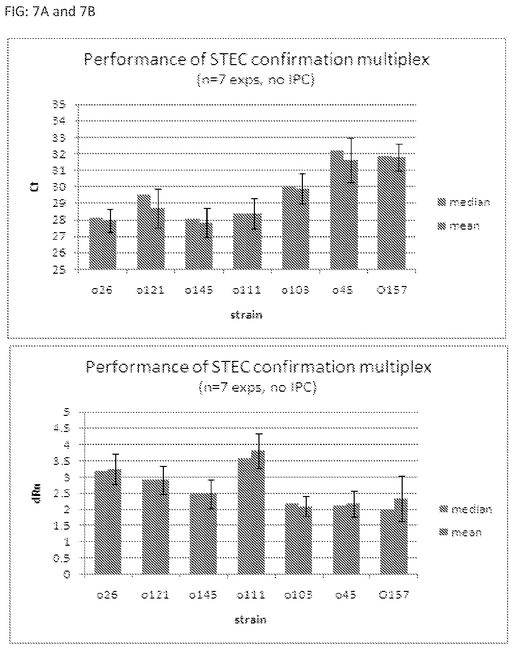

FIG 7A and 7B show results of STEC confirmation assays according to one embodiment.

### DETAILED DESCRIPTION

For the purposes of interpreting of this specification, the following definitions may apply and whenever appropriate, terms used in the singular will also include the plural and vice versa. In the event that any definition set forth below conflicts with the usage of that word in any other document, the definition set forth below shall always control for purposes of interpreting this specification and its associated claims unless a contrary meaning is clearly intended (for example in the document where the term is originally used). It is noted that, as used in this specification and the appended claims, the singular forms "a," "an," and "the," include plural referents unless expressly and unequivocally limited to one referent. The use of "or" means "and/or" unless stated otherwise. The use of "comprise," "comprises," "comprising," "include," "includes," and "including" are interchangeable and not intended to be limiting. Furthermore, where the description of one or more embodiments uses the term "comprising," those skilled in the art would understand that, in some specific instances, the embodiment or embodiments can be alternatively described using the language "consisting essentially of" and/or "consisting of."

As used herein, the phrase "nucleic acid," "nucleic acid sequence," "oligonucleotide", and "polynucleotides" are interchangeable and not intended to be limiting.

As used herein, the phrase "stringent hybridization conditions" refers to hybridization conditions which can take place under a number of pH, salt and temperature conditions. The pH can vary from 6 to 9, preferably 6.8 to 8.5. The salt concentration can vary from 0.15 M sodium to 0.9 M sodium, and other cations can be used as long as the ionic strength is equivalent to that specified for sodium. The temperature of the hybridization reaction can vary from 30°C to 80°C, preferably from 45°C to 70°C. Additionally, other compounds can be added to a hybridization reaction to promote specific hybridization at lower temperatures, such as at or approaching room temperature. Among the compounds contemplated for lowering the temperature requirements is formamide. Thus, a polynucleotide is typically "substantially complementary" to a second polynucleotide if hybridization occurs between the polynucleotide and the second polynucleotide. As used herein, "specific hybridization" refers to hybridization between two polynucleotides under stringent hybridization conditions.

As used herein, the term "polynucleotide" refers to a polymeric form of nucleotides of any length, either ribonucleotides, deoxynucleotides, or peptide nucleic acids (PNA), and includes both double- and single-stranded RNA, DNA, and PNA. A polynucleotide may include nucleotide sequences having different functions, including, for instance, coding regions, and non-coding regions such as regulatory regions. A polynucleotide can be obtained directly from a natural source, or can be prepared with the aid of recombinant, enzymatic, or chemical techniques. A polynucleotide can be linear or circular in topology. A polynucleotide can be, for example, a portion of a vector, such as an expression or cloning vector, or a fragment. An "oligonucleotide" refers to a polynucleotide of the present invention, typically a primer and/or a probe.

As used herein a "target-specific polynucleotide" refers to a polynucleotide having a target-binding segment that is perfectly or substantially complementary to a target sequence, such that the polynucleotide binds specifically to an intended target without significant binding to non-target sequences under sufficiently stringent hybridization conditions. The target-specific polynucleotide can be e.g., a primer or probe and the subject of hybridization with its complementary target sequence.

The term "target sequence", "target signature sequence" "target nucleic acid", "target" or "target polynucleotide sequence" refers to a nucleic acid of interest. Example targets of interest in some embodiments of this application include *stx* encoding nucleic acids and fragments, complements and sequences with 90% homology thereto; eae encoding nucleic acids; nucleic acids specifically found in STEC organisms such as an *E. coli* O157:H7, an *E. coli* 026, an *E. coli* 045, an *E. coli* 0103, an *E. coli* O111, an *E. coli* 0121 and an *E. coli* 0145; and/or *Shigella spp.* specific nucleic acid sequences. The target sequence can be a polynucleotide sequence that is the subject of hybridization with a complementary polynucleotide, e.g. a primer or probe. The target sequence can be composed of DNA, RNA, an analog thereof, and including combinations thereof. The target sequence may be known or not known, in terms of its actual sequence and its amplification can be desired. The target sequence may or may not be of biological significance. Typically, though not always, it is the significance of the target sequence which is being studied in a particular experiment. As non-limiting examples, target sequences may include regions of genomic DNA, regions of genomic DNA which are believed to contain one or more polymorphic sites, DNA encoding or believed to encode genes or portions of genes of known or unknown function, DNA encoding or believed to encode proteins or portions of proteins of known or unknown function, DNA encoding or believed to encode regulatory regions such as promoter sequences, splicing signals, polyadenylation signals, etc.

As used herein an "amplified target polynucleotide sequence product" refers to the resulting amplicon from an amplification reaction such as a polymerase chain reaction. The resulting amplicon product arises from hybridization of complementary primers to a target polynucleotide sequence under suitable hybridization conditions and the repeating in a cyclic manner the polymerase chain reaction as catalyzed by DNA polymerase for DNA amplification or RNA polymerase for RNA amplification.

As used herein, the "polymerase chain reaction" or PCR is a an amplification of nucleic acid consisting of an initial denaturation step which separates the strands of a double stranded nucleic acid sample, followed by repetition of (i) an annealing step, which allows amplification primers to anneal specifically to positions flanking a target sequence; (ii) an extension step which extends the primers in a 5' to 3' direction thereby forming an amplicon polynucleotide complementary to the target sequence, and (iii) a denaturation step which causes the separation of the amplicon from the target sequence (Mullis et al., eds, The Polymerase Chain Reaction, BirkHauser, Boston, Mass. (1994). Each of the above steps may be conducted at a different temperature, preferably using an automated thermocycler (Applied Biosystems LLC, a division of Life Technologies Corporation, Foster City, CA.). If desired, RNA samples can be converted to DNA/RNA heteroduplexes or to duplex cDNA by methods known to one of skill in the art.

As used herein, "amplifying" and "amplification" refers to a broad range of techniques for increasing polynucleotide sequences, either linearly or exponentially. Exemplary amplification techniques include, but are not limited to, PCR or any other method employing a primer extension step. Other nonlimiting examples of amplification include, but are not limited to, ligase detection reaction (LDR) and ligase chain reaction (LCR). Amplification methods may comprise thermal-cycling or may be performed isothermally. In various embodiments, the term "amplification product" or "amplified product" includes products from any number of cycles of amplification reactions.

In certain embodiments, amplification methods comprise at least one cycle of amplification, for example, but not limited to, the sequential procedures of: hybridizing primers to primer-specific portions of target sequence or amplification products from any number of cycles of an amplification reaction; synthesizing a strand of nucleotides in a template-dependent manner using a polymerase; and denaturing the newly-formed nucleic acid duplex to separate the strands. The cycle may or may not be repeated.

Descriptions of certain amplification techniques can be found, among other places, in H. Ehrlich et al., Science, 252:1643-50 (1991), M. Innis et al., PCR Protocols: A Guide to Methods and Applications, Academic Press, New York, N.Y. (1990), R. Favis et al., Nature Biotechnology 18:561-64 (2000), and H. F. Rabenau et al., Infection 28:97-102 (2000); Sambrook and Russell, Molecular Cloning, Third Edition, Cold Spring Harbor Press (2000) (hereinafter "Sambrook and Russell"), Ausubel et al., Current Protocols in Molecular Biology (1993) including supplements through September 2005, John Wiley & Sons (hereinafter "Ausubel et al.").

The term "label" refers to any moiety which can be attached to a molecule and: (i) provides a detectable signal; (ii) interacts with a second label to modify the detectable signal provided by the second label, e.g. FRET; (iii) stabilizes hybridization, i.e. duplex formation; or (iv) provides a capture moiety, i.e. affinity, antibody/antigen, ionic complexation. Labeling can be accomplished using any one of a large number of known techniques employing known labels, linkages, linking groups, reagents, reaction conditions, and analysis and purification methods. Labels include light-emitting compounds which generate a detectable signal by fluorescence, chemiluminescence, or bioluminescence (Kricka, L. in Nonisotopic DNA Probe Techniques (1992), Academic Press, San Diego, pp. 3-28). Another class of labels are hybridization-stabilizing moieties which serve to enhance, stabilize, or influence hybridization of duplexes, e.g. intercalators, minor-groove binders, and cross-linking functional groups (Blackburn, G. and Gait, M. Eds. "DNA and RNA structure" in Nucleic Acids in Chemistry and Biology, 2.sup.nd Edition, (1996) Oxford University Press, pp. 15-81). Yet another class of labels effect the separation or immobilization of a molecule by specific or nonspecific capture, for example biotin, digoxigenin, and other haptens (Andrus, A. "Chemical methods for 5' non-isotopic labelling of PCR probes and primers" (1995) in PCR 2: A Practical Approach, Oxford University Press, Oxford, pp. 39-54).

The terms "annealing" and "hybridization" are used interchangeably and mean the base-pairing interaction of one nucleic acid with another nucleic acid that results in formation of a duplex or other higher-ordered structure. The primary interaction is base specific, i.e. A/T and G/C, by Watson/Crick and Hoogsteen-type hydrogen bonding.

The term "end-point analysis" refers to a method where data collection occurs only when a reaction is substantially complete.

The term "real-time analysis" refers to periodic monitoring during PCR. Certain systems such as the Applied Biosystems 7500 Real-Time PCR System (Applied Biosystems, Foster City, Calif.) conduct monitoring during each thermal cycle at a predetermined or user-defined point. Real-time analysis of PCR with FRET probes measures fluorescent dye signal changes from cycle-to-cycle, preferably minus any internal control signals.

The term "quenching" refers to a decrease in fluorescence of a first moiety (reporter dye) caused by a second moiety (quencher) regardless of the mechanism.

A "primer," as used herein, is an oligonucleotide that is complementary to a portion of target polynucleotide and, after hybridization to the target polynucleotide, may serve as a starting-point for an amplification reaction and the synthesis of an amplification product. Primers include, but are not limited to, spanning primers.A "primer pair" refers to two primers that can be used together for an amplification reaction. A "PCR primer" refers to a primer in a set of at least two primers that are capable of exponentially amplifying a target nucleic acid sequence in the polymerase chain reaction.

The term "probe" comprises a polynucleotide that comprises a specific portion designed to hybridize in a sequence-specific manner with a complementary region of a specific nucleic acid sequence, e.g., a target nucleic acid sequence. In certain embodiments, the specific portion of the probe may be specific for a particular sequence, or alternatively, may be degenerate, e.g., specific for a set of sequences. In certain embodiments, the probe is labeled. The probe can be an oligonucleotide that is complementary to at least a portion of an amplification product formed using two primers.

The terms "complement" and "complementary" as used herein, refer to the ability of two single stranded polynucleotides (for instance, a primer and a target polynucleotide) to base pair with each other, where an adenine on one strand of a polynucleotide will base pair to a thymine or uracil on a strand of a second polynucleotide and a cytosine on one strand of a polynucleotide will base pair to a guanine on a strand of a second polynucleotide. Two polynucleotides are complementary to each other when a nucleotide sequence in one polynucleotide can base pair with a nucleotide sequence in a second polynucleotide. For instance, 5'- ATGC and 5'-GCAT are complementary.

A "label" refers to a moiety attached (covalently or non-covalently), or capable of being attached, to an oligonucleotide, which provides or is capable of providing information about the oligonucleotide (e.g., descriptive or identifying information about the oligonucleotide) or another polynucleotide with which the labeled oligonucleotide interacts (e.g., hybridizes). Labels can be used to provide a detectable (and optionally quantifiable) signal. Labels can also be used to attach an oligonucleotide to a surface.

A "fluorophore" is a moiety that can emit light of a particular wavelength following absorbance of light of shorter wavelength. The wavelength of the light emitted by a particular fluorophore is characteristic of that fluorophore. Thus, a particular fluorophore can be detected by detecting light of an appropriate wavelength following excitation of the fluorophore with light of shorter wavelength.

The term "quencher" as used herein refers to a moiety that absorbs energy emitted from a fluorophore, or otherwise interferes with the ability of the fluorescent dye to emit light. A quencher can re-emit the energy absorbed from a fluorophore in a signal characteristic for that quencher, and thus a quencher can also act as a flourophore (a fluorescent quencher). This phenomenon is generally known as fluorescent resonance energy transfer (FRET). Alternatively, a quencher can dissipate the energy absorbed from a fluorophore as heat (a non-fluorescent quencher).

As used herein the term "sample" refers to a starting material suspected of harboring a particular microorganism or group of microorganisms. A "contaminated sample" refers to a sample harboring a pathogenic microbe thereby comprising nucleic acid material from the pathogenic microbe. Examples of samples include, but are not limited to, food samples (including but not limited to samples from food intended for human or animal consumption such as processed foods, raw food material, produce (*e.g.,* fruit and vegetables), legumes, meats (from livestock animals and/or game animals), fish, sea food, nuts, beverages, drinks, fermentation broths, and/or a selectively enriched food matrix comprising any of the above listed foods), water samples, environmental samples (*e*.*g*., soil samples, dirt samples, garbage samples, sewage samples, industrial effluent samples, air samples, or water samples from a variety of water bodies such as lakes, rivers, ponds *etc.*,), air samples (from the environment or from a room or a building), forensic samples, agricultural samples, pharmaceutical samples, biopharmaceutical samples, samples from food processing and manufacturing surfaces, and/or biological samples.

Disclosed are compositions, assays, methods and kits for the specific detection of microorganisms expressing virulence factors as well as STEC organisms from samples including clinical samples, food samples, complex food matrices, water, a beverage sample, a fermentation broth, a forensic sample, an environmental sample (e.g., soil, dirt, garbage, sewage, air, or water), including food processing and manufacturing surfaces, or a biological sample.

A sample may be tested directly, or may be prepared or processed in some manner prior to testing. For example, a sample may be processed to enrich any contaminating microbe and may be further processed to separate and/or lyse microbial cells contained therein. Lysed microbial cells from a sample may be additionally processed or prepares to separate, isolate and/or extract genetic material from the microbe for analysis to detect and/or identify the contaminating microbe. In some embodiments described here, as sample may be subject to separation to initially separate microbes of interest from other microbes and other sample components. For example, for complex food samples with complex components separation methods can be used to separate microorganisms from food. Separated microbes from samples can also be enriched prior to analysis. Analysis of a sample may include one or more molecular methods. For example, according to some exemplary embodiments of the present disclosure, a sample may be subject to nucleic acid amplification (for example by PCR) using appropriate oligonucleotide primers that are specific to one or more microbe nucleic acid sequences that the sample is suspected of being contaminated with. Amplification products may then be further subject to testing with specific probes (or reporter probes) to allow detection of microbial nucleic acid sequences that have been amplified from the sample. In some embodiments, if a microbial nucleic acid sequence is amplified from a sample, further analysis may be performed on the amplification product to further identify, quantify and analyze the detected microbe (determine parameters such as but not limited to the microbial strain, pathogenecity, quantity *etc.*)*.*

As used herein "preparing" or "preparing a sample" or "processing" or processing a sample" refers to one or more of the following steps to achieve separation of microbes from sample components and in some embodiments optionally extraction and/or separation of a nucleic acid from a sample: (1) optional separation of bacterial cells from sample components (such as a food sample), (2) optional bacterial enrichment, (3) optional cell lysis, and/or (4) optionally nucleic acid extraction and/or purification (*e*.*g*., DNA extraction, total nucleic acid extraction (i.e., DNA and RNA), genomic DNA extraction, RNA extraction). Types of nucleic acid extracted include, but are not limited to, DNA, RNA, mRNA and miRNA.

As used herein, "presence" refers to the existence (and therefore to the detection) of a reaction, a product of a method or a process (including but not limited to, an amplification product resulting from an amplification reaction), or to the "presence" and "detection" of an organism such as a pathogenic organism or a particular strain or species of an organism.

As used herein, "detecting" or "detection" refers to the disclosure or revelation of the presence or absence in a sample of a target polynucleotide sequence or amplified target polynucleotide sequence product. The detecting can be by end point, real-time, enzymatic, and by resolving the amplification product on a gel and determining whether the expected amplification product is present, or other methods known to one of skill in the art.

The presence or absence of an amplified product can be determined or its amount measured. Detecting an amplified product can be conducted by standard methods well known in the art and used routinely. The detecting may occur, for instance, after multiple amplification cycles have been run (typically referred to an end-point analysis), or during each amplification cycle (typically referred to as real-time). Detecting an amplification product after multiple amplification cycles have been run is easily accomplished by, for instance, resolving the amplification product on a gel and determining whether the expected amplification product is present. In order to facilitate real-time detection or quantification of the amplification products, one or more of the primers and/or probes used in the amplification reaction can be labeled, and various formats are available for generating a detectable signal that indicates an amplification product is present. For example, a convenient label is typically a label that is fluorescent, which may be used in various formats including, but are not limited to, the use of donor fluorophore labels, acceptor fluorophore labels, flourophores, quenchers, and combinations thereof. Assays using these various formats may include the use of one or more primers that are labeled (for instance, scorpions primers, amplifluor primers), one or more probes that are labeled (for instance, adjacent probes, TaqMan® probes, light-up probes, molecular beacons), or a combination thereof. The skilled person in view of the present teachings will understand that in addition to these known formats, new types of formats are routinely disclosed. The present invention is not limited by the type of method or the types of probes and/or primers used to detect an amplified product. Using appropriate labels (for example, different fluorophores) it is possible to combine (multiplex) the results of several different primer pairs (and, optionally, probes if they are present) in a single reaction. As an alternative to detection using a labeled primer and/or probe, an amplification product can be detected using a polynucleotide binding dye such as a fluorescent DNA binding dye. Examples include, for instance, SYBR® Green dye or SYBR® Gold dye (Molecular Probes). Upon interaction with the double-stranded amplification product, such polynucleotide binding dyes emit a fluorescence signal after excitation with light at a suitable wavelength. A polynucleotide binding dye such as a polynucleotide intercalating dye also can be used.

As used herein, a "target specific polynucleotide" refers to a nucleic acid sequence that is able to specifically hybridize to a gene and/or an allele and/or a portion thereof and/or a complement thereof that encodes a target (shiga toxin, *eae,* or a target specific to a big 6 pathogen or to *E. coli* O157:H7), under suitable hybridization conditions and which does not hybridize with other nucleic acid sequences that do not encode for the target or portions thereof or complements thereof. In some embodiments, a "target-specific polynucleotide" of the disclosure may be a probe or primer sequence described in SEQ ID NOS: 1-38. It is well within the ability of one skilled in the art, using the present teachings, to determine suitable hybridization conditions based on probe length, G+C content, and the degree of stringency required for a particular application.

It is expected that minor sequence variations in virulence factor specific target nucleotide sequences and microorganism specific target nucleotide sequences associated with nucleotide additions, deletions and mutations, whether naturally occurring or introduced *in vitro,* would not interfere with the usefulness of the various primer and probe nucleic acid sequences disclosed herein, as would be understood by one of skill in the art. Therefore, the scope of the present invention as claimed is intended to encompass minor variations in the sequences of described here and sequences having at least 90% homology to the primer and probe sequences disclosed herein.

A probe may be RNA or DNA. Depending on the detection means employed, the probe may be unlabeled, radiolabeled, chemiluminescent labeled, enzyme labeled, or labeled with a dye. The probe may be hybridized with a sample in solution or immobilized on a solid support such as nitrocellulose, a microarray or a nylon membrane, or the probe may be immobilized on a solid support, such as a silicon chip or a microarray.

Conditions that "allow" an event to occur or conditions that are "suitable" for an event to occur, such as hybridization, strand extension, and the like, or "suitable" conditions are conditions that do not prevent such events from occurring. Thus, these conditions permit, enhance, facilitate, and/or are conducive to the event. Such conditions, known in the art and described herein, may depend upon, for example, the nature of the nucleotide sequence, temperature, and buffer conditions. These conditions may also depend on what event is desired, such as hybridization, cleavage, or strand extension. An "isolated" polynucleotide refers to a polynucleotide that has been removed from its natural environment. A "purified" polynucleotide is one that is at least about 60% free, preferably at least about 75% free, and most preferably at least about 90% free from other components with which they are naturally associated.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

The terms "comprises" and variations thereof do not have a limiting meaning where these terms appear in the description and claims. Unless otherwise specified, "a," "an," "the," and "at least one" are used interchangeably and mean one or more than one.

Also herein, the recitations of numerical ranges by endpoints include all numbers subsumed within that range (e.g., 1 to 5 includes 1, 1.5, 2, 2.75, 3, 3.80, 4, 5, etc.). The term "and/or" means one or all of the listed elements or a combination of any two or more of the listed elements.

There are many known methods of amplifying nucleic acid sequences including e.g., PCR. See, e.g., PCR Technology: Principles and Applications for DNA Amplification (ed. H. A. Erlich, Freeman Press, NY, N.Y., 1992); PCR Protocols: A Guide to Methods and Applications (eds. Innis, et al., Academic Press, San Diego, Calif., 1990); Mattila et al., Nucleic Acids Res. 19, 4967 (1991); Eckert et al., PCR Methods and Applications 1, 17 (1991); PCR (eds. McPherson et al., IRL Press, Oxford); and U.S. Pat. Nos. 4,683,202, 4,683,195, 4,800,159 4,965,188 and 5,333,675.

Nucleic acid amplification techniques are traditionally classified according to the temperature requirements of the amplification process. Isothermal amplifications are conducted at a constant temperature, in contrast to amplifications that require cycling between high and low temperatures. Examples of isothermal amplification techniques are: Strand Displacement Amplification (SDA; Walker et al., 1992, Proc. Natl. Acad. Sci. USA 89:392 396; Walker et al., 1992, Nuc. Acids. Res. 20:1691 1696; and EP 0 497 272, self-sustained sequence replication (3SR; Guatelli et al., 1990, Proc. Natl. Acad. Sci. USA 87:1874 1878), the Q.beta. replicase system (Lizardi et al., 1988, BioTechnology 6:1197 1202), and the techniques disclosed in WO 90/10064 and WO 91/03573.

Examples of techniques that require temperature cycling are: polymerase chain reaction (PCR; Saiki et al., 1985, Science 230:1350 1354), ligase chain reaction (LCR; Wu et al., 1989, Genomics 4:560 569; Barringer et al., 1990, Gene 89:117 122; Barany, 1991, Proc. Natl. Acad. Sci. USA 88:189 193), transcription-based amplification (Kwoh et al., 1989, Proc. Natl. Acad. Sci. USA 86:1173 1177) and restriction amplification (U.S. Pat. No. 5,102,784).

Other exemplary techniques include Nucleic Acid Sequence-Based Amplification ("NASBA"; see U.S. Pat. No. 5,130,238), Qβ replicase system (see Lizardi et al., BioTechnology 6:1197 (1988)), and Rolling Circle Amplification (see Lizardi et al., Nat Genet 19:225 232 (1998)). The amplification primers of the present invention may be used to carry out, for example, but not limited to, PCR, SDA or tSDA. Any of the amplification techniques and methods disclosed herein can be used to practice the claimed invention as would be understood by one of ordinary skill in the art.

PCR is an extremely powerful technique for amplifying specific polynucleotide sequences, including genomic DNA, single-stranded cDNA, and mRNA among others. Various methods of conducting PCR amplification and primer design and construction for PCR amplification will be known to those of skill in the art. Generally, in PCR a double-stranded DNA to be amplified is denatured by heating the sample. New DNA synthesis is then primed by hybridizing primers to the target sequence in the presence of DNA polymerase and excess dNTPs. In subsequent cycles, the primers hybridize to the newly synthesized DNA to produce discreet products with the primer sequences at either end. The products accumulate exponentially with each successive round of amplification.

The DNA polymerase used in PCR is often a thermostable polymerase. This allows the enzyme to continue functioning after repeated cycles of heating necessary to denature the double-stranded DNA. Polymerases that are useful for PCR include, for example, Taq DNA polymerase, Tth DNA polymerase, Tfl DNA polymerase, Tma DNA polymerase, Tli DNA polymerase, and Pfu DNA polymerase. There are many commercially available modified forms of these enzymes including: AmpliTaq® and AmpliTaq Gold® both available from Applied Biosystems. Many are available with or without a 3- to 5' proofreading exonuclease activity. See, for example, Vent® and Vent®, (exo-) available from New England Biolabs.

Other suitable amplification methods include the ligase chain reaction (LCR) (e.g., Wu and Wallace, Genomics 4, 560 (1989) and Landegren et al., Science 241, 1077 (1988)), transcription amplification (Kwoh et al., Proc. Natl. Acad. Sci. USA 86, 1173 (1989)), and self-sustained sequence replication (Guatelli et al., Proc. Nat. Acad. Sci. USA, 87, 1874 (1990)) and nucleic acid based sequence amplification (NABSA). (See, U.S. Pat. Nos. 5,409,818, 5,554517, and 6,063,603). The latter two amplification methods include isothermal reactions based on isothermal transcription, which produce both single-stranded RNA (ssRNA) and double-stranded DNA (dsDNA) as the amplification products in a ratio of about 30 or 100 to 1, respectively.

The present disclosure, in some embodiments, describes compositions, kits and methods for detection of one or more virulence factor expressing microorganisms such as *eae* and/or *stx.* The present disclosure, in some embodiments also describes compositions, kits and methods for detection of one or more STEC microbes including *E. coli* 0157:H7 and non-0157:H7 STEC microorganisms. Some non-O157:H7 STEC microbes include an *E. coli* 026, an *E. coli* 045, an *E. coli* 0103, an *E. coli* O111, an *E. coli* 0121 and an *E. coli* 0145.

Some species of *Escherichia coli* (*E. coli*) have the potential to be pathogenic to humans. Among these are the shigatoxigenic group of *E. coli* (known as *STEC* or *VTEC*) which produce shiga toxins (also known as verotoxins). These toxins are proteins encoded by the *stx* genes (*stx1* and/or *stx2*) and may produce symptoms ranging from simple diarrhea to hemorrhagic diarrhea. Some STEC's may be lethal to humans, especially infants under five years old and immuno-compromised humans. Some STECs not only produce one or more shiga toxins but they can also attach to the intestinal wall because of a protein intimin (an adhesin) encoded by a gene called *eae. E. coli* carrying these two genes (*stx* and *eae*) have previously been identified as being responsible for Uremic and Haemolytic Syndrome (UHS). They are commonly known as Entero-Hemorrhagic *Escherichia coli (EHEC).* The present specification refers to shiga toxins and *eae* genes as virulence factors as they contribute to the virulence of STEC microorganisms.

In addition to *E. coli* O157:H7, the USDA-FSIS has recently identified six strains of non-O157:H7 *E. coli* STEC including *E. coli* serotypes 026, 045, 0103, O111, 0121, 0145 as microbes that must be tested as food contaminants to prevent and reduce the incidence of food-borne pathogen outbreaks and fatalities. The European Food Safety Association (EFSA) is also requiring testing of at least four of these *E. coli* serotypes including 026, 0103, O111, 0145.

Compositions, kits and methods of the present disclosure provide rapid tests for detecting these microbial contaminants in samples including complex food samples.

Some embodiments describe compositions, kits and methods for detection of microorganisms expressing a virulence factor. Virulence factors comprise one or more genes, alleles and/or variants encoding a *stx* and/or an *eae.* In some embodiments, detection of virulence factors in a microorganism may comprise detection of one or more genes, alleles and/or portions and/or fragments and/or complements thereof encoding a shiga toxin and/or an *eae.*

Shiga toxins are a family of related toxins with two major groups, *Stx*1 and *Stx2,* whose genes encoded by the loci termed *stx1* and *stx2* respectively are considered to be part of the genome of lambdoid prophages. These toxins were first described in explaining the bacterial origin of dysentery caused by *Shigella dysenteriae.* The most common sources for shiga toxins are the bacteria *S*. *dysenteriae* and the *Shigatoxigenic group* of *Escherichia coli* (STEC), which includes serotypes O157:H7, O104:H4, and other enterohemorrhagic *E. coli* (EHEC).

In one embodiment of the current specification, bioinformatic and direct DNA sequencing comparisons of several organisms that express shiga toxins were conducted in an effort to identify shiga toxin encoding sequences, collectively referred to as *stx* loci. Alignment of these sequences using custom algorithms identified several *stx* target regions to which primer pairs of the disclosure were designed for each identified *stx* target regions to specifically amplify only the unique *stx* target sequences against both inclusion (organism to be detected, *i.e.,* shiga toxin producing organisms) and exclusion genomes (organisms not to be detected, non-shiga toxin producing organisms).

Several programs for designing primers such as Primer3 (Steve Rozen and Helen J. Skaletsky (2000) "Primer3" on the World Wide Web for general users and for biologist programmers as published in: Krawetz S, Misener S (eds) Bioinformatics Methods and Protocols: Methods in Molecular Biology. Humana Press, Totowa, NJ, pp 365-386), Primer Express® software (Applied Biosystems), and OLIGO 7 (Wojciech Rychlik (2007). "OLIGO 7 Primer Analysis Software". Methods Mol. Biol. 402: 35-60) and variations thereof may be used for primer designing. The subsequently designed PCR primers and probes for use in assays by real-time PCR detected unambiguously, specifically and with great sensitivity shiga toxin producing organisms and *eae* encoding organisms.

In some embodiments, compositions comprising primer and/or probe sequences are described comprising isolated nucleic acid sequences having the nucleotide sequence of SEQ ID NOS: 1-14, complements thereof and sequences having about 90% identity to the foregoing sequences. In some embodiments, the present disclosure describes designing degenerate primers. In some embodiments the present disclosure describes designing multiplex primers that may be suitable for multiplex PCR type of assays. In some embodiments, isolated nucleic acid sequence compositions of the disclosure may further comprise one or more label, such as, but not limited to, a dye, a radioactive isotope, a chemiluminescent label, a fluorescent moiety, a bioluminescent label an enzyme, and combinations thereof.

The specification also describes methods for detection of a shiga toxin producing organism from a sample and describes methods to exclude the presence of a shiga toxin producing organism in a sample, wherein the detection of at least one nucleic acid sequence that is expressed in a shiga toxin producing organism is indicative of the presence of a shiga toxin producing organism and the absence of detection of any nucleic acid sequence unique to shiga toxin producing organism is indicative of the absence of a shiga toxin producing organism in the sample.

In some embodiments, methods of detecting in a sample the presence of microorganisms of different species, serotypes and strains that encode a shiga toxin virulence factor are disclosed. In some embodiments, methods of detecting the presence of shiga toxin producing *E. coli* strains (STEC organisms are described). In some embodiments, methods of detecting the presence of shiga toxin producing *Shigella* strains are described.

A method of the disclosure, in some embodiments, may comprise detecting, in a sample, at least one (or more) nucleic acid sequence(s) having at least 10 to at least 25 nucleic acids of one (or more) shiga toxin encoding loci such as *stx1* and/or *stx2* and/or complementary sequences thereof, wherein detection of at least one nucleic acid sequence indicates the presence of an shiga toxin producing organism in the sample. Methods of detection may also comprise identification steps and may further comprise steps of sample preparation. These embodiments are described in detail in other sections of this application.

Detection of shiga toxin producing organisms by the use of methods described herein may use a polymerase chain reaction for rapid detection. In general all methods of the disclosure may include comparing for presence of an *stx* expressing organism using suitable controls, for example, an internal positive control may be used in a PCR reaction which will have a detectable signal/positive result, a suitable negative control may also be used that will have no detectable signal.

In one embodiment, a method for detection of a shiga toxin producing organism from a sample may comprise: detecting the presence of an *Stx*1 encoding nucleic acid and/or a fragment or a complement thereof comprising: contacting nucleic acids present in the sample with at least one primer set, having one forward primer and one reverse primer, comprising primers having SEQ ID NO: 1 and SEQ ID NO: 2 under conditions suitable for amplification; amplifying an *Stx*1 encoding nucleic acid and/or a fragment or a complement thereof; and detecting an amplified nucleic acid, wherein detecting/presence of an amplified nucleic acid using said primers confirms the presence of a *stx*1 producing organism in a sample and not detecting an amplified nucleic acid indicates the absence of an *stx*1 producing organism. A probe having SEQ ID NO: 3 may be used to detect an *stx*1 amplification product amplified by primers with SEQ ID NO: 1 and SEQ ID NO: 2 respectively.

Another embodiment method for detection of a shiga toxin producing organism from a sample may comprise: detecting the presence of an *stx2* encoding nucleic acid and/or a fragment or a complement thereof comprising: contacting nucleic acids present in the sample with at least one primer set, each primer set having one forward primer and one reverse primer, comprising the at least one primer set selected from: a first primer set having SEQ ID NO: 4 and SEQ ID NO: 5; and/or a second primer set having SEQ ID NO: 6 and SEQ ID NO:7; and/or a third primer set having SEQ ID NO: 8 and SEQ ID NO: 9; providing conditions suitable for an nucleic acid amplification reaction to amplify an *stx2* encoding nucleic acid and/or a fragment or a complement thereof; and detecting an amplified nucleic acid, wherein detecting an amplified nucleic acid using said primers confirms the presence of a *stx2* producing organism in a sample and not detecting an amplified nucleic acid indicates the absence of an *stx2* producing organism. In some embodiments, at least two primer sets can be used to detect an *stx2* microbe. A probe having SEQ ID NO: 10 may be used to detect an *stx*1 amplification product amplified by each of the *stx2* primer sets described here.

Some embodiments describe a method for detection of a shiga toxin producing organism from a sample comprising: detecting the presence of one or more *stx* encoding nucleic acids including detecting an *stx1* encoding nucleic acid and/or a fragment or a complement thereof comprising and/or detecting an *stx2* encoding nucleic acid and/or a fragment or a complement thereof comprising: a) amplifying from a sample an *stx1* encoding nucleic acid and/or a fragment or a complement thereof by contacting nucleic acids present in the sample with at least one primer set, having one forward primer and one reverse primer, comprising primers having SEQ ID NO: 1 and SEQ ID NO: 2; and b) amplifying simultaneously from the same sample an *stx2* encoding nucleic acid and/or a fragment or a complement thereof by simultaneously contacting nucleic acids present in the sample with at least one primer set, each primer set having one forward primer and one reverse primer, comprising the at least one primer set selected from: a first primer set having SEQ ID NO: 4 and SEQ ID NO: 5; and/or a second primer set having SEQ ID NO: 6 and SEQ ID NO:7; and/or a third primer set having SEQ ID NO: 8 and SEQ ID NO: 9, wherein the contacting is performed under conditions suitable for a nucleic acid amplification reaction; and detecting at least one amplified nucleic acid amplified by either the amplification reactions of steps a) and/or b), wherein detection of at least one amplified nucleic acid indicates the presence of a shiga toxin producing organism in the sample.

In one embodiment method for detection of a shiga toxin producing organism from a sample may comprise: detecting the presence of one or more *stx 1* and/or *stx2* encoding nucleic acid and/or a fragment or a complement thereof comprising: contacting nucleic acids present in a sample with a multiplex of primer sets comprising at least two primer sets, each primer set having one forward primer and one reverse primer, the first primer set having SEQ ID NO: 1 and SEQ ID NO: 2 and the second primer set selected from: a primer set having SEQ ID NO: 4 and SEQ ID NO: 5; and/or a primer set having SEQ ID NO: 6 and SEQ ID NO: 7; and/or a primer set having SEQ ID NO: 8 and SEQ ID NO: 9; providing conditions optimal for an amplification reaction to obtain one or more amplified nucleic acids; and detecting the one or more amplified nucleic acids, wherein detecting an amplified nucleic acid using the first primer set indicates the presence of a *stx1* producing organism and detecting an amplified nucleic acid using the second primer set indicates the presence of an *stx2* allele in the sample.

In other embodiments, not detecting any amplified product using the methods described above may be used to exclude the presence of a shiga toxin producing organism in a sample.

In some embodiments of the present methods, one assay alone may not be definitive for detecting a shiga toxin producing organism due to genomic similarity between the genomic regions of other non-shiga toxin producing organisms. Yet, when two (or more) assays such as but not limited to the assays shown in Table 1 are used either in parallel or as a multiplex assay, e.g., in a real-time TaqMan® assay, for example, where each probe in each of the two (or more) assays has a different label for distinguishing results on a real-time PCR instrument, e.g., a 7500 Fast Real-Time PCR System (Applied Biosystems), a positive result from such an assay is indicative of the presence of a shiga toxin producing organism.

In other embodiments, dual or multiplex (more than 2 assay sets) assay approach can be used to detect and distinguish shiga toxin producing organism. In some embodiments, assay may comprise detecting the presence of genes, alleles and/or fragments of such genes/alleles encoding one or more shiga-toxin encoding gene loci such as but not limited to *stx1* and *stx2.* Some embodiments describe detecting all these gene loci as positive identification of a shiga toxin producing organism. Some embodiments describe detecting at least two of these gene loci as positive identification of a shiga toxin producing organism.

In some embodiments, the present disclosure describes isolated nucleic acid sequence comprising SEQ ID NO:11-SEQ ID NO:14, fragments thereof, complements thereof, sequences comprising at least 90% nucleic acid sequence identity thereto and labeled derivatives thereof. These include primer and probe sequences operable to bind to and/or amplify and/or detect and/or identify any of the approximately 25 alleles of the *eae* gene, encoding intimin.

In one embodiment method for detection of an eae expressing organism from a sample may comprise: detecting the presence of one or more *eae* encoding nucleic acid and/or a fragment or a complement thereof comprising: contacting nucleic acids present in a sample with at least one primer set selected from: a first primer set having SEQ ID NO: 11 and SEQ ID NO: 12, and/or a second primer having SEQ ID NO: 11 and SEQ ID NO: 13; providing conditions optimal for an amplification reaction to obtain one or more amplified nucleic acids; and detecting the one or more amplified nucleic acids, wherein detecting an amplified nucleic acid using said primers confirms the presence of an *eae* expressing organism in the sample. An amplified nucleic acid corresponding to an *eae* gene or fragment thereof may be detected by a probe sequence having SEQ ID NO: 14.

Some embodiments relate to compositions comprising isolated nucleic acid sequences that are operable to hybridize to nucleic acid regions that are uniquely found in a STEC microorganism. Nucleic acid regions uniquely found in a STEC microorganism specific are referred to herein as microorganism specific target nucleic acids or STEC microorganism specific target nucleic acids.

Bioinformatic and direct DNA sequence comparisons of several STEC organisms were conducted in an effort to identify various STEC specific target nucleic acid sequences, also referred to herein as microorganism specific target nucleic acids (or nucleic acid regions or target regions). Alignment of these sequences using custom algorithms identified several STEC target nucleic acid regions to which primer pairs described herein were designed for each identified STEC target regions to specifically amplify only the unique STEC target sequences against both inclusion (organism to be detected, *i.e.,* STEC organisms) and exclusion genomes (organisms not to be detected, non-STEC organisms). STEC specific target regions were also used to design probes that can be used to hybridize to and detect STEC specific amplified targets or STEC specific regions in isolated microbial nucleic acids.

In some embodiments, the present disclosure describes isolated nucleic acid sequence comprising SEQ ID NOS: 15-SEQ ID NO: 32, fragments thereof, complements thereof, sequences comprising at least 90% nucleic acid sequence identity thereto and labeled derivatives thereof. These include primer and probe sequences operable to bind to and/or amplify and/or detect and/or identify an *E. coli* STEC of a strain including 026, 045, 0103, O111, 0145 and 0121.

In some embodiments, the present disclosure describes designing degenerate primers. In some embodiments the present disclosure describes designing multiplex primers that may be suitable for multiplex PCR type of assays. In some embodiments, isolated nucleic acid sequence compositions of the disclosure including primers and probes may further comprise one or more label, such as, but not limited to, a dye, a radioactive isotope, a chemiluminescent label, a fluorescent moiety, a bioluminescent label an enzyme, and combinations thereof.

Primer and probe sequences operable to bind to and/or amplify and/or detect and/or identify an *E. coli* 0157:H7 microorganism are described in SEQ ID NOS: 33-SEQ ID NO: 38. Sequences, compositions thereof and methods for detection of an *E. coli* 0157:H7 are also described in U.S. Provisional Patent Application, Serial No. 61/178,931, filed May 15, 2009 (Attorney Docket NO. LT00032Pro); US. Patent Application, Serial NO. 12/780,707 filed May 14, 2010 (Attorney Docket NO. LT00032US); PCT Application Serial No. PCT/US2010/034998, filed May 14, 2010 (Attorney Docket NO. LT00032PCT); and European Patent Application Serial No. 10720105.5, filed May 14, 2010, (Attorney Docket NO. LT00032EP).

The present application, in some embodiments describes methods for detecting various STEC strains of *E. coli* such as an *E. coli* O157:H7, an *E. coli* 026, an *E. coli* 045, an *E. coli* 0103, an *E. coli* O111, an *E. coli* 0121 and an *E. coli* 0145 in a sample, wherein detection of at least one nucleic acid sequence that is expressed in a STEC organism is indicative of the presence of a STEC organism in the sample. Methods to exclude the presence of STEC *E. coli* in a sample are also described wherein the absence of detection of any nucleic acid sequence unique to a STEC organism is indicative of the absence of a STEC organism in the sample.

Some methods disclosed here are singleplex methods and some methods disclosed here are multiplex methods.

A method of the disclosure, in some embodiments, may comprise detecting, in a sample, at least one (or more) nucleic acid sequence(s) having at least 10 to at least 25 nucleic acids of one (or more) nucleic acids unique to a STEC organism, or a complementary sequences thereof, wherein detection of at least one nucleic acid sequence unique to a STEC microbe indicates the presence of STEC organism in the sample. Methods of detection may also comprise identification steps. These embodiments are described in detail in sections below of this application.

Some embodiments describe methods for detection of an *E. coli* 0121 in a sample comprising: hybridizing at least a first pair of nucleic acid amplification primers comprising nucleic acids of SEQ ID NO: 15 and SEQ ID NO: 16, fragments having at least 10 contiguous nucleotides thereof, complements thereof, and sequences having at least 90% homology thereto, to at least a first target polynucleotide sequence present in the sample; amplifying at least the first target polynucleotide sequence or a fragment or a complement thereof to obtain at least one amplified target polynucleotide sequence; and detecting the at least one amplified target polynucleotide sequence; wherein detection of the at least one amplified target polynucleotide sequence is indicative of the presence of an *E. coli* 0121 in the sample. A method to detect *E. coli* 0121 may further comprise using a probe to detect the at least one amplified target polynucleotide sequence wherein the probe comprises a sequence of SEQ ID NO: 17, fragments having at least 10 contiguous nucleotides thereof, complements thereof, and sequences having at least 90% homology thereto.

Some embodiments describe methods for detection of an *E. coli* 0145 in a sample comprising: hybridizing at least a first pair of nucleic acid amplification primers comprising nucleic acids of SEQ ID NO: 18 and SEQ ID NO: 19, fragments having at least 10 contiguous nucleotides thereof, complements thereof, and sequences having at least 90% homology thereto, to at least a first target polynucleotide sequence present in the sample; amplifying at least the first target polynucleotide sequence or a fragment or a complement thereof to obtain at least one amplified target polynucleotide sequence; and detecting the at least one amplified target polynucleotide sequence; wherein detection of the at least one amplified target polynucleotide sequence is indicative of the presence of an *E. coli* 0145 in the sample. A method to detect an *E. coli* 0145 may further comprise using a probe to detect the at least one amplified target polynucleotide sequence wherein the probe comprises a sequence of SEQ ID NO: 20, fragments having at least 10 contiguous nucleotides thereof, complements thereof, and sequences having at least 90% homology thereto.

Some embodiments describe methods for detection of an *E. coli* 026 in a sample and comprise: hybridizing at least a first pair of nucleic acid amplification primers comprising nucleic acids of SEQ ID NO: 21 and SEQ ID NO: 22, fragments having at least 10 contiguous nucleotides thereof, complements thereof, and sequences having at least 90% homology thereto, to at least a first target polynucleotide sequence present in the sample; amplifying at least the first target polynucleotide sequence or a fragment or a complement thereof to obtain an amplified target polynucleotide sequence; and detecting the at least one amplified target polynucleotide sequence; wherein detection of the at least one amplified target polynucleotide sequence is indicative of the presence of an *E. coli* 026 in the sample. A method to detect an *E. coli* 026 may further comprise using a probe to detect the at least one amplified target polynucleotide sequence wherein the probe comprises a sequence of SEQ ID NO: 23, fragments having at least 10 contiguous nucleotides thereof, complements thereof, and sequences having at least 90% homology thereto.

Some embodiments describe methods for detection of an *E. coli* 045 in a sample comprising: hybridizing at least a first pair of nucleic acid amplification primers comprising nucleic acids of SEQ ID NO: 24 and SEQ ID NO: 25, fragments having at least 10 contiguous nucleotides thereof, complements thereof, and sequences having at least 90% homology thereto, to at least a first target polynucleotide sequence present in the sample; amplifying at least the first target polynucleotide sequence or a fragment or a complement thereof to obtain an amplified target polynucleotide sequence; and detecting the at least one amplified target polynucleotide sequence; wherein detection of the at least one amplified target polynucleotide sequence is indicative of the presence of an *E. coli* 045 in the sample. A method to detect an *E. coli* 045 may further comprise using a probe to detect the at least one amplified target polynucleotide sequence wherein the probe comprises a sequence of SEQ ID NO: 26, fragments having at least 10 contiguous nucleotides thereof, complements thereof, and sequences having at least 90% homology thereto.

Some embodiments describe methods for detection of an *E. coli* 0103 in a sample comprising: hybridizing at least a first pair of nucleic acid amplification primers comprising nucleic acids of SEQ ID NO: 27 and SEQ ID NO: 28, complements thereof, and sequences having at least 90% homology thereto, to at least a first target polynucleotide sequence present in the sample; amplifying at least the first target polynucleotide sequence or a fragment or a complement thereof to obtain an amplified target polynucleotide sequence; and detecting the at least one amplified target polynucleotide sequence; wherein detection of the at least one amplified target polynucleotide sequence is indicative of the presence of an *E. coli* 0103 in the sample. A method to detect an *E. coli* 0103 may further comprise using a probe to detect the at least one amplified target polynucleotide sequence wherein the probe comprises a sequence of SEQ ID NO: 29, fragments having at least 10 contiguous nucleotides thereof, complements thereof, and sequences having at least 90% homology thereto.

Some embodiments describe methods for detection of an *E. coli* O111 in a sample comprising: hybridizing at least a first pair of nucleic acid amplification primers comprising nucleic acids of SEQ ID NO: 30 and SEQ ID NO: 31, fragments having at least 10 contiguous nucleotides thereof, complements thereof, and sequences having at least 90% homology thereto, to at least a first target polynucleotide sequence present in the sample; amplifying at least the first target polynucleotide sequence or a fragment or a complement thereof to obtain an amplified target polynucleotide sequence; and detecting the at least one amplified target polynucleotide sequence; wherein detection of the at least one amplified target polynucleotide sequence is indicative of the presence of an *E. coli* O111 in the sample. A method to detect an *E. coli* O111 may further comprise using a probe to detect the at least one amplified target polynucleotide sequence wherein the probe comprises a sequence of SEQ ID NO: 32, fragments having at least 10 contiguous nucleotides thereof, complements thereof, and sequences having at least 90% homology thereto.

Detection of STEC strains described here may use a polymerase chain reaction (PCR) for rapid amplification and detection of target regions. In general all methods of the disclosure may include comparing the detection or absence of an *STEC* organism using a suitable control, for example, an internal positive control may be used in a PCR reaction which will have a detectable signal/positive result, and a suitable negative control may also be used that will have no detectable signal.

In some embodiments, methods of detecting in a sample the presence of STEC microorganisms may further comprise detecting the presence of a *stx* encoding nucleic acid and/or an *eae* encoding nucleic acid.

The specification, in some embodiments, describes a method for detection of one or more microorganisms, each microorganism expressing one or more virulence factors comprising: 1) detecting the presence of one or more virulence factors comprising: a) contacting a sample suspected of containing one or more microorganisms expressing one or more virulence factors with one or more sets of oligonucleotide primers specific to hybridize to target sequences of one or more virulence factors; b) amplifying at least one target nucleic acid sequence encoding at least one of the virulence factors by a multiplex amplification method to obtain one or more amplified virulence factor specific nucleic acids; c) detecting the one or more amplified virulence factor specific nucleic acids or fragments or complements thereof; and d) optionally identifying the amplified virulence factor specific nucleic acids, wherein detecting the one or more amplified virulence factor specific nucleic acids or fragments or complements thereof is indicative of the presence of a microorganism in the sample expressing the one or more virulence factor; and 2) determining the strain of the one or more microorganism expressing the one or more virulence factor comprising: a) contacting the sample with at least one set of oligonucleotide primers specific to hybridize to target nucleic acids specific to a strain of one or more microorganisms; b) coamplifying at least one strain specific target nucleic acid sequence encoding for nucleic acid targets specific to at least one strain of the microorganism by a multiplex amplification method to obtain one or more amplified strain specific nucleic acids; c) detecting the one or more amplified strain specific nucleic acid or fragments or complements thereof; and d) optionally identifying the amplified strain specific nucleic acids. In some embodiments of the method each set of oligonucleotide primers specific to the one or more virulence factors is labeled with a different label. In some embodiments of the method, each set of oligonucleotide primers specific to the one or more strain of the microorganism is labeled with a different label and wherein each set of oligonucleotide primers specific to the one or more virulence factors is labeled with a different label.

In some embodiments, steps described in the method above of 1) detecting presence of one or more virulence factors and 2) determining strain of the one or more microorganism expressing the one or more virulence factor can be performed simultaneously or consequently and/or in any order.

In some embodiments of a method for detection of one or more microorganisms expressing one or more virulence factors, the one or more sets of oligonucleotide primers specific to the one or more virulence factors each comprise: at least one forward primer and at least a reverse primer operable to hybridize to one or more virulence factors and under appropriate amplification conditions form an amplified virulence factor specific nucleic acid product. Virulence factors in some embodiments comprise but are not limited to one or more of the following: all variants of shiga toxin (*stx*) encoding nucleic acids, alleles and fragments thereof; and/or all variants of *eae* gene encoding nucleic acids, alleles and fragments thereof. A *stx* encoding nucleic acid may comprise an *stx1* gene, a *stx2* gene, an allele of an *stx1* gene an allele of an *stx2* gene, or a fragments thereof. Table 1 below lists several *stx1* and *stx2* subtypes and variants that are detected by the methods described herein.

**Table 1:**

| **Stx Type** | **Stx Subtypes** | **Stx variants** |
|---|---|---|
| *Stx1* | 3 | 1a, 1c, 1d |
| *Stx2* | 7 | 2a, 2b, 2c, 2d, 2e, 2f, 2g |

A method of detecting all *stx* variants, including *stx1* (1a, 1c and 1d) and *stx2* (2a, 2b, 2c, 2d, 2e, 2f and 2g) comprise detecting all know variants of *stx1* and *stx2* and comprise performing a multiplex assay comprising hybridizing at least two pairs of PCR primers, a first PCR primer pairs having nucleic acid sequences of SEQ ID NO: 1 and SEQ ID NO: 2; and the second primer set selected from primer sets having SEQ ID NO: 4 and SEQ ID NO: 5; and/or SEQ ID NO: 6 and SEQ ID NO: 7 and/or SEQ ID NO: 8 and SEQ ID NO: 9, (as well as primers having fragments having at least 10 contiguous nucleotides to the sequences described above, complements thereof, and sequences having at least 90% homology thereto), to at least a first target polynucleotide sequence present in the sample; amplifying at least the first target polynucleotide sequence or a fragment or a complement thereof to obtain an amplified target polynucleotide sequence; and detecting the at least one amplified target polynucleotide sequence; wherein detection of the at least one amplified target polynucleotide sequence is indicative of the presence of an *stx* encoding nucleic acid in the sample. In some embodiments, all the four primer pairs described above are used to amplify one or more target *stx* nucleic acid sequences in the sample and wherein the detection of at least one amplified target *stx* nucleic acid is indicative of the presence of a microorganism having a *stx* encoding loci in its nucleic acids. A method for detecting a microbe expressing an *stx* gene may further comprise using a probe to detect the at least one amplified target polynucleotide sequence, wherein the probe comprises a sequence of SEQ ID NO: 3 to detect an *stx*1 nucleic acid product amplified by using primer pair having SEQ ID NO. 1 and SEQ ID NO. 2, wherein the probe comprises a sequence of SEQ ID NO: 10 to detect an *stx2* nucleic acid product amplified by using primer pair having SEQ ID NO. 4 and SEQ ID NO. 5; and/or SEQ ID NO. 6 and SEQ ID NO. 7 and SEQ ID NO. 8 and SEQ ID NO. 9. Probes having SEQ ID NO: 3 and SEQ ID NO: 10 may also comprise fragments having at least 10 contiguous nucleotides thereof, complements thereof, and sequences having at least 90% homology thereto. All *stx* variants may be detected using these methods due to degenerate primers and probes that contain miss-matched bases at known polymorphic sites.

An *eae* encoding nucleic acid may comprise an *eae* gene, an allele of an *eae* gene, or a fragments thereof. Microorganisms expressing one or more virulence factors include for example Shiga toxin-producing *E. coli* (STEC).

A method of detecting the presence of an *eae* expressing microbe in a sample comprises hybridizing at least one pair of PCR primers such as a primer having nucleic acid sequences of SEQ ID NO: 11 and SEQ ID NO: 12 and/or SEQ ID NO: 13, fragments having at least 10 contiguous nucleotides thereof, complements thereof, and sequences having at least 90% homology thereto, to at least a first target polynucleotide sequence present in the sample; amplifying at least the first target polynucleotide sequence or a fragment or a complement thereof to obtain an amplified target polynucleotide sequence; and detecting the at least one amplified target polynucleotide sequence; wherein detection of the at least one amplified target polynucleotide sequence is indicative of the presence of an *eae* encoding nucleic acid in the sample. A method for detecting a microbe expressing an *eae* gene may further comprise using a probe to detect the at least one amplified target polynucleotide sequence, wherein the probe comprises a sequence of SEQ ID NO: 14 (to detect an *eae* nucleic acid product amplified by using primer pairs having SEQ ID NO. 11 and SEQ ID NO. 12 or SEQ ID NO. 13), fragments thereof and complements thereof and sequences having at least 90% sequence homology thereto.

In some embodiments of a method described above the step 1) of determining the presence of one or more virulence factor, the one or more oligonucleotide primer sets specific for one or more virulence factors may comprise: a first primer set having SEQ ID NO: 1 and SEQ ID NO:2; a second primer set having SEQ ID NO: 4 and SEQ ID NO:5; a third primer set having SEQ ID NO: 6 and SEQ ID NO:7; a fourth primer set having SEQ ID NO: 8 and SEQ ID NO: 9; and/or a fifth primer set having SEQ ID NO: 11, SEQ ID NO: 12; and SEQ ID NO: 13. A method may further comprise the steps of detecting the one or more amplified virulence factor specific nucleic acids or fragments or complements thereof comprises hybridizing the one or more amplified virulence factor specific nucleic acids or fragments or complements thereof with a probe selected from SEQ ID NO: 3, SEQ ID NO: 10, and SEQ ID NO: 14.

In other embodiments, not detecting any amplified product using the methods described above may be used to exclude the presence of a microorganism expressing a virulence factor in a sample.

In some embodiments of a method as described above, the step 2) of determining the strain of the one or more microorganism expressing the one or more virulence factor, the at least one set of oligonucleotide primers specific to the strain of the one or more microorganism may comprise a first primer set having SEQ ID NO: 15 and SEQ ID NO:16; a second primer set having SEQ ID NO: 18 and SEQ ID NO:19; a third primer set having SEQ ID NO: 21 and SEQ ID NO: 22; a fourth primer set having SEQ ID NO: 24 and SEQ ID NO: 25, a fifth primer set having SEQ ID NO: 27, SEQ ID NO: 28; a sixth primer set having SEQ ID NO: 30 and SEQ ID NO: 31; a seventh primer set having SEQ ID NO: 33 and SEQ ID NO:34; and/or an eighth primer set having SEQ ID NO: 36 and SEQ ID NO: 37. In some embodiments, the steps of detecting the one or more amplified strain specific nucleic acids or fragments or complements thereof comprises hybridizing the one or more amplified strain specific nucleic acids or fragments or complements thereof with a probe selected from SEQ ID NO: 17, SEQ ID NO: 20, SEQ ID NO: 23, SEQ ID NO: 26, SEQ ID NO: 29, SEQ ID NO: 32, SEQ ID NO: 35, and SEQ ID NO: 38.

In some embodiments, detecting an amplified nucleic acid comprises one or more methods such as but not limited to hybridization, mass spectrometry, molecular barcoding, microfluidics, chemiluminescence, enzyme technologies and combinations thereof. Some embodiments may also comprise identifying a microbe and may comprise methods such as DNA sequencing.

In some embodiments of the present methods, one assay alone may not be definitive for detecting a STEC organism (or any microbe expressing a virulence factor such as *Shigella spp*.) due to genomic similarity between the genomic regions of other non-STEC organisms (or non-*Shigella spp.*). Yet, when two (or more) assays such as but not limited to the assays (combinations of primers to detect *stx*1 and *stx*2) described herein are used either in parallel or as a multiplex assay, *e.g.*, in a real-time TaqMan® assay, for example, where each probe in each of the two (or more) assays has a different label for distinguishing results on a real-time PCR instrument, e.g., a 7500 Fast Real-Time PCR System (Applied Biosystems), a positive result from such an assay is indicative of the presence of an STEC organism.

In other embodiments, dual or multiplex (more than 2 assay sets) assay approach can be used to detect and distinguish STEC organism strains from each other as well as detecting and distinguishing expression of *stx* alleles/variants and/or *eae.* In some embodiments, assay may comprise detecting the presence of genes, alleles and/or fragments of such genes/alleles encoding one or more shiga-toxin encoding gene loci such as but not limited to *stx1* and *stx2.* Some embodiments describe detecting all these gene loci as positive identification of a shiga toxin producing organism. Some embodiments describe detecting at least two of these gene loci as positive identification of a shiga toxin producing organism. Some embodiments describe detection of an *eae* loci. Some embodiments describe detection of different amplification products for different STEC strains such as an *E. coli* O157:H7, an *E*. *coli* 026, an *E. coli* 045, an *E. coli* 0103, an *E. coli* O111, an *E. coli* 0121 and an *E. coli* 0145.

Methods may include multiplex assays such as polymerase chain reactions, wherein hybridizing and amplifying of said first pair of polynucleotide primers occurs in a first vessel and said hybridizing and amplifying of said second pair of polynucleotide primers occurs in a second vessel, or hybridizing and amplifying of said first pair of polynucleotide primers and said hybridizing and amplifying of said second pair of polynucleotide primers occurs in a single vessel, the detection is a real-time assay, the real-time assay is a SYBR® Green dye assay or a TaqMan® assay. Methods may also comprise using additional primers such as a third primer pair and a fourth primer pair and so on.

A method of the disclosure may further comprise providing a first probe and a second probe (and additional probes such as a third probe and a fourth probe and so on), wherein the first and second probes are different from each other, the first probe operable to identify the first amplified target polynucleotide sequence and the second probe operable to identify the second amplified target nucleotide sequence, the first probe further comprises a first label and said second probe further comprises a second label, wherein both labels are selected from a dye, a radioactive isotope, a chemiluminescent label, and an enzyme, the dye comprises a fluorescein dye, a rhodamine dye, or a cyanine dye, the dye is a fluorescein dye and first probe is labeled with FAM™ dye and said second probe is labeled with VIC® dye; and hybridizing the first and second probes to the PCR amplified fragments to detect the presence of the first amplified target polynucleotide sequence and the second amplified target polynucleotide sequence from the sample.

In some embodiments, a sample to be tested for potential contamination by a microbe expressing a virulence factor and/or an STEC microbe, can be tested directly or may be "prepared" or "processed" in some manner prior to molecular testing and analysis (such as by PCR). For example, a sample can be processed to separate and/or to enrich a contaminating microbe. A sample can also be further processed to separate microbial nucleic acids from the remainder of the sample by lysing microbial cells. Lysing can be accomplished using a variety of buffers that can comprise lysing agents such as but not limited to chaotropic agents, and/or enzymatic agents and/or proteases. Lysed microbial cells from a sample can be additionally processed to separate, isolate and/or extract genetic material from the microbe prior to the amplification analysis methods described herein by several methods known to the skilled artisan. For example, nucleic acid extraction can be performed by kits and reagents from Life Technologies Corporation such as The PrepSEQ™ Rapid Spin Sample Preparation Kit can be used to prepare DNA from food and/or environmental samples for use in PCR amplification reactions. Using a simple spin protocol, the PrepSEQ™ Rapid Spin efficiently inactivates PCR inhibitors. The kit provides a fast, cost-effective solution for extracting high-quality DNA from a broad range of sample types. The PrepSEQ® Nucleic Acid Extraction Kit from Life Technologies also produces high-quality bacterial DNA samples for PCR-based detection from a wide range of food and environmental samples.

Some embodiments can comprise one or more of the following steps to achieve separation of microbes and/or their nucleic acids from sample components prior to analysis of microbial nucleic acids as described herein: (1) optional bacterial enrichment to enrich certain types of bacteria *(e.g.,* by providing conditions to selectively increase one bacterial type), (2) optional bacterial cell lysis, (3) optional nucleic acid extraction and/or purification (*e*.*g*., DNA extraction, total nucleic acid extraction (i.e., DNA and RNA), genomic DNA extraction, RNA extraction using spin columns and/or buffers and/or other known methods in the art).

In some embodiments, the disclosure describes workflows of methods for detecting and identifying one or more Shiga toxin-producing *E. coli* (STEC) microorganisms in a sample comprising the steps of: a) optionally enriching the separated STEC microorganisms; b) extracting STEC nucleic acids; c) performing a multiplex polymerase chain reaction (PCR) on the separated STEC microorganisms to amplify and detect the presence of at least one nucleic acids selected from: nucleic acids encoding for a shiga toxin gene, including nucleic acids encoding *stx1* and *stx*2; nucleic acids encoding an *eae* gene; nucleic acids specific for an *E. coli* strain O157:H7, wherein the detection of at least one nucleic acid above indicates the presence of an STEC microorganism or *E. coli* 0157:H7 or both; and d) if a nucleic acid is detected in step c) performing a second round of multiplex PCR with primers specific to amplify and detect the presence one or more nucleic acids encoding for target regions associated with one or more STEC microorganisms including an *E. coli* O157:H7, an *E. coli* 026, an *E. coli* 045, an *E. coli* 0103, an *E. coli* O111, an *E. coli* 0121 and an *E. coli* 0145. In some embodiments, the disclosure describes a workflow to detect the USDA identified Big 6 STEC microorganisms.

In other embodiments, not detecting any amplified product using the methods described above may be used to exclude the presence of a STEC microorganism in a sample.

Amplification of target nucleic acids of targets including *stx1, stx2, eae, E. coli* O157:H7, an *E. coli* 026, an *E. coli* 045, an *E. coli* 0103, an *E. coli* O111, an *E. coli* 0121 and an *E. coli* 0145 can be detected by using a plurality of labels on each different set of primers and/or probes. For example, in step c) above primers and/or probes with a different label for each of *stxl,* sxt2, *eae* and *E. coli* 0157:H7 may be used and in step d) primers and/or a probes with a different label each may be used for the various microorganisms.

In some embodiments, methods of the disclosure can be performed on an automated system. Automation decreases the time as well as efficiency and allows processing multiples samples. Automated systems may comprise a magnetic separation platform such as but not limited to MagMAX™ Express-96 Magnetic Particle Processor Platform (Life Technologies Corporation), Pathatrix (Matrix Microsciences).

Compositions and methods of the present disclosure are ideally suited for the preparation of kits. One example is a kit suitable for detecting the presence of a microorganism expressing a virulence factor. Such a kit may comprise at least one set of oligonucleotide primers for use in an nucleic acid amplification process for the detection of a virulence factors associated with microbes, said set of primers comprising a primers set operable to hybridize to virulence factors comprising all variants of shiga toxin and/or *eae.*

Some embodiments describe kits suitable for identifying the presence of a shiga toxin producing organism. Such a kit may comprise at least one set of oligonucleotide primers for use in an amplification process (such as PCR and in some embodiments a multiplex PCR) for the detection of *stx* virulence factors, the set of primers comprising primers operable to hybridize to virulence factors comprising all variants, genes, allele and/or fragments and complements of shiga toxin. A *stx* detection kit may further comprise probe sequences to detect amplified *stx* target nucleic acids. In one example embodiment, a kit for detecting microorganisms expressing *stx1* and *stx2* variants comprises primers and probes having SEQ ID NOS: 1-10.

Some embodiments describe kits suitable for identifying the presence of an *eae* encoding organism. Such a kit may comprise at least one set of oligonucleotide primers for use in a PCR process for the detection of an *eae* virulence factor, the set of primers comprising a primers operable to hybridize to *eae* encoding genes, alleles, complementary regions thereof and fragments thereof comprising all variants of *eae.* A kit may further comprise probe sequences to detect amplified *eae* target nucleic acids. In one example embodiment, a kit for detecting microorganisms expressing *eae* comprises primers and probes having SEQ ID NOS: 11-14.

Some kits of the disclosure can detect virulence factors including *stx* and *eae* and comprise primer sets selected from: a first primer set having SEQ ID NO: 1 and SEQ ID NO: 2; a second primer set having SEQ ID NO:4 and SEQ ID NO:5; a third primer set having SEQ ID NO: 6 and SEQ ID NO: 7; an fourth primer set having SEQ ID NO:8 and SEQ ID NO:9; a fifth primer set having as forward primer SEQ ID NO: 11 and as reverse primers SEQ ID NO: 12 or SEQ ID NO: 13; and further optionally additionally at least one more probe selected from probes having SEQ ID NO: 3, SEQ ID NO: 10, and SEQ ID NO: 14, or sequences complementary thereto, or sequences comprising at least 90% nucleic acid sequence identity thereof, or a labeled derivatives thereof.

In some embodiments, a kit of the disclosure may alone or in addition to the virulence factor primer and probes described above further comprise one or more primer sets operable to hybridize to unique nucleic acid targets of one or more strains of STEC such as but not limited to an *E. coli* O157:H7, an *E. coli* 026, an *E. coli* 045, an *E. coli* 0103, an *E. coli* O111, an *E. coli* 0121 and an *E. coli* 0145.

All kits of the disclosure may additionally comprise one or more reagents such as but are not limited to, buffers, nucleotide triphosphates, DNA polymerases, intercalating dye, primers, probes, salt, and instructions for the use of the kit.

An exemplary kit for detection of a Shiga toxin-producing *E. coli* (STEC) microorganism comprises: two or more pairs of forward and reverse polymerase chain reaction (PCR) primers selected from a first primer set having SEQ ID NO: 15 and SEQ ID NO:16; a second primer set having SEQ ID NO: 18 and SEQ ID NO:19; a third primer set having SEQ ID NO:21 and SEQ ID NO:22; a fourth primer set having SEQ ID NO: 24 and SEQ ID NO:25, a fifth primer set having SEQ ID NO:27 and SEQ ID NO:28; a sixth primer set having SEQ ID NO: 30 and SEQ ID NO:31, or sequences complementary thereto, or sequences comprising at least 90% nucleic acid sequence identity thereto, fragments having at least 10 contiguous nucleotides thereof, or a labeled derivative thereof; optionally at least one probe and one or more components selected from a group consisting of: at least one enzyme; dNTPs, at least one buffer, at least one salt, at least one control nucleic acid sample and an instruction protocol. Such as kit can detect the presence of one or more STEC organisms including an *E. coli* 026, an *E. coli* 045, an *E. coli* 0103, an *E. coli* O111, an *E. coli* 0121 and an *E. coli* 0145.

In some embodiments, a kit of the disclosure may also or alternatively comprise one or more pairs of forward and reverse PCR primers further selected from a seventh primer set having SEQ ID NO: 11 and SEQ ID NO: 12 and SEQ ID NO: 13; an eighth primer set having SEQ ID NO: 1 and SEQ ID NO: 2; a ninth primer set having SEQ ID NO:4 and SEQ ID NO:5; a tenth primer set having SEQ ID NO: 6 and SEQ ID NO: 7; an eleventh primer set having SEQ ID NO:8 and SEQ ID NO:9; and further optionally additionally at least one more probe selected from probes having SEQ ID NO: 14, SEQ ID NO: 3, SEQ ID NO: 10, or sequences complementary thereto, or sequences comprising at least 90% nucleic acid sequence identity thereof, or a labeled derivatives thereof. Such as kit can detect the presence of one or more STEC organisms expressing virulence factors *stx* and *eae.* These kits can also detect the presence of the specific STEC strain such as if the contaminant is an *E. coli* 026, an *E. coli* 045, an *E. coli* 0103, an *E. coli* O111, an *E. coli* 0121 and an *E. coli* 0145.

In some embodiments, kits of the disclosure may further comprise additionally one or more pairs of forward and reverse PCR primers further selected from a twelfth primer set having SEQ ID NO: 33 and SEQ ID NO: 34; a thirteenth primer set having SEQ ID NO: 36 and SEQ ID NO: 37; and optionally additionally at least one more probe further selected from probes having SEQ ID NO: 35, SEQ ID NO: 38, or sequences complementary thereto, or sequences comprising at least 90% nucleic acid sequence identity thereof, or labeled derivatives thereof. Such as kit can additionally detect the presence of an *E. coli* 0157:H7 in the sample.

In some embodiments, kit primers may be labeled. A kit comprising multiple pairs of primers may have primer pairs each labeled with different labels that may be detectable separately. Probes comprised in kits of the disclosure may be labeled. If a kit comprises multiple probes each probe may be labeled with a different label to allow detection of different products that may be the target of each different probe. Components may be provided as solutions or as lyophilized powders which may be later reconstituted if needed in solutions and/or buffers which may also be provided.

Components of kits may be individually and in various combinations comprised in one or a plurality of suitable container means. It is within the scope of these teachings to provide test kits for use in manual applications or test kits for use with automated sample preparation, reaction set-up, detectors or analyzers. In some embodiments, a kit amplification product may be further analyzed by methods such as but not limited to electrophoresis, hybridization, mass spectrometry, molecular barcoding, microfluidics, chemiluminescence and/or enzyme technologies.

Those having ordinary skill in the art, in light of this specification, will understand that many modifications, alternatives, and equivalents of the embodiments described above are possible. All such modifications, alternatives, and equivalents are intended to be encompassed herein.

### EXAMPLES

The following procedures are representative examples of embodiments according to the disclosure that may be employed for the detection of a STEC organism and/or an organism expressing a virulence factor. These examples are not intended to be limiting to the scope of the claims and/or the disclosure in any way.

### Example 1: Compositions & Methods to Detect Shiga Toxin Producing Organisms

The present example describes examplary assays designed to detect shiga toxin producing organisms using probe and primer sequences designed by the present inventors. Primer sequences comprising pairs of forward and reverse primers and corresponding probe sequences are shown in Table 2 below.

An exemplary method of detecting the presence of a shiga toxin producing organism in a sample comprises: extracting (and/or isolating) nucleic acid from a sample and detecting the presence of at least one *stx*1 or *stx*2 locus or fragment thereof or complement thereof as shown using an assay described in Table 2 below. In some embodiments, microbial cells can be directly subject to detecting target *stx* nucleic acid loci or fragments without any nucleic acid extraction.

As shown in Table 2, an Assay ID number (such as 14252, 14254 etc.) is assigned to describe an associated primer pair (or primer pairs for multiplex assays) and associated probes that may be used for detection of a target *stx* sequence (see *stx1, stx2,* both *stx1* and *stx2* in Table 2). These specific combinations of primer pairs and probe sequences have designed to selectively amplify *stx* loci genes present. In some embodiments these primer pairs are degenerate.

As shown in Table 2, a forward and reverse primer pair is shown in a row, if used in an amplification reaction, will amplify the corresponding target *stx1* or *stx2* nucleic acid. For example, Assay ID number 14252 maybe used to detect the presence of a shiga toxin producing organism having an *stx1* gene by: contacting a sample suspected of being contaminated with a forward primer having the nucleic acid sequence GTGACAGTAGCTATACCACGTTACA (SEQ ID NO: 1) and a reverse primer having the nucleic acid sequence AGTGTTGTACGAAATCCCCTCT (SEQ ID NO: 2) under conditions to amplify a target nucleic acid sequence of an *stx1* gene loci (or fragment or complement therof). Melting temperatures Tm for forward and reverse primers are also shown in Table 2. In some embodiments, an amplified product amplified using the primers of a row can be detected using a probe described in the same row, for example in Assay ID 14252 a probe having the nucleic acid sequence ATGGCGATTTATCTGCATCCC (SEQ ID NO: 3) can be used.

Assay ID's of Table 2 can be combined to form multiplex assays. For example, one multiplex assay combines assays with Assay ID's 14252 with either Assay ID 14254, 29133, and/or 29134 of Table 2 and can detect all *stx1* and *stx2* alleles. Multiplex assays may be performed by simultaneously contacting a sample with the one or more primer pairs. In some embodiments, multiplex assays can be performed in parallel or can be performed sequentially.

**Table 2: Assays & Melting Temperatures for detecting stx target nucleic acids:**

| ***Assay ID** | ***Forward primer** | ***Reverse primer** | ***Probe** | ***For Tm** | ***Rev Tm** | ***Prob** |
|---|---|---|---|---|---|---|
| *stx1* | | | | | | |
| 14252 | GTGACAGTAGCTATACCAC GTTACA (SEQ ID NO: 1) | AGTGTTGTACGAAATCCC CTCT (SEQ ID NO: 2) | ATGGCGATTTATCTGCAT CCC (SEQ ID NO: 3) | 61.26 | 60.73 | 71.89 |

| *stx2* | | | | | | |
|---|---|---|---|---|---|---|
| 14254 | CGTTTTGACCATCTTCGTC TGATT (SEQ ID NO: 4) | TGCGACACGTTGCAGAGT (SEQ ID NO: 5) | ACGGACAGCAGTTATAC (SEQ ID NO: 10) | 61.09 | 62.37 | 70.1 |
| 29133 | CGTTTTGACCATCTTCGTC TGATT (SEQ ID NO: 6) | TGCGACACGTTGCAGCGT (SEQ ID NO: 7) | ACGGACAGCAGTTATAC (SEQ ID NO: 10) | 61.09 | 65.99 | 70.1 |
| 29134 | GATTTCTCACATATTTCAG TGCCTGATG (SEQ ID NO: 8) | CCAGATCTGCGATTCGCT GTAAT (SEQ ID NO: 9) | ACGGACAGCAGTTATAC (SEQ ID NO: 10) | 61.64 | 62.51 | 70.1 |

| | | | | | | |
|---|---|---|---|---|---|---|
| *Assay ID describes an assay comprising using Forward Primers and Reverse Primers described in one row and in some embodiments Probe described in the same row. * For Tm describes the Tm of the Forward Primers; Rev Tm describes the Tm of Reverse Primers and Probe Tm describs the Tm of the Probe. | | | | | | |

Detecting can also comprise methods such as amplification, hybridization, mass spectrometry, nanostring, microfluidics, chemiluminescence, enzyme technologies and combinations thereof. Detecting steps using probes may comprise providing at least a first probe (and in some embodiments such as multiplex assays additional probes - such as a second probe, a third probe, a fourth probe etc.,), wherein the first and second probes are different from each other, the first probe operable to identify the first amplified target polynucleotide sequence and the second probe operable to identify the second amplified target nucleotide sequence, the first probe further comprises a first label and said second probe further comprises a second label, wherein both labels are selected from a dye, a radioactive isotope, a chemiluminescent label, and an enzyme, the dye comprises a fluorescein dye, a rhodamine dye, or a cyanine dye, the dye is a fluorescein dye and first probe is labeled with FAM™ dye and said second probe is labeled with VIC® dye; and hybridizing the first and second probes to the PCR amplified fragments to detect the presence of the first amplified target polynucleotide sequence and the second amplified target polynucleotide sequence from the sample.

### Example 2: Multiplex Methods to detect E. coli expressing Virulence Factors comprising shiga toxin encoding loci

In some embodiments methods are described comprising multiplex assays designed to detect combinations of multiple gene loci that are present STEC organisms including *E. Coli* 0157:H7 and *E. Coli* O104:H4. Detection of combinations of these genes indicates the presence of an organism expressing shiga toxin.

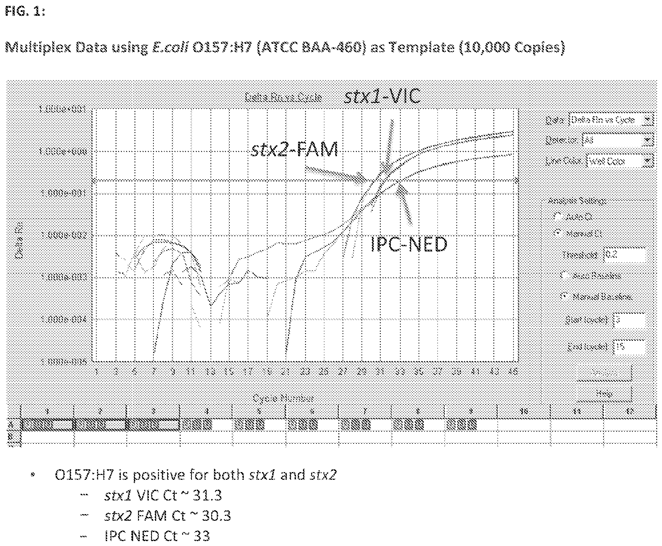

According to some embodiments, detecting the presence combinations of target genes of *stx* loci of *E. Coli* 0157:H7 which is inherently positive for both *stx1* and *stx2* is shown in FIG 1. FIG. 1 shows the results of a multiplex assay showing the presence of *stx1* shown by a VIC probe as *stx1* VIC Ct ∼ 31.3 and shows the presence of *stx2* shown by a FAM probe as *stx2* FAM Ct ∼ 30.3. The internal positive control (IPC) is shown using another labeled probe NED as IPC NED Ct ∼ 33.

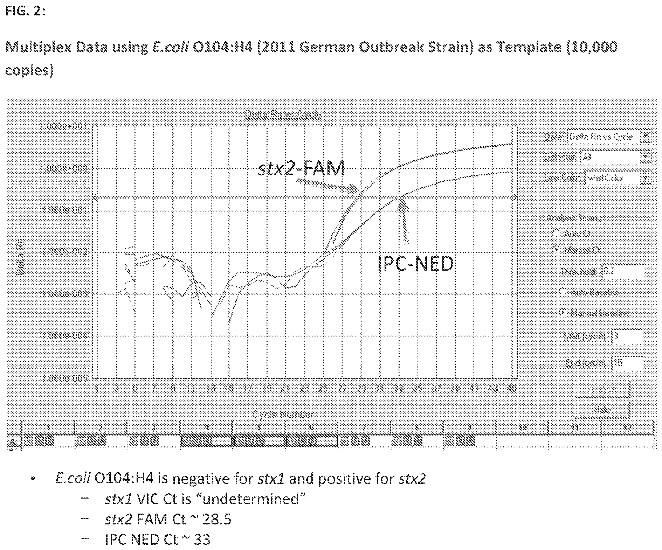

In some embodiments, detecting the presence combinations of target genes of *stx* loci of *E. coli* O104:H4 which is inherently negative for *stx1* and positive for *stx2* is shown in FIG 2. FIG. 2 shows the results of a multiplex assay showing the absence of *stx1* shown by a VIC probe as *stx1* VIC Ct is "undetermined" and shows the presence of *stx2* shown by a FAM probe as *stx2* FAM Ct - 28.5. The internal positive control (IPC) is shown using another labeled probe NED as IPC NED Ct ∼ 33.

In the methods of the disclosure described, detecting each of the above referenced genes or gene loci to identify combination of genes, using the probes and primers described in this specification, may comprises using methods such as but not limited to amplification, hybridization, mass spectrometry, nanostring, microfluidics, chemiluminescence, enzyme technologies and combinations thereof. Amplification may comprise a polymerase chain reaction (PCR), RT-PCR, asynchronous PCR (A-PCR), and asymmetric PCR (AM-PCR), strand displacement amplification (SDA), multiple displacement amplification (MDA), nucleic acid strand-based amplification (NASBA), rolling circle amplification (RCA), transcription-mediated amplification (TMA). Methods may also further comprise isolating nucleic acid from a sample.

An example embodiment is described for use of two probes for detection, which may also be applied to using more than two probes for detecting multiple gene loci. For example, in a detecting step of a method as described herein a first probe and a second probe may be used, wherein the first and second probes are different from each other, the first probe operable to identify the first amplified target polynucleotide sequence and the second probe operable to identify the second amplified target nucleotide sequence, the first probe further comprises a first label and said second probe further comprises a second label, wherein both labels are selected from a dye, a radioactive isotope, a chemiluminescent label, and an enzyme, the dye comprises a fluorescein dye, a rhodamine dye, or a cyanine dye, the dye is a fluorescein dye and first probe is labeled with FAM™ dye and said second probe is labeled with VIC® dye; and hybridizing the first and second probes to the PCR amplified fragments to detect the presence of the first amplified target polynucleotide sequence and the second amplified target polynucleotide sequence from the sample.

### Example 3: Detection of stx1 and stx2 loci in a panel of microbes

Methods of the present disclosure were used to screen a panel of microbes to detect organisms expressing *stx* loci.

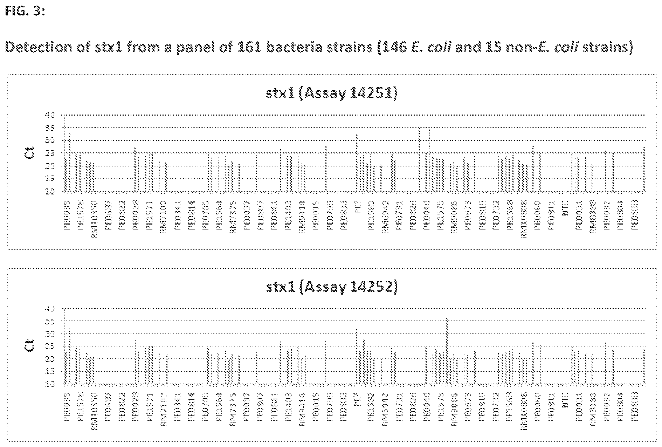

For example, as shown in FIG. 3 a panel of 161 bacterial strains comprising 146 *E. coli* strains and 15 non-*E*. *coli* strains were subject to detection of *stx1* genes using Assay ID 14251 and 14252 respectively. Results obtained by the tests are shown in FIG. 3 which shows that *stx1* loci was detected in some bacteria and not in others using the assays of the disclosure.

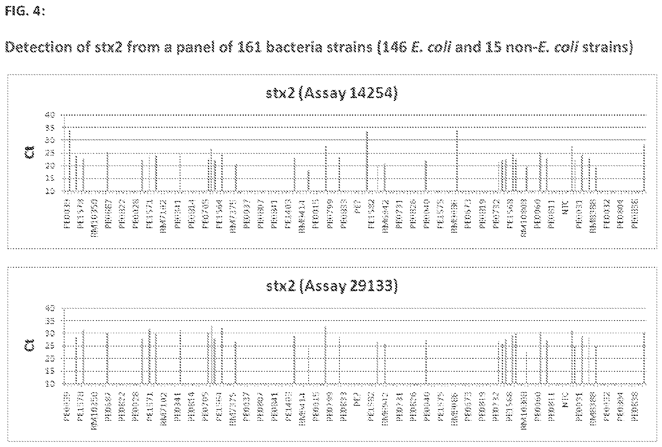

As shown in FIG. 4 a panel of 161 bacterial strains comprising 146 *E. coli* strains and 15 non-E coli strains were subject to detection of *stx2* genes using Assay ID 14254 and 29133 respectively. Results obtained by the tests are shown in FIG. 4 which shows that *stx2* loci was detected in some bacteria and not in others using the assays of the disclosure.

### Example 4: Compositions & Methods to Detect STEC Microorganisms

Real-time PCR methods were developed to detect one or more *E. coli* strains. Some of these strains are adulterants in beef samples. The USDA has classified *E. coli* 0157:H7 as an adulterant since 1994 and has added to the list of adulterants in 2011 several non-O157:H7 shiga-toxin producing *E. coli* (STEC) serotypes if they contain both *stx* and *eae* virulence factors which includes the six serotypes O26, O45, O103, O111, O121, and O145. These 6 serotypes of non-O157:H7 STEC are also referred to as the "big 6" or "top six."

In some embodiments, several singleplex PCR assay methods were presently developed for each adulterant STEC *E. coli* microorganism including 026, 045, 0103, O111, 0121, and 0145 and extensively tested for specificity and sensitivity.

The present example describes methods comprising singleplex assays designed to detect STEC *E. coli* microorganism including serotypes 026, 045, 0103, O111, 0121, and 0145 in a sample using probe and primer sequences designed herein. The present example also describes methods comprising singleplex assays designed to detect the presence of an *eae* virulence factor present in STEC *E. coli* adulterants in a sample using probe and primer sequences designed herein. Primer sequences comprising pairs of forward and reverse primers and corresponding probe sequences for detection of the big 6 serotypes, *eae* as well as *stx1, stx2* and *E. coli* 0157:H7 are shown in Table 3 below.

In one embodiment, an exemplary method of detecting the presence of a STEC organisms in a sample, may comprise: 1) separating or isolating a STEC microorganism present in a sample from other sample components; and 2) detecting the presence of at least one unique nucleic acid target region of a STEC serotype of 026, 045, 0103, O111, 0121, and 0145 and/or fragments thereof and/or complement thereof (and in some embodiments additionally detecting the presence of target nucleic acids of virulence factors such as *stx* and/or *eae* and/or fragments thereof and/or complements thereof) using various primer and/or probe combinations described in Table 3 below.

In some embodiments, an exemplary method of detecting the presence of a STEC organisms in a sample may comprise 1) optionally separating or isolating a STEC microorganism present in a sample from other sample components; 1) extracting or isolating nucleic acid from a sample; and 2) detecting the presence of at least one unique nucleic acid target region of STEC serotypes 026, 045, 0103, O111, 0121, and 0145 and/or fragments thereof and/or complement thereof. A method according to the present disclosure, in some embodiments can in addition to the steps described above (or without the steps described above) also comprise detecting the presence of STEC virulence factors such as *stx* and/or *eae* and/or fragments thereof and/or complement thereof using various primer and/or probe combinations described in Table 3 below.

Various assays comprising primer probe combinations for PCR are shown in Table 3. The first column of Table 3 describes a gene target or microbe name such as *stx1, stx2, eae, E. coli* 0121, *E. coli* 0145, *E. coli* 026, *E. coli* 045, *E. coli* 0103, *E. coli* O111, and *E. coli* O157:H7. The second column describes an Assay ID number (such as 14252, 14254 etc.) which is assigned to describe a primer pair and associated probe that may be used for detection of that target nucleic acid sequence. Column 3 of Table 3 describes the name of each primer and probe sequences described by the assay of column 2 (for example "14252 FOR" describes the forward primer, 14252 REV describes the reverse primer, and 14252 VIC describes a probe sequence, all three primer and probe sequences designed for Assay ID Number 14252 which is an assay to detect an *stx1* specific nucleic acid). Column 4 of Table 3 describes the nucleic acid sequence of the probe or primer described in column 3. These specific combinations of primer pairs and probe sequences have been designed to selectively amplify a target nucleic acid from a microbe or a virulence factor described in column 1. In some embodiments these primer pairs are degenerate.

For example, in Table 3, a forward (with suffix FOR) and reverse (with suffix REV) primer pair is shown for each target gene, which if used in an amplification reaction, will amplify the corresponding target nucleic acid specific for a virulence factor or a microbe.

Table 3 describes singleplex assays as outlined above with primer and probe sequences for *stx1* (1 assay 14252) with a probe described in SEQ ID NO:3 that can detet amplification product of *stx1*; for *stx2* (3 assays 14254, 29133 and 29134) with an "All*stx2* VIC" probe described in SEQ ID NO. 10 that can detect all three of the *stx2* assay amplified products; for *eae* (1 assay 29131 comprising one forward primer and two reverse primers and a probe); for *E. coli* 0121 (1 assay 28292); for *E coli* 0145 (1 assay 28408); for *E. coli* 026 (1 assay 28611);for *E. coli* 045 (1 assay 28689);for *E. coli* 0103 (1 assay 28191);for *E. coli* O111 (1 assay 28230) and for *E. coli* 0157:H7 (2 assays 18158 and 19055). Primers and probes for an internal positive control (IPC) are also used as controls (not expressly described).

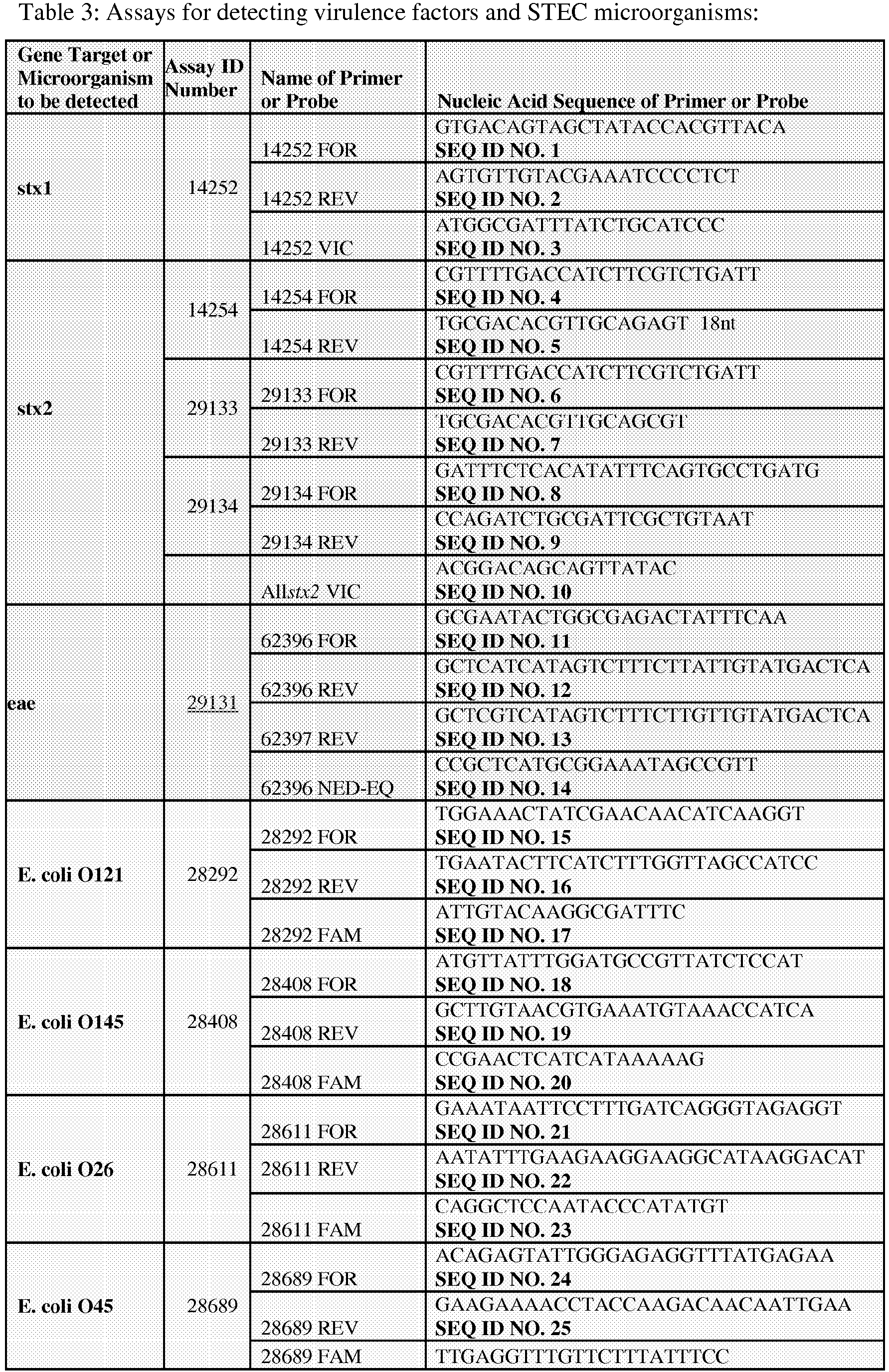

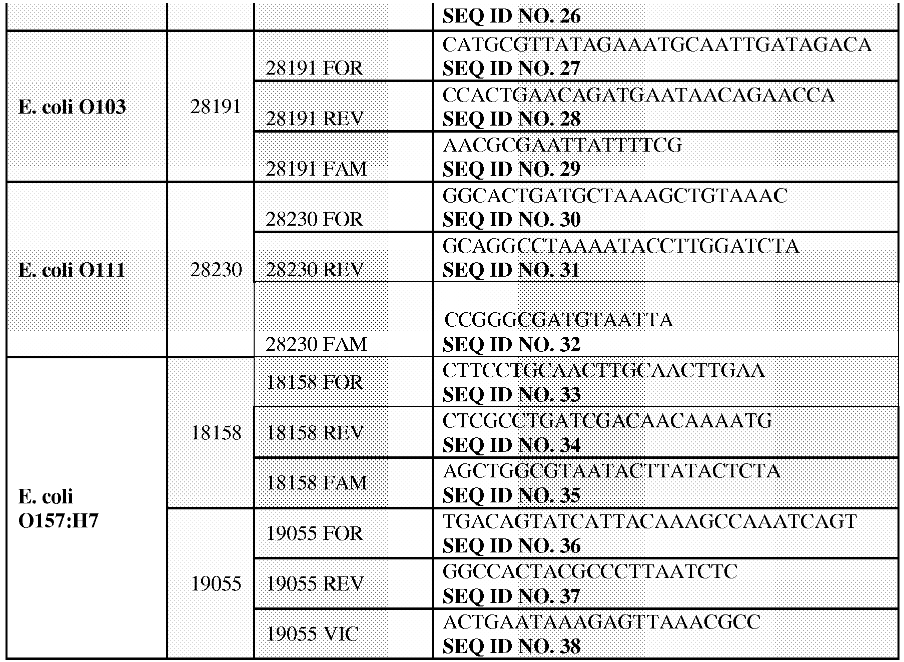

The assays described in Table 3 above can be performed individually as single plex assays or combined as multiplex assays.

In one embodiment, an exemplary method of detecting the presence of an *eae* virulence factor expressing microbe in a sample is described in Table 3 and comprises hybridizing at least one pair of PCR primers such as a forward primer having nucleic acid sequences of SEQ ID NO: 11 and a reverse primer having a nucleic acid sequence of SEQ ID NO:12 and/or SEQ ID NO: 13, fragments having at least 10 contiguous nucleotides thereof, complements thereof, and sequences having at least 90% homology thereto, to at least a first target polynucleotide sequence present in the sample; amplifying at least the first target polynucleotide sequence or a fragment or a complement thereof to obtain an amplified target polynucleotide sequence; and detecting the at least one amplified target polynucleotide sequence; wherein detection of the at least one amplified target polynucleotide sequence is indicative of the presence of an *eae* encoding nucleic acid in the sample. A method for detecting a microbe expressing an *eae* gene may further comprise using a probe to detect the at least one amplified target polynucleotide sequence, wherein the probe comprises a sequence of SEQ ID NO: 14 (to detect an *eae* nucleic acid product amplified by using primer pairs of SEQ ID NO. 11 and SEQ ID NO. 12 and/or SEQ ID NO. 13), fragments thereof and complements thereof and sequences having at least 90% sequence homology thereto.

Table 3 also describes an exemplary methods for detection of an *E. coli* 0121 in a sample comprising: hybridizing at least a first pair of PCR primers comprising nucleic acids of SEQ ID NO: 15 (as a forward primer) and SEQ ID NO: 16 (as a reverse primer), fragments having at least 10 contiguous nucleotides thereof, complements thereof, and sequences having at least 90% homology thereto, to at least a first target polynucleotide sequence present in the sample; amplifying at least the first target polynucleotide sequence or a fragment or a complement thereof to obtain at least one amplified target polynucleotide sequence; and detecting the at least one amplified target polynucleotide sequence; wherein detection of the at least one amplified target polynucleotide sequence is indicative of the presence of an *E. coli* 0121 in the sample. A method to detect *E. coli* 0121 may further comprise using a probe to detect the at least one amplified target polynucleotide sequence wherein the probe comprises a sequence of SEQ ID NO: 17, fragments having at least 10 contiguous nucleotides thereof, complements thereof, and sequences having at least 90% homology thereto.

Table 3 also describes an exemplary method for detection of an *E. coli* 0145 in a sample comprising: hybridizing at least a first pair of PCR primers comprising nucleic acids of SEQ ID NO: 18 and SEQ ID NO: 19, fragments having at least 10 contiguous nucleotides thereof, complements thereof, and sequences having at least 90% homology thereto, to at least a first target polynucleotide sequence present in the sample; amplifying at least the first target polynucleotide sequence or a fragment or a complement thereof to obtain at least one amplified target polynucleotide sequence; and detecting the at least one amplified target polynucleotide sequence; wherein detection of the at least one amplified target polynucleotide sequence is indicative of the presence of an *E. coli* 0145 in the sample. A method to detect an *E. coli* 0145 may further comprise using a probe to detect the at least one amplified target polynucleotide sequence wherein the probe comprises a sequence of SEQ ID NO: 20, fragments having at least 10 contiguous nucleotides thereof, complements thereof, and sequences having at least 90% homology thereto.

Table 3 also describes an exemplary method for detection of an *E. coli* 026 in a sample and comprise: hybridizing at least a first pair of PCR primers comprising nucleic acids of SEQ ID NO: 21 and SEQ ID NO: 22, fragments having at least 10 contiguous nucleotides thereof, complements thereof, and sequences having at least 90% homology thereto, to at least a first target polynucleotide sequence present in the sample; amplifying at least the first target polynucleotide sequence or a fragment or a complement thereof to obtain an amplified target polynucleotide sequence; and detecting the at least one amplified target polynucleotide sequence; wherein detection of the at least one amplified target polynucleotide sequence is indicative of the presence of an *E. coli* 026 in the sample. A method to detect an *E. coli* 026 may further comprise using a probe to detect the at least one amplified target polynucleotide sequence wherein the probe comprises a sequence of SEQ ID NO: 23, fragments having at least 10 contiguous nucleotides thereof, complements thereof, and sequences having at least 90% homology thereto.

Table 3 also describes an exemplary method detection of an *E. coli* 045 in a sample comprising: hybridizing at least a first pair of PCR primers comprising nucleic acids of SEQ ID NO: 24 and SEQ ID NO: 25, fragments having at least 10 contiguous nucleotides thereof, complements thereof, and sequences having at least 90% homology thereto, to at least a first target polynucleotide sequence present in the sample; amplifying at least the first target polynucleotide sequence or a fragment or a complement thereof to obtain an amplified target polynucleotide sequence; and detecting the at least one amplified target polynucleotide sequence; wherein detection of the at least one amplified target polynucleotide sequence is indicative of the presence of an *E. coli* 045 in the sample. A method to detect an *E. coli* 045 may further comprise using a probe to detect the at least one amplified target polynucleotide sequence wherein the probe comprises a sequence of SEQ ID NO: 26, fragments having at least 10 contiguous nucleotides thereof, complements thereof, and sequences having at least 90% homology thereto.

Table 3 also describes an exemplary method for detection of an *E. coli* 0103 in a sample comprising: hybridizing at least a first pair of PCR primers comprising nucleic acids of SEQ ID NO: 27 and SEQ ID NO: 28, complements thereof, and sequences having at least 90% homology thereto, to at least a first target polynucleotide sequence present in the sample; amplifying at least the first target polynucleotide sequence or a fragment or a complement thereof to obtain an amplified target polynucleotide sequence; and detecting the at least one amplified target polynucleotide sequence; wherein detection of the at least one amplified target polynucleotide sequence is indicative of the presence of an *E. coli* 0103 in the sample. A method to detect an *E. coli* 0103 may further comprise using a probe to detect the at least one amplified target polynucleotide sequence wherein the probe comprises a sequence of SEQ ID NO: 29, fragments having at least 10 contiguous nucleotides thereof, complements thereof, and sequences having at least 90% homology thereto.

Table 3 also describes an exemplary method for detection of an *E. coli* O111 in a sample comprising: hybridizing at least a first pair of PCR primers comprising nucleic acids of SEQ ID NO: 30 and SEQ ID NO: 31, fragments having at least 10 contiguous nucleotides thereof, complements thereof, and sequences having at least 90% homology thereto, to at least a first target polynucleotide sequence present in the sample; amplifying at least the first target polynucleotide sequence or a fragment or a complement thereof to obtain an amplified target polynucleotide sequence; and detecting the at least one amplified target polynucleotide sequence; wherein detection of the at least one amplified target polynucleotide sequence is indicative of the presence of an *E. coli* O111 in the sample. A method to detect an *E. coli* O111 may further comprise using a probe to detect the at least one amplified target polynucleotide sequence wherein the probe comprises a sequence of SEQ ID NO: 32, fragments having at least 10 contiguous nucleotides thereof, complements thereof, and sequences having at least 90% homology thereto.

Table 3 also describes example primers, probes and assay methods for detecting *E. coli* O157:H7, including the two assays 18158 and 19055 which are also described in U.S. Provisional Patent Application, Serial No. 61/178,931, filed May 15, 2009 (Attorney Docket NO. LT00032Pro); US. Patent Application, Serial NO. 12/780,707 filed May 14, 2010 (Attorney Docket NO. LT00032US); PCT Application Serial No. PCT/US2010/034998, filed May 14, 2010 (Attorney Docket NO. LT00032PCT); and European Patent Application Serial NO. 10720105.5, filed May 14, 2010, (Attorney Docket NO.

Table 3 also describes example primer, probe sequences operable to bind to and/or amplify and/or detect and/or identify a *stx* gene in SEQ ID NO. 1-SEQ ID NO. 10. Methods of assays for *stxl*: Assay ID NO. 14252 and for *stx2*: Assay ID Nos.14254, 29133 and 29134 are described in U.S. Provisional Patent Application, Serial No. 61/527,107, filed August 24, 2011 (Attorney Docket NO. LT00569Pro). Probe primers and assays for *stx*, including multiplex assays for detection of all *stx* variants are also described in Example 1 above.

### Example 5: Specificity of Big 6 O-serotype, eae, and stx Assays: Inclusion Exclusion Data

The present example describes specificity testing performed with the assays described in Table 3 to show specific detection of the microbe or virulence factor that the assay was designed for. The assays were tested on various species of microbes and specific detection of only microbes that the assays were designed for, *i*.*e*. detection of Big 6 pathogens and detection of microbes expressing *eae* and/or *stx*, was seen.

*Stxl* inclusion testing was done using a panel of *E. coli* strains that contain all known *stx* serotypes (Table 4 below). All known *stxl* serotypes were detected with a single *stx* assay, and all known *stx2* serotypes were detected by one of three *stx2* assays. When the *stxl* and the three *stx2* assays were combined into a single assay mixture then all *stxl* and *stx2* serotypes were detected in one reaction mix.

This reference panel of strains contain all known *stxl* and *stx2* subtypes consisting of *stx1*a, *stx1*c, *stx1*d, *stx2*a, *stx2*b, *stx2*c, *stx2*d, *stx2*e, *stx2*f, and *stx2*f (P.C.H. Feng et al, (2011) Appl. Environ. Microbiol. 77:6699-6702). Each strain was tested with a singleplex real-time PCR assay and with the multiplex real-time PCR assay created by combining the assays for *stxl*, *stx2*, *eae*, and O157:H7 into a single reaction mix. For the multiplex real-time PCR assay, *stxl* and *stx2* were detected with VIC, O157:H7 was detected with FAM, and *eae* was detected with NED. Positive detection is indicated with a "+" and no detection is left blank.

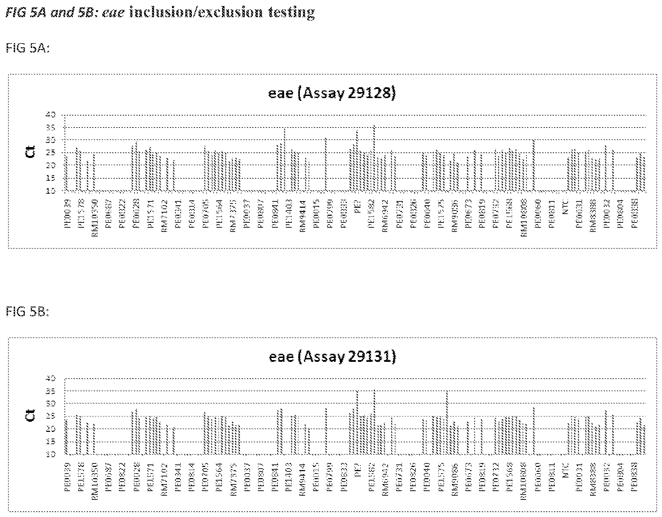

*Eae* inclusion and exclusion testing was done with 161 independent strains and is shown in FIG 5A and 5B. The presence of *eae* in the panel of *E. coli* strains were not known, and therefore the ability to detect *eae* was determined based on the consistency of detecting *eae* with two independent *eae* assays. The results of FIG 5A and FIG 5B show nearly identical detection of *eae* in 85 of the 161 *E. coli* strains.

In these studies, a panel of 161 independent *E. coli* strains were screened for the presence of an *eae* gene target using two real-time PCR assays against *eae.* The *eae* genotype of the strains were not known. FIG. 5A shows the results for *eae* assay 29128, and FIG. 5B shows the results for *eae* assay 29131. The results indicate similar detection by the two independent assays. The strain designations are listed on the X axis, and positive detection is indicated by the presence of a bar on the graph above the strain (based on Ct score).

The assays for the STEC big 6 described here are unique and highly specific. Each assay was tested against an inclusion/exclusion panel of 242 *E. coli* strains and 31 non-E. *coli* bacteria to determine assay specificity and sensitivity. The inclusion panel included all known *E. coli* O-types, strains belonging to various STEC serogroups, and 62 of the big six non-0157 STEC strains. The results of each assay showed 100% detection of inclusion strains within the targeted serotype, and no detection of exclusion strains from other serotypes. Table 4 is shown on the next page.

| **Strain** | **Serotype** | **Stx subtype** | **Stx1 assay ID 14252** | **Stx2 assay ID 14254** | **Stx2 assay ID 29134** | **Stx2 assay ID 29133** | **Multiplex Screening Assay** | | |
|---|---|---|---|---|---|---|---|---|---|
| | | | | | | | **Stx1/2** | **eae** | **O157 :H7** |
| **AA1** | **O174:H8** | **1c, 2b** | ++++ | ++++ | | | ++++ | +++ | |
| **BB2** | **O55:H7** | **1a** | ++++ | | | | ++++ | ++++ | + |
| **CC3** | **O128ac[H2]** | **2f** | | | | ++++ | ++++ | ++++ | |
| **DD4** | **O177:[H25]** | **2c, 2d** | | ++++ | | | ++++ | ++++ | |
| **EE5** | **O111:[H8]** | **1a, 2a** | ++++ | ++++ | | | ++++ | ++++ | |
| **FF6** | **0113:H4** | **1c, 2b** | ++++ | ++++ | | | ++++ | +++ | |
| **GG7** | **0103:H2** | **1a** | ++++ | | | | ++++ | ++++ | |
| **HH8** | **O26:H11** | **1a** | ++++ | | | | ++++ | ++++ | |
| **H9** | **O41:H26** | **1d** | ++++ | | | | ++++ | | |
| **JJ10** | **O157:H7** | **2c** | | ++++ | | | ++++ | ++++ | ++++ |
| **O5622** | **O138** | **2e** | | ++++ | | | ++++ | | |
| **B2F1** | **O91:H21** | **2d** | | ++++ | | | ++++ | | |
| **D3509** | **O2:H25** | **2g** | | | ++++ | | ++++ | | |

### Example 6: Multiple STEC Organism Detection Methods

In some embodiments, singleplex assays described in examples above have been combined into multiplex assays. Two example multiplex real-time PCR assay methods are described here which work together to initially screen a sample for the presence of an adulterant STEC organism and then to confirm the presence of an STEC adulterant in the sample.

In one example method, a first multiplex assay, also sometimes referred to as a first "screening assay" detects the presence in a sample of one or more target nucleic acid sequences of *E. coli* O157:H7, *stxl*, *stx2*, and *eae.* If a sample is positive it may have a target nucleic acid corresponding to either *E. coli* O157:H7 and/or *stx* (*stxl* and/or *stx2*) and/or *eae,* or have two of the three targets, or have all the three targets. Such a positive sample is then tested again by a second multiplex "confirmation assay" which is operable to detect all 6 STEC serotypes, including *E. coli* 026, 045, 0103, O111, 0121, and 0145, and is also operable to confirm the presence of *E. coli* O157:H7. The results from both assays will indicate the absence or possible presence of all *E. coli* STEC serotypes that have been currently identified by the USDA as adulterants.

Multiplex assays as described here allow individual detection of all nucleic acid targets of the STEC microorganisms desired to be detected within two reaction tubes by using multiple fluorophores. For example, in one example a "screening" multiplex assay detects *E*. *coli* O157:H7 with the fluorophore FAM; detects *stx1* and *stx2* with the fluorophore VIC; detects *eae* with the fluorophore LIZ; and detects an internal positive control (IPC) with the fluorophore NED. The "confirmation" multiplex assay detects *E. coli* O157:H7 with VIC; the big 6 non-O157:H7 STEC with FAM; and detects IPC with LIZ. The results from these two assays reveals the genotype of microorganisms within a sample matrix being tested, allowing a user (such as a food sample analyst) to decide if the sample is potentially adulterated and needs further testing.

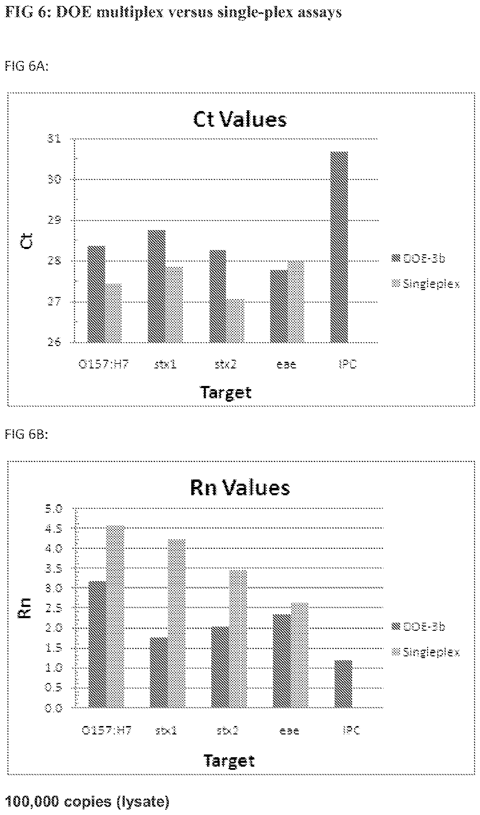

In some exemplary embodiments, TaqMan chemistry was used to design a real-time PCR method to detect *E. coli* O157:H7 and the "big 6" non-O157:H7 STEC serotypes. A minimum of four TaqMan® real-time PCR assays were designed against each of the 6 non-0157 STEC O-serotypes using Life Technologies proprietary assay design software. Additional assays were also designed against virulence factors *stxl*, *stx2*, and *eae.* Each assay was tested against an inclusion/exclusion panel of 241 *E. coli* strains consisting of 167 of the 180 known *E*. *coli* O-types to determine assay specificity and sensitivity (results described in Example 5). Multiple assays for each of the 6 non-O157 STECs detected all inclusion strains within the targeted serotype, and no exclusion strains from other serotypes. The *stx* assays detected all variants of *stxl* and *stx2* tested including *stx2f* and *stx2*g*.* The assays were combined into two separate multiplex assays and optimized using statistical methods such as Design of Experiments (DOE) (see FIG 6A and 6B).

### Example 7: Multiple STEC Organism Detection Workflows and Kits

The present disclosure also describes a workflow that combines the compositions and methods described herein to provide a rapid testing workflow for sample testing, such as testing complex food samples to determine the presence of adulterants. The present workflows provide a rapid time-to-result testing method for testing food samples, in less than 10 hours, rapidly to determine if food samples are contaminated with STEC organisms.

Ground beef and beef trim samples comprising 375 g of meat can be combined with 1 liter of prewarmed TSB broth (prewarmed to 48°C) and homogenized by hand mixing. The meat samples can be incubated at 42°C in a forced air incubator for 8 hours, and then the enriched meat samples can be tested for *E. coli* adulterants using the STEC real-time PCR screening and real-time PCR confirmation assays. The enriched samples can be prepared for real-time PCR by one of several methods. One method uses the PrepSEQ Nucleic Acid Extraction Kit (Life Technologies Corporation, Carlsbad, California) together with the MagMAX Express-96 Magnetic Particle Processor to extract DNA for real-time PCR. The protocol combines 200 µL of enriched sample with the kit lysis reagents and then captures nucleic acids on magnetic beads. The magnetic beads are washed and the nucleic acids released into 140 µL of elution buffer that can be used directly in a real-time PCR reaction. Real-time PCR can be performed on a 7500 Fast instrument in less than 50 minutes.

A workflow method for obtaining rapid time-to result according to one example comprises: 1) Sample preparation which may comprise for example PrepSEQ Nucleic Acid Extraction; 2) Screening Assay which may comprise for example: a) Standard lyophized bead assay consisting of primers and probe for all assays, master mix solution, and excipients required for lyophilization, b) one assay for *E. coli* O157:H7 (one of the two assays for O157:H7), assays for *stx1*/*2* (capable of detecting all known variants), an assay for *eae* (capable of detecting all known variants) and an assay for an internal positive control (IPC); and performing 3) a Confirmation Assay comprising: a) Standard lyophilized bead assay consisting of primers and probe for all assays, master mix solution, and excipients required for lyophilization, b) one assay for *E. coli* O157:H7 (one of the two assays for O157:H7), one assays each for the Big six O types of *E. coli* as described in Table 3 above (Big 6 are as defined by the USDA Regulation); and an assay for IPC.

A workflow for rapid time to results according to one example includes Real-time PCR detection using the Applied Biosystems 7500 Fast instrument and selecting fast cycling conditions. Default cycling conditions for the 7500 Fast instrument (using fast cycling mode) includes a 95°C denaturation step for 2 minutes, followed by 40 cycles consisting of a 95°C denaturation step for 3 seconds and a 60°C polymerization step for 30 seconds. The default PCR cycling conditions can be changed to increase the 95°C denaturation step to 5 minutes, and the amplification step to cycle between 95°C for 5 seconds and a 60°C for 30 seconds.

A workflow for rapid time to results according to one example includes a software analysis tool for interpreting real-time PCR results in order to provide a simple presence/absence call. One example of a software analysis tool is Rapid Finder Express software, or RFE. RFE software contains settings for PCR cycle number and relative fluorescence signal strength to set thresholds that must be met to determine if a sample is positive.

While the foregoing specification teaches the principles of the present invention, with examples provided for the purpose of illustration, it will be appreciated by one skilled in the art from reading this disclosure that various changes in form and detail can be made without departing from the spirit and scope of the invention. These methods are not limited to any particular type of sample or nucleic acid contained therein for e.g., total genome DNA, RNA, cDNA and the like may be analyzed using some or all of the methods disclosed in this disclosure. The methods are also not limited by the specific multiplex examples described in examples and any combination of singleplex assays may be used to make a multiplex design. This disclosure provides powerful tools for analysis of complex nucleic acid samples. From experiment design to detection of shiga toxin expressing microbes, the above disclosure provides for fast, efficient and inexpensive methods for detection of pathogenic organisms.

Although the present invention has been described in some detail by way of illustration and example for purposes of clarity and understanding, it will be apparent that certain changes and modifications may be practiced within the scope of the appended claims.

## Claims

1. A method for detection of one or more microorganisms expressing one or more virulence factors comprising:
a) contacting a sample suspected of containing one or more microorganisms expressing the one or more virulence factors with at least two sets of oligonucleotide primers specific to hybridize to target nucleic acids specific to the one or more virulence factors;
b) amplifying at least one target nucleic acid sequence specific to the one or more virulence factors by a multiplex amplification method to obtain one or more amplified virulence factor specific nucleic acids;
c) detecting the one or more amplified virulence factor specific nucleic acids or fragments or complements thereof; and
d) identifying the amplified virulence factor specific nucleic acids, wherein detecting the one or more amplified virulence factor specific nucleic acids or fragments or complements thereof is indicative of the presence of a microorganism in the sample expressing the one or more virulence factor, wherein at least two sets of the oligonucleotide primers specific to the one or more virulence factors comprise at least a forward primer and at least a reverse primer operable to hybridize to a shiga toxin *(stx)* specific target nucleic acid and at least a forward primer and at least a reverse primer operable to hybridize to an *eae* specific target nucleic acid,
wherein the oligonucleotide primers specific to hybridize to a *stx* specific target nucleic acid comprises one or more primer sets selected from a first primer set having SEQ ID NO: 1 and SEQ ID NO: 2; a second primer set having SEQ ID NO: 4 and SEQ ID NO: 5; a third primer set having SEQ ID NO: 6 and SEQ ID NO: 7; or a fourth primer set having SEQ ID NO: 8 and SEQ ID NO: 9 and the oligonucleotide primers specific to hybridize to an *eae* specific target nucleic acid comprise one or more primer sets selected from a primer set having SEQ ID NO: 11 and SEQ ID NO: 12 or a primer set having SEQ ID NO: 11 and SEQ ID NO: 13.

2. The method of claim 1, comprising detecting an *STEC* organism or a *Shigella spp.*

3. A method of claim 1, wherein the one or more virulence factor is a shiga toxin comprising:
a) contacting nucleic acids present in the sample with at least one *stx1* primer set, having one forward primer and one reverse primer, comprising primers having SEQ ID NO: 1 and SEQ ID NO 2 under conditions to amplify from the sample an *stx1* encoding nucleic acid, a fragment or a complement thereof by; and
b) amplifying simultaneously from the same sample an *stx2* encoding nucleic acid, a fragment or a complement thereof by simultaneously contacting nucleic acids present in the sample with at least one *stx2* specific primer set, each *stx2* primer set having one forward primer and one reverse primer, selected from: a first *stx2* primer set having SEQ ID NO: 4 and SEQ ID NO 5; and/or a second *stx2* primer set having SEQ ID NO: 6 and SEQ ID NO:7; and/or a third *stx2* primer set having SEQ ID NO: 8 and SEQ ID NO: 9, wherein the contacting is performed under conditions suitable for a nucleic acid amplification reaction; and
c) detecting at least one amplified nucleic acid amplified by either the amplification reactions of steps a) and/or b), wherein detection of at least one amplified nucleic acid indicates the presence of a shiga toxin producing organism in the sample.

4. The method of claim 1, further comprising:
determining the strain of the one or more microorganism expressing the one or more virulence factor comprising:
a) contacting the sample with at least one set of oligonucleotide primers specific to the strain of the one or more microorganism;
b) coamplifying at least one strain specific target nucleic acid sequence encoding for nucleic acid targets specific to at least one strain of the microorganism by a multiplex amplification method to obtain one or more amplified strain specific nucleic acids;
c) detecting the one or more amplified strain specific nucleic acid or fragments or complements thereof; and
d) identifying the amplified strain specific nucleic acids.

5. The method of claim 4, wherein each set of oligonucleotide primers specific to the one or more virulence factors is labeled with a different label and/or wherein each set of oligonucleotide primers specific to the one or more strain of the microorganism is labeled with a different label.

6. The method of claim 4, wherein the microorganism is an *E. coli* O26, an *E. coli* O45, an *E. coli* 0103, an *E. coli* O111, an *E. coli* O121 or an *E. coli* O145*.*

7. The method of claim 6, wherein the microorganism is an *E. coli* 0121 and wherein the oligonucleotide primers specific to the strain comprises nucleic acids of SEQ ID NO: 15 and SEQ ID NO: 16, complements thereof, and sequences having at least 90% homology thereto; and
the method optionally further comprising using a probe to detect the at least one amplified *E. coli* 0121 target polynucleotide sequence wherein the probe comprises a sequence of SEQ ID NO: 17, complements thereof, and sequences having at least 90% homology thereto.

8. The method of claim 6, wherein the microorganism is an *E. coli* 0145 and wherein the oligonucleotide primers specific to the strain comprises nucleic acids of SEQ ID NO: 18 and SEQ ID NO: 19, complements thereof, and sequences having at least 90% homology thereto; and
the method further optionally comprising using a probe to detect the at least one amplified *E. coli* 0145 target polynucleotide sequence wherein the probe comprises a sequence of SEQ ID NO: 20, complements thereof, and sequences having at least 90% homology thereto.

9. The method of claim 6, wherein the microorganism is an *E. coli* O26 and wherein the oligonucleotide primers specific to the strain comprises nucleic acids of SEQ ID NO: 21 and SEQ ID NO: 22, complements thereof, and sequences having at least 90% homology thereto; and
the method further optionally comprising using a probe to detect the at least one amplified *E. coli* O26 target polynucleotide sequence wherein the probe comprises a sequence of SEQ ID NO: 23, complements thereof, and sequences having at least 90% homology thereto.

10. The method of claim 6, wherein the microorganism is an *E. coli* O45 and wherein the oligonucleotide primers specific to the strain comprises nucleic acids of SEQ ID NO: 24 and SEQ ID NO: 25, complements thereof, and sequences having at least 90% homology thereto; and
the method further optionally comprising using a probe to detect the at least one amplified *E. coli* O45 target polynucleotide sequence wherein the probe comprises a sequence of SEQ ID NO: 26, complements thereof, and sequences having at least 90% homology thereto.

11. The method of claim 6, wherein the microorganism is an *E. coli* O103 and wherein the oligonucleotide primers specific to the strain comprises nucleic acids of SEQ ID NO: 27 and SEQ ID NO: 28, complements thereof, and sequences having at least 90% homology thereto; and
the method further optionally comprising using a probe to detect the at least one amplified *E. coli* 0103 target polynucleotide sequence wherein the probe comprises a sequence of SEQ ID NO: 29, complements thereof, and sequences having at least 90% homology thereto.

12. The method of claim 6, wherein the microorganism is an *E. coli* O111 and wherein the oligonucleotide primers specific to the strain comprises nucleic acids of SEQ ID NO: 30 and SEQ ID NO: 31, complements thereof, and sequences having at least 90% homology thereto; and
the method further optionally comprising using a probe to detect the at least one amplified *E. coli* O111 target polynucleotide sequence wherein the probe comprises a sequence of SEQ ID NO: 32, complements thereof, and sequences having at least 90% homology thereto.

13. A kit for detection of one or more microorganisms expressing one or more virulence factors comprising:
at least two sets of oligonucleotide primers specific to hybridize to target nucleic acids specific to one or more virulence factors, wherein the oligonucleotide primers specific to the one or more virulence factors comprise at least a forward primer and at least a reverse primer operable to hybridize to a shiga toxin *(stx)* specific target nucleic acid and at least a forward primer and at least a reverse primer operable to hybridize to an *eae* specific target nucleic acid,
wherein the one or more sets of oligonucleotide primers specific for the shiga toxin *(stx)* specific target nucleic acid producing organism comprise:
at least one pair of forward and reverse PCR primer sets selected from a first primer set having SEQ ID NO: 1 and SEQ ID NO 2; and/or a second primer set having SEQ ID NO: 4 and SEQ ID NO 5; and/or a third primer set having SEQ ID NO: 6 and SEQ ID NO:7; and/or a fourth primer set having SEQ ID NO: 8 and SEQ ID NO 9, and/or sequences comprising at least 90% nucleic acid sequence identity thereof, or a labeled derivative thereof; and
optionally comprising at least one probe selected from probes selected from SEQ ID NO: 3, and SEQ ID NO: 10 or sequences comprising at least 90% nucleic acid sequence identity thereof, or a labeled derivative thereof; and
wherein the one or more sets of oligonucleotide primers specific for the eae specific target nucleic acid producing organism comprise:
at least one pair of forward and reverse PCR primer sets selected from a first primer set having having SEQ ID NO: 11 and SEQ ID NO: 12; or having SEQ ID NO: 11 and SEQ ID NO: 13 or sequences complementary thereto, or sequences comprising at least 90% nucleic acid sequence identity thereof, or a labeled derivative thereof; and
optionally at least one probe is further selected SEQ ID NO: 14, SEQ ID NO: 3, SEQ ID NO: 10, or sequences complementary thereto, or sequences comprising at least 90% nucleic acid sequence identity thereof, or a labeled derivative thereof; and
one or more components selected from a group consisting of: at least one enzyme, dNTPs, at least one buffer, at least one salt, at least one control nucleic acid sample and an instruction protocol.

14. The kit of claim 13, further useful for determining the strain of the one or more microorganism expressing the one or more virulence factor, comprising:
one or more set of oligonucleotide primers specific to the strain of the one or more microorganism selected from:
a) SEQ ID NO: 15 and SEQ ID NO: 16, complements thereof, and sequences having at least 90% homology thereto and labeled derivatives thereof and the kit further optionally comprising a probe having SEQ ID NO: 17, complements thereof, labeled deritvative thereof and sequences having at least 90% homology thereto for detection of an *E*. *coli* O121 strain;
b) SEQ ID NO: 18 and SEQ ID NO: 19 complements thereof, labeled derivatives thereof and sequences having at least 90% homology thereto; and the kit further optionally comprising a probe having SEQ ID NO: 20, complements thereof, labeled derivatives thereof and sequences having at least 90% homology thereto for detection of an . *E.coli* 0145 strain;
c) SEQ ID NO: 21 and SEQ ID NO: 22, complements thereof, labeled derivatives thereof and sequences having at least 90% homology thereto: and the kit further optionally comprising a probe having SEQ ID NO: 23, complements thereof, labeled derivatives thereof and sequences having at least 90% homology thereto for detection of an *E. coli* O26 strain;
d) SEQ ID NO: 24 and SEQ ID NO: 25, complements thereof, labeled derivatives thereof and sequences having at least 90% homology thereto: and the kit further optionally comprising a probe having SEQ ID NO: 26, complements thereof, labeled derivatives thereof and sequences having at least 90% homology thereto for detection of an *E. coli* O45 strain;
e) SEQ ID NO: 27 and SEQ ID NO: 28, complements thereof, labeled derivatives thereof and sequences having at least 90% homology thereto; and the kit further optionally comprising a probe having SEQ ID NO: 29, complements thereof, and sequences having at least 90% homology thereto for detection of an E. *coli* O103 strain;
f) SEQ ID NO: 30 and SEQ ID NO: 31, complements thereof, labeled derivatives thereof and sequences having at least 90% homology thereto; and the kit further optionally comprising a probe having SEQ ID NO: 32, labeled derivatives thereof complements thereof, and sequences having at least 90% homology thereto for detection of an *E. coli* O111 strain.

15. The kit of claim 14 comprising:
two or more pairs of forward and reverse polymerase chain reaction (PCR) primers selected from a first primer set having SEQ ID NO:15 and SEQ ID NO: 16; a second primer having SEQ ID NO: 18 and SEQ ID NO: 19; a third primer set having SEQ ID NO: 21 and SEQ ID NO: 22; a fourth primer set having SEQ ID NO: 24 and SEQ ID NO: 25, a fifth primer set having SEQ ID NO:27 and SEQ ID NO:28; a sixth primer set having SEQ ID NO: 30 and SEQ ID NO:31, or sequences complementary thereto, or sequences comprising at least 90% nucleic acid sequence identity thereto, or a labeled derivative thereof;
optionally at least one probe selected from SEQ ID NO: 17; SEQ ID NO: 20; SEQ ID NO: 23; SEQ ID NO: 26; SEQ ID NO: 29; and SEQ ID NO: 32 and/or sequences comprising at least 90% nucleic acid sequence identity thereof, or a labeled derivative thereof;
and further optionally wherein the two or more pairs of forward and reverse PCR primers are further selected from a seventh primer set having SEQ ID NO: 11 and SEQ ID NO: 12 and SEQ ID NO: 13; an eighth primer set having SEQ ID NO: 1 and SEQ ID NO: 2; a ninth primer set having SEQ ID NO:4 and SEQ ID NO:5; a tenth primer set having SEQ ID NO: 6 and SEQ ID NO:7; an eleventh primer set having SEQ ID NO:8 and SEQ ID NO:9 and/or sequences comprising at least 90% nucleic acid sequence identity thereof, or a labeled derivative thereof; and
optionally at least one probe further is selected SEQ ID NO: 14, SEQ ID NO: 3, SEQ ID NO: 10, or sequences complementary thereto, or sequences comprising at least 90% nucleic acid sequence identity thereof, or a labeled derivative thereof; and
further optionally wherein the two or more pairs of forward and reverse PCR primers are further selected from a twelfth primer set having SEQ ID NO: 33 and SEQ ID NO: 34; a thirteenth primer set having SEQ ID NO: 36 and SEQ ID NO: 37 and/or sequences comprising at least 90% nucleic acid sequence identity thereof, or a labeled derivative thereof; and
optionally the at least one probe is further selected from SEQ ID NO: 35, SEQ ID NO: 38; or sequences complementary thereto, or sequences comprising at least 90% nucleic acid sequence identity thereof, or a labeled derivative thereof.

## Patentansprüche

1. Verfahren zum Detektieren eines oder mehrerer Mikroorganismen, die einen oder mehrere Virulenzfaktoren exprimieren, umfassend:
a) Inkontaktbringen einer Probe, bei der der Verdacht besteht, dass sie einen oder mehrere Mikroorganismen enthält die den einen oder die mehreren Virulenzfaktoren exprimieren, mit mindestens zwei Sätzen von Oligonukleotidprimern, die spezifisch sind, um zu Ziel-Nukleinsäuren zu hybridisieren, die spezifisch für den einen oder die mehreren Virulenzfaktoren sind;
b) Amplifizieren von mindestens einer Ziel-Nukleinsäuresequenz, die spezifisch für den einen oder die mehreren Virulenzfaktoren ist, durch ein Multiplex-Amplifizierungsverfahren, um eine oder mehrere spezifische Nukleinsäuren für den amplifizierten Virulenzfaktor zu erlangen;
c) Detektieren der einen oder mehreren spezifischen Nukleinsäuren für den amplifizierten Virulenzfaktor oder Fragmente oder Komplemente davon; und
d) Identifizieren der spezifischen Nukleinsäuren für den amplifizierten Virulenzfaktor, wobei ein Detektieren der einen oder mehreren spezifische Nukleinsäuren für den amplifizierten Virulenzfaktor oder Fragmente oder Komplemente davon ein Hinweis auf das Vorhandensein eines Mikroorganismus in der Probe ist, der den einen oder die mehreren Virulenzfaktoren exprimiert, wobei mindestens zwei Sätze der Oligonukleotidprimer, die spezifisch für den einen oder die mehreren Virulenzfaktoren sind, mindestens einen Vorwärtsprimer und mindestens einen Rückwärtsprimer umfassen, die eingesetzt werden können, um zu einer spezifischen Ziel-Nukleinsäure für Shiga-Toxin (*stx*) zu hybridisieren, und mindestens einen Vorwärtsprimer und mindestens einen Rückwärtsprimer, die eingesetzt werden können, um zu einer spezifischen Ziel-Nukleinsäure für *eae* zu hybridisieren,
wobei die Oligonukleotidprimer, die spezifisch sind, um zu einer spezifischen Ziel-Nukleinsäure für *stx* zu hybridisieren, einen oder mehrere Primersätze umfassen, die ausgewählt sind aus einem ersten Primersatz mit der SEQ ID NO: 1 und SEQ ID NO: 2; einem zweiten Primersatz mit der SEQ ID NO: 4 und SEQ ID NO: 5; einem dritten Primersatz mit der SEQ ID NO: 6 und SEQ ID NO: 7; oder einem vierten Primersatz mit der SEQ ID NO: 8 und SEQ ID NO: 9 und die Oligonukleotidprimer, die spezifisch sind, um zu einer spezifischen Ziel-Nukleinsäure für *eae* zu hybridisieren, einen oder mehrere Primersätze umfassen, die ausgewählt sind aus einem Primersatz mit der SEQ ID NO: 11 und SEQ ID NO: 12 oder einem Primersatz mit der SEQ ID NO: 11 und SEQ ID NO: 13.

2. Verfahren nach Anspruch 1, umfassend ein Detektieren eines *STEC*-Organismus oder eines *Shigella spp.*

3. Verfahren nach Anspruch 1, wobei der eine oder die mehreren Virulenzfaktoren ein Shiga-Toxin ist, umfassend:
a) Inkontaktbringen von Nukleinsäuren, die in der Probe vorhanden sind, mit mindestens einem *stx1*-Primersatz, der einen Vorwärtsprimer und einen Rückwärtsprimer hat, umfassend Primer mit der SEQ ID NO: 1 und SEQ ID NO: 2, unter Bedingungen, um aus der Probe eine *stx1*-kodierende Nukleinsäure, ein Fragment oder ein Komplement davon zu amplifizieren; und
b) gleichzeitiges Amplifizieren aus der gleichen Probe einer stx2-kodierenden Nukleinsäure, eines Fragments oder eines Komplements davon durch gleichzeitiges Inkontaktbringen von Nukleinsäuren, die in der Probe vorhanden sind, mit mindestens einem *stx2*-spezifischen Primersatz, wobei jeder stx2-Primersatz einen Vorwärtsprimer und einen Rückwärtsprimer hat, ausgewählt aus: einem ersten stx2-Primersatz mit der SEQ ID NO: 4 und SEQ ID NO 5; und/oder einem zweiten stx2-Primersatz mit der SEQ ID NO: 6 und SEQ ID NO:7; und/oder einem dritten *stx2*-Primersatz mit der SEQ ID NO: 8 und SEQ ID NO: 9, wobei das Inkontaktbringen unter Bedingungen ausgeführt wird, die für eine Nukleinsäure-Amplifizierungsreaktion geeignet sind; und
c) Detektieren von mindestens einer amplifizierten Nukleinsäure, die entweder durch die Amplifizierungsreaktionen des Schritts a) und/oder b) amplifiziert wurden, wobei eine Detektion von mindestens einer amplifizierten Nukleinsäure ein Hinweis auf das Vorhandensein eines Organismus in der Probe ist, der Shiga-Toxin erzeugt.

4. Verfahren nach Anspruch 1, ferner umfassend:
Bestimmen des Stamms des einen oder der mehreren Mikroorganismen, die den einen oder die mehreren Virulenzfaktoren exprimieren, umfassend:
a) Inkontaktbringen der Probe mit mindestens einem Satz von Oligonukleotidprimern, die spezifisch für den Stamm des einen oder der mehreren Mikroorganismen sind;
b) Co-Amplifizieren von mindestens einer stammspezifischen Ziel-Nukleinsäuresequenz, die für Nukleinsäureziele kodiert, die spezifisch für mindestens einen Stamm des Mikroorganismus ist, durch ein Multiplex-Amplifizierungsverfahren, um eine oder mehrere amplifizierte stammspezifische Nukleinsäuren zu erlangen;
c) Detektieren der einen oder mehreren amplifizierten stammspezifischen Nukleinsäuren oder Fragmenten oder Komplementen davon; und
d) Identifizieren der amplifizierten stammspezifischen Nukleinsäuren.

5. Verfahren nach Anspruch 4, wobei jeder Satz von Oligonukleotidprimern, die spezifisch für den einen oder die mehreren Virulenzfaktoren sind, mit einem verschiedenen Label markiert ist, und/oder wobei jeder Satz von Oligonukleotidprimern, die spezifisch für den einen oder die mehreren Stämme des Mikroorganismus sind, mit einem verschiedenen Label markiert ist.

6. Verfahren nach Anspruch 4, wobei der Mikroorganismus ein *E*. *coli* O26, ein *E*. *coli* O45, ein *E. coli* O103, ein *E*. *coli* O111, ein *E*. *coli* O121 oder ein *E. coli* O145 ist.

7. Verfahren nach Anspruch 6, wobei der Mikroorganismus ein *E. coli* O121 ist, und wobei die Oligonukleotidprimer, die spezifisch für den Stamm sind, Nukleinsäuren der SEQ ID NO: 15 und SEQ ID NO: 16, Komplemente davon und Sequenzen mit einer Homologie von mindestens 90 % dazu umfassen; und
wobei das Verfahren optional ferner ein Verwenden einer Sonde umfasst, um die mindestens eine amplifizierte Ziel-Polynukleotidsequenz für *E. coli* O121 zu detektieren, wobei die Sonde eine Sequenz von SEQ ID NO: 17, Komplemente davon und Sequenzen mit einer Homologie von mindestens 90 % dazu umfasst.

8. Verfahren nach Anspruch 6, wobei der Mikroorganismus ein *E. coli* O145 ist, und wobei die Oligonukleotidprimer, die spezifisch für den Stamm sind, Nukleinsäuren der SEQ ID NO: 18 und SEQ ID NO: 19, Komplemente davon und Sequenzen mit einer Homologie von mindestens 90 % dazu umfassen; und
wobei das Verfahren optional ferner ein Verwenden einer Sonde umfasst, um die mindestens eine amplifizierte Ziel-Polynukleotidsequenz für *E. coli* O145 zu detektieren, wobei die Sonde eine Sequenz von SEQ ID NO: 20, Komplemente davon und Sequenzen mit einer Homologie von mindestens 90 % dazu umfasst.

9. Verfahren nach Anspruch 6, wobei der Mikroorganismus ein *E. coli* O26 ist, und wobei die Oligonukleotidprimer, die spezifisch für den Stamm sind, Nukleinsäuren der SEQ ID NO: 21 und SEQ ID NO: 22, Komplemente davon und Sequenzen mit einer Homologie von mindestens 90 % dazu umfassen; und
wobei das Verfahren optional ferner ein Verwenden einer Sonde umfasst, um die mindestens eine amplifizierte Ziel-Polynukleotidsequenz für *E. coli* O26 zu detektieren, wobei die Sonde eine Sequenz von SEQ ID NO: 23, Komplemente davon und Sequenzen mit einer Homologie von mindestens 90 % dazu umfasst.

10. Verfahren nach Anspruch 6, wobei der Mikroorganismus ein *E. coli* O45 ist, und wobei die Oligonukleotidprimer, die spezifisch für den Stamm sind, Nukleinsäuren der SEQ ID NO: 24 und SEQ ID NO: 25, Komplemente davon und Sequenzen mit einer Homologie von mindestens 90 % dazu umfassen; und
wobei das Verfahren optional ferner ein Verwenden einer Sonde umfasst, um die mindestens eine amplifizierte Ziel-Polynukleotidsequenz für *E. coli* O45 zu detektieren, wobei die Sonde eine Sequenz von SEQ ID NO: 26, Komplemente davon und Sequenzen mit einer Homologie von mindestens 90 % dazu umfasst.

11. Verfahren nach Anspruch 6, wobei der Mikroorganismus ein *E. coli* O103 ist, und wobei die Oligonukleotidprimer, die spezifisch für den Stamm sind, Nukleinsäuren der SEQ ID NO: 27 und SEQ ID NO: 28, Komplemente davon und Sequenzen mit einer Homologie von mindestens 90 % dazu umfassen; und
wobei das Verfahren optional ferner ein Verwenden einer Sonde umfasst, um die mindestens eine amplifizierte Ziel-Polynukleotidsequenz für *E. coli* O103 zu detektieren, wobei die Sonde eine Sequenz von SEQ ID NO: 29, Komplemente davon und Sequenzen mit einer Homologie von mindestens 90 % dazu umfasst.

12. Verfahren nach Anspruch 6, wobei der Mikroorganismus ein *E. coli* O111 ist, und wobei die Oligonukleotidprimer, die spezifisch für den Stamm sind, Nukleinsäuren der SEQ ID NO: 30 und SEQ ID NO: 31, Komplemente davon und Sequenzen mit einer Homologie von mindestens 90 % dazu umfassen; und
wobei das Verfahren optional ferner ein Verwenden einer Sonde umfasst, um die mindestens eine amplifizierte Ziel-Polynukleotidsequenz für *E. coli* O111 zu detektieren, wobei die Sonde eine Sequenz von SEQ ID NO: 32, Komplemente davon und Sequenzen mit einer Homologie von mindestens 90 % dazu umfasst.

13. Kit zum Detektieren eines oder mehrerer Mikroorganismen, die einen oder mehrere Virulenzfaktoren exprimieren, umfassend:
mindestens zwei Sätze von Oligonukleotidprimern, die spezifisch für den einen oder die mehreren Virulenzfaktoren sind, mindestens einen Vorwärtsprimer und mindestens einen Rückwärtsprimer umfassen, die eingesetzt werden können, um zu einer spezifischen Ziel-Nukleinsäure für Shiga-Toxin (*stx*) zu hybridisieren, und mindestens einen Vorwärtsprimer und mindestens einen Rückwärtsprimer, die eingesetzt werden können, um zu einer spezifischen Ziel-Nukleinsäure für *eae* zu hybridisieren,
wobei der eine oder die mehreren Sätze von Oligonukleotidprimern, die spezifisch für den Organismus sind, der die spezifische Ziel-Nukleinsäure für das Shiga-Toxin (*stx*) erzeugt, folgendes umfassen:
mindestens ein Paar Vorwärts- und Rückwärts-PCR-Primersätze, ausgewählt aus einem ersten Primersatz mit der SEQ ID NO: 1 und SEQ ID NO 2; und/oder einem zweiten Primersatz mit der SEQ ID NO: 4 und SEQ ID NO 5; und/oder einem dritten Primersatz mit der SEQ ID NO: 6 und SEQ ID NO:7; und/oder einem vierten Primersatz mit der SEQ ID NO: 8 und SEQ ID NO 9, und/oder Sequenzen, die eine Nukleinsäuresequenz-Identität von mindestens 90 % davon umfassen, oder einem markierten Derivat davon; und
optional umfassend mindestens eine Sonde, die ausgewählt ist aus Sonden, die ausgewählt sind aus SEQ ID NO: 3, und SEQ ID NO: 10 oder Sequenzen, die eine Nukleinsäuresequenz-Identität von mindestens 90 % davon umfassen, oder einem markierten Derivat davon; und wobei der eine oder die mehreren Sätze von Oligonukleotidprimern, die spezifisch für den Organismus sind, der die spezifische Ziel-Nukleinsäure für das *eae* erzeugt, folgendes umfassen:
mindestens ein Paar Vorwärts- und Rückwärts-PCR-Primersätze, ausgewählt aus einem ersten Primersatz mit der SEQ ID NO: 11 und SEQ ID NO: 12; oder mit der SEQ ID NO: 11 und SEQ ID NO: 13 oder dazu komplementären Sequenzen, oder Sequenzen, die eine Nukleinsäuresequenz-Identität von mindestens 90 % davon umfassen, oder einem markierten Derivat davon; und
optional mindestens eine Sonde ferner ausgewählt ist aus SEQ ID NO: 14, SEQ ID NO: 3, SEQ ID NO: 10, oder dazu komplementären Sequenzen, oder dazu komplementären Sequenzen, oder Sequenzen, die eine Nukleinsäuresequenz-Identität von mindestens 90 % davon umfassen, oder einem markierten Derivat davon; und
eine oder mehrere Komponenten ausgewählt sind aus einer Gruppe bestehend aus: mindestens einem Enzym, dNTPs, mindestens einem Puffer, mindestens einem Salz, mindestens einer Kontrollnukleinsäure-Probe und einem Anweisungsprotokoll.

14. Kit nach Anspruch 13, das ferner nützlich zum Bestimmen des Stamms des einen oder der mehreren Mikroorganismen ist, die den einen oder die mehreren Virulenzfaktoren exprimieren, umfassend:
einen oder mehrere Sätze von Oligonukleotidprimern, die spezifisch für den Stamm des einen oder der mehreren Mikroorganismen sind, ausgewählt aus:
a) SEQ ID NO: 15 und SEQ ID NO: 16, Komplementen davon und Sequenzen mit einer Homologie von mindestens 90 % dazu und markierten Derivaten davon, und das Kit ferner optional umfassend eine Sonde mit SEQ ID NO: 17, Komplemente davon, ein markiertes Derivat davon und Sequenzen mit einer Homologie von mindestens 90 % dazu zur Detektion eines Stamms *E. coli* 0121;
b) SEQ ID NO: 18 und SEQ ID NO: 19, Komplementen davon, markierten Derivaten davon und Sequenzen mit einer Homologie von mindestens 90 % dazu, und das Kit ferner optional umfassend eine Sonde mit SEQ ID NO: 20, Komplemente davon, ein markiertes Derivat davon und Sequenzen mit einer Homologie von mindestens 90 % dazu zur Detektion eines Stamms *E*. *coli* 0145;
c) SEQ ID NO: 21 und SEQ ID NO: 22, Komplementen davon, markierten Derivaten davon und Sequenzen mit einer Homologie von mindestens 90 % dazu, und das Kit ferner optional umfassend eine Sonde mit SEQ ID NO: 23, Komplemente davon, ein markiertes Derivat davon und Sequenzen mit einer Homologie von mindestens 90 % dazu zur Detektion eines Stamms *E*. *coli* O26;
d) SEQ ID NO: 24 und SEQ ID NO: 25, Komplementen davon, markierten Derivaten davon und Sequenzen mit einer Homologie von mindestens 90 % dazu, und das Kit ferner optional umfassend eine Sonde mit SEQ ID NO: 26, Komplemente davon, ein markiertes Derivat davon und Sequenzen mit einer Homologie von mindestens 90 % dazu zur Detektion eines Stamms *E*. *coli* O45;
e) SEQ ID NO: 27 und SEQ ID NO: 28, Komplementen davon, markierten Derivaten davon und Sequenzen mit einer Homologie von mindestens 90 % dazu, und das Kit ferner optional umfassend eine Sonde mit SEQ ID NO: 29, Komplemente davon und Sequenzen mit einer Homologie von mindestens 90 % dazu zur Detektion eines Stamms *E. coli* O103;
f) SEQ ID NO: 30 und SEQ ID NO: 31, Komplementen davon, markierten Derivaten davon und Sequenzen mit einer Homologie von mindestens 90 % dazu, und das Kit ferner optional umfassend eine Sonde mit SEQ ID NO: 32, markierte Derivate davon, Komplemente davon und Sequenzen mit einer Homologie von mindestens 90 % dazu zur Detektion eines Stamms *E. coli* O111.

15. Kit nach Anspruch 14, umfassend:
zwei oder mehrere Paare Vorwärts- und Rückwärts-Polymerase-Kettenreaktion-Primersätze (polymerase chain reaction, PCR) ausgewählt aus einem ersten Primersatz mit der SEQ ID NO:15 und SEQ ID NO: 16; einem zweiten Primer mit der SEQ ID NO: 18 und SEQ ID NO: 19; einem dritten Primersatz mit der SEQ ID NO: 21 und SEQ ID NO: 22; einem vierten Primersatz mit der SEQ ID NO: 24 und SEQ ID NO: 25, einem fünften Primersatz mit der SEQ ID NO:27 und SEQ ID NO:28; einem sechsten Primersatz mit der SEQ ID NO: 30 und SEQ ID NO: 31, oder dazu komplementären Sequenzen, oder Sequenzen, die eine Nukleinsäuresequenz-Identität von mindestens 90 % davon umfassen, oder einem markierten Derivat davon;
optional mindestens eine Sonde ausgewählt aus SEQ ID NO: 17; SEQ ID NO: 20; SEQ ID NO: 23; SEQ ID NO: 26; SEQ ID NO: 29; und SEQ ID NO: 32 und/oder Sequenzen, die eine Nukleinsäuresequenz-Identität von mindestens 90 % davon umfassen, oder einem markierten Derivat davon;
und ferner optional wobei die zwei oder mehreren Paare Vorwärts- und Rückwärts-PCR-Primer ferner ausgewählt sind aus einem siebten Primersatz mit der SEQ ID NO: 11 und SEQ ID NO: 12 und SEQ ID NO: 13; einem achten Primersatz mit der SEQ ID NO: 1 und SEQ ID NO: 2; einem neunten Primersatz mit der SEQ ID NO:4 und SEQ ID NO: 5; einem zehnten Primersatz mit der SEQ ID NO: 6 und SEQ ID NO: 7; einem elften Primersatz mit der SEQ ID NO:8 und SEQ ID NO: 9 und/oder Sequenzen, die eine Nukleinsäuresequenz-Identität von mindestens 90 % davon umfassen, oder einem markierten Derivat davon; und
optional mindestens eine Sonde ferner ausgewählt ist aus SEQ ID NO: 14, SEQ ID NO: 3, SEQ ID NO: 10, oder dazu komplementären Sequenzen, oder Sequenzen, die eine Nukleinsäuresequenz-Identität von mindestens 90 % davon umfassen, oder einem markierten Derivat davon; und
ferner optional wobei die zwei oder mehreren Paare Vorwärts- und Rückwärts-PCR-Primer ferner ausgewählt sind aus einem zwölften Primersatz mit der SEQ ID NO: 33 und SEQ ID NO: 34; einem dreizehnten Primersatz mit der SEQ ID NO: 36 und SEQ ID NO: 37 und/oder Sequenzen, die eine Nukleinsäuresequenz-Identität von mindestens 90 % davon umfassen, oder einem markierten Derivat davon; und
optional die mindestens eine Sonde ferner ausgewählt ist aus SEQ ID NO: 35, SEQ ID NO: 38; oder dazu komplementären Sequenzen, oder Sequenzen, die eine Nukleinsäuresequenz-Identität von mindestens 90 % davon umfassen, oder einem markierten Derivat davon.

## Revendications

1. Procédé permettant la détection d'un ou plusieurs microorganismes exprimant un ou plusieurs facteurs de virulence comprenant :
a) la mise en contact d'un échantillon censé contenir un ou plusieurs microorganismes exprimant lesdits un ou plusieurs facteurs de virulence avec au moins deux ensembles d'amorces oligonucléotidiques spécifiques pour s'hybrider à des acides nucléiques cibles spécifiques auxdits un ou plusieurs facteurs de virulence ;
b) l'amplification d'au moins une séquence d'acide nucléique cible spécifique auxdits un ou plusieurs facteurs de virulence par un procédé d'amplification par multiplexage pour obtenir un ou plusieurs acides nucléiques amplifiés spécifiques à un facteur de virulence ;
c) la détection desdits un ou plusieurs acides nucléiques amplifiés spécifiques à un facteur de virulence ou de fragments ou compléments de ceux-ci ; et
d) l'identification des acides nucléique amplifiés spécifiques à un facteur de virulence, ladite détection desdits un ou plusieurs acides nucléiques amplifiés spécifiques à un facteur de virulence ou de fragments ou compléments de ceux-ci étant révélatrice de la présence d'un microorganisme dans l'échantillon exprimant lesdits un ou plusieurs facteurs de virulence, lesdits au moins deux ensembles des amorces oligonucléotidiques spécifiques auxdits un ou plusieurs facteurs de virulence comprenant au moins une amorce sens et au moins une amorce antisens servant à s'hybrider à un acide nucléique cible spécifique à la toxine de Shiga (*stx*) et au moins une amorce sens et au moins une amorce antisens servant à s'hybrider à un acide nucléique cible spécifique à l'*eae*,
lesdites amorces oligonucléotidiques spécifiques pour s'hybrider à un acide nucléique cible spécifique à la *stx* comprenant un ou plusieurs ensembles d'amorces choisis parmi un premier ensemble d'amorces ayant la SEQ ID n° : 1 et la SEQ ID n° : 2 ; un deuxième ensemble d'amorces ayant la SEQ ID n° : 4 et la SEQ ID n° : 5 ; un troisième ensemble d'amorces ayant la SEQ ID n° : 6 et la SEQ ID n° : 7 ; un quatrième ensemble d'amorces ayant la SEQ ID n° : 8 et la SEQ ID n° : 9 ; et les amorces oligonucléotidiques spécifiques pour s'hybrider à un acide nucléique cible spécifique à l'*eae* comprenant une ou plusieurs amorces choisies parmi un ensemble d'amorces ayant la SEQ ID n° : 11 et la SEQ ID n° : 12 ou un ensemble d'amorces ayant la SEQ ID n° : 11 et la SEQ ID n° : 13.

2. Procédé selon la revendication 1, comprenant la détection d'un organisme *STEC* ou d'une *Shigella spp.*

3. Procédé selon la revendication 1, lesdits un ou plusieurs facteurs de virulence étant une toxine de Shiga comprenant :
a) la mise en contact des acides nucléiques présents dans l'échantillon avec au moins un ensemble d'amorces de *stxl,* comportant une amorce sens et une amorce antisens, comprenant des amorces ayant la SEQ ID n° : 1 et la SEQ ID n° : 2 dans des conditions permettant d'amplifier à partir de l'échantillon un acide nucléique codant *stxl,* un fragment ou un complément de celui-ci ; et
b) l'amplification simultanée à partir du même échantillon d'un acide nucléique codant *stx2,* un fragment ou un complément de celui-ci par mise en contact simultanée d'acides nucléiques présents dans l'échantillon avec au moins un ensemble d'amorces spécifique à *stx2,* chaque ensemble d'amorces de *stx2* comportant une amorce sens et une amorce antisens, choisies parmi : un premier ensemble d'amorces de *stx2* ayant la SEQ ID n° : 4 et la SEQ ID n° : 5 ; et/ou un deuxième ensemble d'amorces de *stx2* ayant la SEQ ID n° : 6 et la SEQ ID n° : 7 ; et/ou un troisième ensemble d'amorces de *stx2* ayant la SEQ ID n° : 8 et la SEQ ID n° : 9, ladite mise en contact étant effectuée dans des conditions appropriées à une réaction d'amplification d'acides nucléiques ; et
c) la détection d'au moins un acide nucléique amplifié qui a été amplifié par les réactions d'amplification des étapes a) et/ou b), ladite détection d'au moins un acide nucléique amplifié indiquant la présence d'un organisme producteur de la toxine de Shiga dans l'échantillon.

4. Procédé selon la revendication 1, comprenant en outre :
la détermination de la souche desdits un ou plusieurs microorganismes exprimant lesdits un ou plusieurs facteurs de virulence comprenant :
a) la mise en contact de l'échantillon avec au moins un ensemble d'amorces oligonucléotidiques spécifiques à la souche desdits un ou plusieurs microorganismes ;
b) la co-amplification d'au moins une séquence d'acide nucléique cible spécifique à une souche codant des cibles d'acides nucléiques spécifiques à au moins une souche du microorganisme par un procédé d'amplification par multiplexage pour obtenir un ou plusieurs acides nucléiques amplifiés spécifiques à une souche ;
c) la détection desdits un ou plusieurs acides nucléiques amplifiés spécifiques à une souche ou de fragments ou compléments de ceux-ci ; et
d) l'identification des acides nucléiques amplifiés spécifiques à une souche.

5. Procédé selon la revendication 4, chaque ensemble d'amorces oligonucléotidiques spécifique auxdits un ou plusieurs facteurs de virulence étant étiqueté avec une étiquette différente et/ou chaque ensemble d'amorces oligonucléotidiques spécifique auxdites une ou plusieurs souches du microorganisme étant étiqueté avec une étiquette différente.

6. Procédé selon la revendication 4, ledit microorganisme étant un *E coli* O26, un *E coli* O45, un *E*. *coli* O103, un *E. coli* O111, un *E. coli* O121 ou un *E*. *coli* O145.

7. Procédé selon la revendication 6, ledit microorganisme étant un *E. coli* O121 et lesdites amorces oligonucléotidiques spécifiques à la souche comprenant des acides nucléiques de SEQ ID n° : 15 et SEQ ID n° : 16, des compléments de celles-ci, et des séquences présentant une homologie d'au moins 90 % avec celles-ci ; et
le procédé comprenant en outre éventuellement l'utilisation d'une sonde pour détecter ladite au moins une séquence polynucléotidique cible d'*E. coli* O121 amplifiée, ladite sonde comprenant une séquence de SEQ n° : 17, des compléments de celle-ci, et des séquences présentant une homologie d'au moins 90 % avec celle-ci.

8. Procédé selon la revendication 6, ledit microorganisme étant un *E. coli* O145 et lesdites amorces oligonucléotidiques spécifiques à la souche comprenant des acides nucléiques de SEQ ID n° : 18 et SEQ ID n° : 19, des compléments de celles-ci, et des séquences présentant une homologie d'au moins 90 % avec celles-ci ; et
le procédé comprenant en outre éventuellement l'utilisation d'une sonde pour détecter ladite au moins une séquence polynucléotidique cible d'*E. coli* O145 amplifiée, ladite sonde comprenant une séquence de SEQ n° : 20, des compléments de celle-ci, et des séquences présentant une homologie d'au moins 90 % avec celle-ci.

9. Procédé selon la revendication 6, ledit microorganisme étant un *E. coli* O26 et lesdites amorces oligonucléotidiques spécifiques à la souche comprenant des acides nucléiques de SEQ ID n° : 21 et SEQ ID n° : 22, des compléments de celles-ci, et des séquences présentant une homologie d'au moins 90 % avec celles-ci ; et
le procédé comprenant en outre éventuellement l'utilisation d'une sonde pour détecter ladite au moins une séquence polynucléotidique cible d'*E. coli* O26 amplifiée, ladite sonde comprenant une séquence de SEQ n° : 23, des compléments de celle-ci, et des séquences présentant une homologie d'au moins 90 % avec celle-ci.

10. Procédé selon la revendication 6, ledit microorganisme étant un *E. coli* O45 et lesdites amorces oligonucléotidiques spécifiques à la souche comprenant des acides nucléiques de SEQ ID n° : 24 et SEQ ID n° : 25, des compléments de celles-ci, et des séquences présentant une homologie d'au moins 90 % avec celles-ci ; et
le procédé comprenant en outre éventuellement l'utilisation d'une sonde pour détecter ladite au moins une séquence polynucléotidique cible d'*E. coli* O45 amplifiée, ladite sonde comprenant une séquence de SEQ n° : 26, des compléments de celle-ci, et des séquences présentant une homologie d'au moins 90 % avec celle-ci.

11. Procédé selon la revendication 6, ledit microorganisme étant un *E. coli* O103 et lesdites amorces oligonucléotidiques spécifiques à la souche comprenant des acides nucléiques de SEQ ID n° : 27 et SEQ ID n° : 28, des compléments de celles-ci, et des séquences présentant une homologie d'au moins 90 % avec celles-ci ; et
le procédé comprenant en outre éventuellement l'utilisation d'une sonde pour détecter ladite au moins une séquence polynucléotidique cible d'*E. coli* O103 amplifiée, ladite sonde comprenant une séquence de SEQ n° : 29, des compléments de celle-ci, et des séquences présentant une homologie d'au moins 90 % avec celle-ci.

12. Procédé selon la revendication 6, ledit microorganisme étant un *E. coli* O111 et lesdites amorces oligonucléotidiques spécifiques à la souche comprenant des acides nucléiques de SEQ ID n° : 30 et SEQ ID n° : 31, des compléments de celles-ci, et des séquences présentant une homologie d'au moins 90 % avec celles-ci ; et
le procédé comprenant en outre éventuellement l'utilisation d'une sonde pour détecter ladite au moins une séquence polynucléotidique cible d'*E. coli* O111 amplifiée, ladite sonde comprenant une séquence de SEQ n° : 32, des compléments de celle-ci, et des séquences présentant une homologie d'au moins 90 % avec celle-ci.

13. Kit de détection d'un ou plusieurs microorganismes exprimant un ou plusieurs facteurs de virulence comprenant :
au moins deux ensembles d'amorces oligonucléotidiques spécifiques pour s'hybrider à des acides nucléiques cibles spécifiques à un ou plusieurs facteurs de virulence, lesdites amorces oligonucléotidiques spécifiques auxdits un ou plusieurs facteurs de virulence comprenant au moins une amorce sens et au moins une amorce antisens servant à s'hybrider à un acide nucléique cible spécifique à la toxine de Shiga (*stx*) et au moins une amorce sens et au moins une amorce antisens servant à s'hybrider à un acide nucléique cible spécifique à l*'eae*,
lesdits un ou plusieurs ensembles d'amorces oligonucléotidiques spécifiques à l'organisme producteur d'acide nucléique cible spécifique à la toxine de Shiga (*stx*) comprenant :
au moins une paire d'ensembles d'amorces de PCR sens et antisens choisis parmi un premier ensemble d'amorces ayant la SEQ ID n° : 1 et la SEQ ID n° : 2 ; et/ou un deuxième ensemble d'amorces ayant la SEQ ID n° : 4 et la SEQ ID n° : 5 ; et/ou un troisième ensemble d'amorces ayant la SEQ ID n° : 6 et la SEQ ID n° : 7 ; et/ou un quatrième ensemble d'amorces ayant la SEQ ID n° : 8 et la SEQ ID n° : 9, et/ou des séquences comprenant une identité de séquence d'acide nucléique d'au moins 90 % avec celles-ci, ou un dérivé étiqueté de celles-ci ; et
comprenant éventuellement au moins une sonde choisie parmi des sondes choisies parmi la SEQ ID n° : 3 et la SEQ ID n° : 10 ou des séquences comprenant une identité de séquence d'acide nucléique d'au moins 90 % avec celles-ci, ou un dérivé étiqueté de celles-ci ; et
lesdits un ou plusieurs ensembles d'amorces oligonucléotidiques spécifiques à l'organisme producteur d'acide nucléique cible spécifique à l'*eae* comprenant :
au moins une paire d'ensembles d'amorces de PCR sens et antisens choisis parmi un premier ensemble d'amorces ayant la SEQ ID n° : 11 et la SEQ ID n° : 12, ou ayant la SEQ ID n° : 11 et la SEQ ID n° : 13 ou des séquences complémentaires à celles-ci, ou des séquences comprenant une identité de séquence d'acide nucléique d'au moins 90 % avec celles-ci, ou un dérivé étiqueté de celles-ci ; et
éventuellement au moins une sonde étant en outre choisie parmi SEQ ID n° : 14, SEQ ID n° : 3, SEQ ID n° : 10, ou des séquences complémentaires à celles-ci, ou des séquences comprenant une identité de séquence d'acide nucléique d'au moins 90 % avec celles-ci, ou un dérivé étiqueté de celles-ci ; et
un ou plusieurs composants choisis dans le groupe constitué par : au moins une enzyme, des dNTP, au moins un tampon, au moins un sel, au moins un échantillon d'acide nucléique témoin et un protocole d'instructions.

14. Kit selon la revendication 13, utile en outre pour déterminer la souche desdits un ou plusieurs microorganismes exprimant lesdits un ou plusieurs facteurs de virulence, comprenant :
un ou plusieurs ensembles d'amorces oligonucléotidiques spécifiques à la souche desdits un ou plusieurs microorganismes choisis parmi :
a) SEQ ID n° : 15 et SEQ ID n° : 16, des compléments de celles-ci, et des séquences présentant une homologie d'au moins 90 % avec celles-ci et des dérivés étiquetés de celles-ci ; et le kit comprenant en outre éventuellement une sonde ayant la SEQ ID n° : 17, des compléments de celle-ci, un dérivé étiqueté de celle-ci et des séquences présentant une homologie d'au moins 90 % avec celle-ci pour la détection d'une souche d'*E. coli* 0121 ;
b) SEQ ID n° : 18 et SEQ ID n° : 19, des compléments de celles-ci, des dérivés étiquetés de celles-ci et des séquences présentant une homologie d'au moins 90 % avec celles-ci ; et le kit comprenant en outre éventuellement une sonde ayant la SEQ ID n° : 20, des compléments de celle-ci, des dérivés étiquetés de celle-ci et des séquences présentant une homologie d'au moins 90 % avec celle-ci pour la détection d'une souche d'*E. coli* 0145 ;
c) SEQ ID n° : 21 et SEQ ID n° : 22, des compléments de celles-ci, des dérivés étiquetés de celles-ci et des séquences présentant une homologie d'au moins 90 % avec celles-ci ; et le kit comprenant en outre éventuellement une sonde ayant la SEQ ID n° : 23, des compléments de celle-ci, des dérivés étiquetés de celle-ci et des séquences présentant une homologie d'au moins 90 % avec celle-ci pour la détection d'une souche d'*E. coli* O26 ;
d) SEQ ID n° : 24 et SEQ ID n° : 25, des compléments de celles-ci, des dérivés étiquetés de celles-ci et des séquences présentant une homologie d'au moins 90 % avec celles-ci ; et le kit comprenant en outre éventuellement une sonde ayant la SEQ ID n° : 26, des compléments de celle-ci, des dérivés étiquetés de celle-ci et des séquences présentant une homologie d'au moins 90 % avec celle-ci pour la détection d'une souche d'*E. coli* O45 ;
e) SEQ ID n° : 27 et SEQ ID n° : 28, des compléments de celles-ci, des dérivés étiquetés de celles-ci et des séquences présentant une homologie d'au moins 90 % avec celles-ci ; et le kit comprenant en outre éventuellement une sonde ayant la SEQ ID n° : 29, des compléments de celle-ci, et des séquences présentant une homologie d'au moins 90 % avec celle-ci pour la détection d'une souche d'*E*. *coli* O103 ;
f) SEQ ID n° : 30 et SEQ ID n° : 31, des compléments de celles-ci, des dérivés étiquetés de celles-ci et des séquences présentant une homologie d'au moins 90 % avec celles-ci ; et le kit comprenant en outre éventuellement une sonde ayant la SEQ ID n° : 32, des dérivés étiquetés de celle-ci, des compléments de celle-ci et des séquences présentant une homologie d'au moins 90 % avec celle-ci pour la détection d'une souche d'*E. coli* O111 ;

15. Kit selon la revendication 14 comprenant :
deux paires d'amorces d'amplification en chaîne par polymérase (PCR) sens et antisens ou plus choisies parmi un premier ensemble d'amorces ayant la SEQ ID n° : 15 et la SEQ ID n° : 16 ; un deuxième ensemble d'amorces ayant la SEQ ID n° : 18 et la SEQ ID n° : 19 ; un troisième ensemble d'amorces ayant la SEQ ID n° : 21 et la SEQ ID n° : 22, un quatrième ensemble d'amorces ayant la SEQ ID n° : 24 et la SEQ ID n° : 25, un cinquième ensemble d'amorces ayant la SEQ ID n° : 27 et la SEQ ID n° : 28, un sixième ensemble d'amorces ayant la SEQ ID n° : 30 et la SEQ ID n° : 31, ou des séquences complémentaires à celles-ci, ou des séquences comprenant une identité de séquence d'acide nucléique d'au moins 90 % avec celles-ci, ou un dérivé étiqueté de celles-ci ;
éventuellement au moins une sonde choisie parmi SEQ ID n° : 17, SEQ ID n° : 20, SEQ ID n° : 23 ; SEQ ID n° : 26, SEQ ID n° : 29 et SEQ ID n° : 32 ; et/ou des séquences comprenant une identité de séquence d'acide nucléique d'au moins 90 % avec celles-ci, ou un dérivé étiqueté de celle-ci ; et
en outre éventuellement lesdites deux paires d'amorces de PCR sens et antisens ou plus étant en outre choisies parmi un septième ensemble d'amorces ayant la SEQ ID n° : 11 et la SEQ ID n° : 12 et SEQ ID n° : 13 ; un huitième ensemble d'amorces ayant la SEQ ID n° : 1 et la SEQ ID n° : 2 ; un neuvième ensemble d'amorces ayant la SEQ ID n° : 4 et la SEQ ID n° : 5, un dixième ensemble d'amorces ayant la SEQ ID n° : 6 et la SEQ ID n° : 7, un onzième ensemble d'amorces ayant la SEQ ID n° : 8 et la SEQ ID n° : 9, et/ou des séquences comprenant une identité de séquence d'acide nucléique d'au moins 90 % avec celles-ci, ou un dérivé étiqueté de celles-ci ; et
éventuellement au moins une sonde étant en outre choisie parmi SEQ ID n° : 14, SEQ ID n° : 3, SEQ ID n° : 10, ou des séquences complémentaires à celles-ci, ou des séquences comprenant une identité de séquence d'acide nucléique d'au moins 90 % avec celles-ci, ou un dérivé étiqueté de celle-ci ; et
en outre éventuellement lesdites deux paires d'amorces de PCR sens et antisens ou plus étant en outre choisies parmi un douzième ensemble d'amorces ayant la SEQ ID n° : 33 et la SEQ ID n° : 34 ; un treizième ensemble d'amorces ayant la SEQ ID n° : 36 et la SEQ ID n° : 37, et/ou des séquences comprenant une identité de séquence d'acide nucléique d'au moins 90 % avec celles-ci, ou un dérivé étiqueté de celles-ci ; et
éventuellement ladite au moins une sonde étant en outre choisie parmi SEQ ID n° : 35, SEQ ID n° : 38, ou des séquences complémentaires à celles-ci, ou des séquences comprenant une identité de séquence d'acide nucléique d'au moins 90 % avec celles-ci, ou un dérivé étiqueté de celle-ci.
